# EUROPEAN PATENT APPLICATION

(11) **EP 4 527 415 A1**
(43) Date of publication of application: **26.03.2025**
(21) Application number: 23806927.2
(22) Date of filing: 16.05.2023
(51) Int. Cl.: A61K 47/68, C07K 16/28, A61K 39/00, A61P 35/00

(54) **ANTIBODY-DRUG CONJUGATE CONTAINING PROTEIN DEGRADATION AGENT BIOACTIVE COMPOUND, METHOD FOR PREPARING SAME, AND USE THEREOF**

(30) Priority: 18.05.2022 CN 202210543924; 22.11.2022 CN 202211466328
(71) Applicant: Medilink Therapeutics (Suzhou) Co., Ltd., Suzhou, Jiangsu 215000 (CN)
(72) Inventor: XIAO, Liang, Suzhou, Jiangsu 215000 (CN); SONG, Shuai, Suzhou, Jiangsu 215000 (CN); SHAN, Ailin, Suzhou, Jiangsu 215000 (CN); CAI, Jiaqiang, Suzhou, Jiangsu 215000 (CN); XUE, Tongtong, Suzhou, Jiangsu 215000 (CN); SHI, Xinzhen, Suzhou, Jiangsu 215000 (CN)
(74) Representative: dompatent von Kreisler Selting Werner - Partnerschaft von Patent- und Rechtsanwälten mbB
(86) International application number: PCT/CN2023/094519
(87) International publication number: WO 2023/221975

(57) **Abstract**

Provided are an antibody-drug conjugate containing a protein degradation agent bioactive compound represented by formula I, a method for preparing same, and use thereof in preventing and/or treating diseases related to abnormal cell activity, including but not limited use thereof in preventing and/or treating tumor diseases,

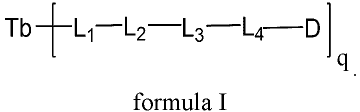

## Description

### TECHNICAL FIELD

The present disclosure belongs to the field of pharmaceutical technology, and relates to an antibody-drug conjugate of a protein degradation agent bioactive compound, a protein degradation agent bioactive compound, a drug-linker conjugate, a method for preparing same, and use thereof in the prevention and/or treatment of a disease associated with abnormal cell activity, including but not limited to the prevention and/or treatment of a tumor disease.

### BACKGROUND

Many diseases are associated with intracellular protein dysfunction, and the main means for treating these diseases are small molecule compounds. However, over 80% of proteins lack sites that can generate druggable effects, and thus such targets are thought to be undruggable by traditional small molecules.

Targeted protein degradation (TPD) agents, such as proteolysis targeting chimeras (PROTACs) and molecular glue degraders (MGDs), mediate target protein degradation through ubiquitin-proteasome pathways, and can play a role without tightly binding to sites that affect protein activity, so that the "undruggable" proteins described above can become new drug targets. Meanwhile, the targeted protein degradation therapy can continuously induce pathogenic proteins to be rapidly and efficiently degraded, thereby reducing the generation of drug resistance of the target proteins.

Currently, targeted protein degradation agents have entered the clinical development stage. C4 Therapeutics has demonstrated preclinical data for its molecular glue CFT7455 targeting IKZF1/3 at AACR in 2021. The company demonstrated clinical results of CFT7455 at the AACR meeting in April 2022, and although CFT7455 showed clinical benefits of deep target degradation, but decreased neutrophil was dose-limiting.

Currently, in the field of antibody-drug conjugates (ADCs), maleimide is widely used for coupling to thiol of an antibody by a Michael addition reaction to form an ADC molecule due to its high selectivity, high reactivity and relatively good stability. Of the 15 marketed ADC drugs, 9 were coupled via maleimide. It has been reported in a number of documents that the thiosuccinimide linkage is unstable in the presence of thiol-containing substances (e.g., in the plasma) and can be cleaved by a retro-Michael reaction or exchanged with endogenous thiols such as albumin (HAS) and glutathione (GSH), causing poor pharmacodynamics, pharmacokinetics and safety.

### SUMMARY

The present invention relates to an antibody-drug conjugate (ADC) of formula I and use thereof. ADC drugs combine the tumor-targeting effect of an antibody and the high activity of a bioactive compound to become a biological missile with very expected efficacy and safety advantages. The antibody directs ADCs to bind to target cells, so that tumor tissue enrichment is realized, exposure of non-target tissues is reduced, and toxicity possibly brought by systemic administration of bioactive compounds is reduced. ADCs binding to tumor cells can be cleaved in the tumor microenvironment and release bioactive molecules to kill the tumor cells. ADCs can also be internalized by tumor cells, and enzymolysis is carried out in the cells under the action of specific enzymes to release small molecule drugs, thereby treating diseases. Therefore, the ADC formed by combining a protein degradation agent and a tumor-targeting antibody can be expected to realize tumor enrichment, eliminate or reduce toxic and side effects caused by the protein degradation agent acting on non-disease tissues, and improve the treatment effect. Using ADC technology to realize tumor targeting of the protein degradation agent and reduce the toxic and side effects thereof will have high clinical value.

For this purpose, in a first aspect of the present disclosure, the present disclosure provides an antibody-drug conjugate of formula I, 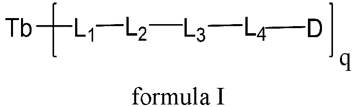
or a stereoisomer of the antibody-drug conjugate, a prodrug thereof, a pharmaceutically acceptable salt thereof or a pharmaceutically acceptable solvate thereof,
wherein,
Tb is an antibody or an antigen-binding fragment thereof;
q is the number of L₁-L₂-L₃-L₄-D coupled to Tb;
D is a protein degradation agent bioactive compound fragment;
L₁ is a linker unit;
L₂ is a connector unit;
L₃ is selected from an amino acid residue represented by AA¹ or a short peptide consisting of 2-10 amino acid residues comprising the amino acid residue represented by AA¹; the amino acid residue is selected from a natural amino acid residue, a non-natural amino acid residue, the amino acid residue represented by AA¹ or a stereoisomer thereof;
the amino acid residue represented by AA¹ has a structure shown below: 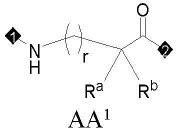 wherein:
   R^{a} and R^{b} are each independently selected from hydrogen or 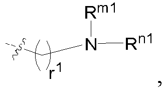 and R^{a} and R^{b} are not both hydrogen;
   or R^{a} and R^{b}, together with the carbon atom to which they are both linked, form a 4-10 membered heterocycle, and the 4-10 membered heterocycle is optionally substituted with one or more R⁰;
   r and r¹ are each independently selected from any integer between 0 and 20;
   R^{m1} and Rⁿ¹ are each independently selected from hydrogen, C1-6 alkyl or C3-6 cycloalkyl;
   or R^{m1} and Rⁿ¹, together with the nitrogen atom to which they are both linked, form a 4-10 membered heterocycle, and the 4-10 membered heterocycle is optionally substituted with one or more R^{0'};
   R⁰ and R^{0'} are each independently selected from C1-6 alkyl, C3-6 cycloalkyl, -NR^{m2}Rⁿ², or 4-10 membered heterocyclyl optionally substituted with C1-6 alkyl;
   R^{m2} and Rⁿ² are each independently selected from hydrogen or C1-6 alkyl;
   L₄ is absent or present, and when L₄ is present, L₄ is selected from 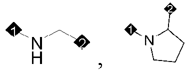 or 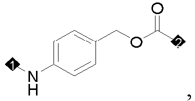 wherein position 1 is attached to L₃, and position 2 is attached to D.

In some embodiments, the protein degradation agent bioactive compound is selected from a proteolysis targeting chimera or a molecular glue.

In some embodiments, the protein degradation agent bioactive compound is selected from one or more of the following:
(1) a proteolysis targeting chimera that can cause the degradation of family proteins such as GSPT1, IKZF1 and/or 3, CK1α; and
(2) a molecular glue that can cause the degradation of the binding of family proteins such as GSPT1, IKZF1 and/or 3, CK1α.

In some embodiments, the protein degradation agent bioactive compound is a molecule that can lead to the degradation of GSPT1 family proteins.

In some embodiments, q is selected from any value between 0.1 and 12.0.

In some embodiments, q is selected from any value between 1.0 and 10.0.

In some embodiments, q is selected from 1, 2, 3, 4, 5, 6, 7, 8, 9 or 10.

In some embodiments, q is selected from any value between 2 and 8.

In some embodiments, q is selected from 2, 4, 6 or 8.

In some embodiments, L₁ is selected from 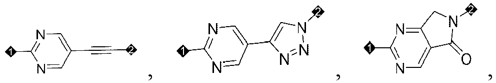 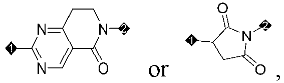 wherein position 1 is attached to Tb via an S atom, and position 2 is attached to L₂.

In some embodiments, L₁ is selected from 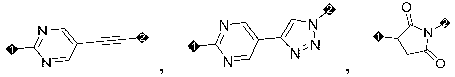 or 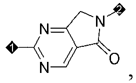 wherein position 1 is attached to Tb via an S atom, and position 2 is attached to L₂.

In some embodiments, L₂ is selected from 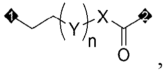 wherein position 1 is attached to L₁, and position 2 is attached to L₃.

In some embodiments, L₂ is selected from 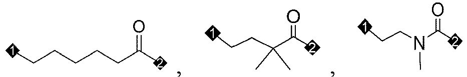 or 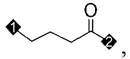 wherein position 1 is attached to L₁, and position 2 is attached to L₃.

In some embodiments, Y is selected from -CH₂- or -OCH₂CH₂-.

In some embodiments, Y is -CH₂-.

In some embodiments, n is selected from any integer between 0 and 10.

In some embodiments, n is selected from 0, 1, 2 or 3.

In some embodiments, n is selected from 1, 2 or 3.

In some embodiments, X is selected from -CR^{m}Rⁿ- or -NR^{m}-.

In some embodiments, X is selected from -CH₂-, -C(CH₃)₂-, -N(CH₃)- or -NH-.

In some embodiments, R^{m} and Rⁿ are each independently selected from hydrogen, C1-4 alkyl, C2-4 alkenyl, C2-4 alkynyl, C3-6 cycloalkyl or 3-6 membered heterocyclyl;

or R^{m} and Rⁿ, together with the carbon atom to which they are both linked, form a 3-6 membered carbocycle or a 3-6 membered heterocycle.

In some embodiments, R^{m} and Rⁿ are each independently selected from hydrogen or methyl.

In some embodiments, L₁-L₂ is selected from 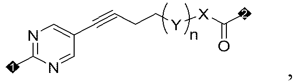 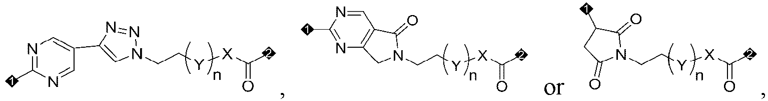 wherein position 1 is attached to Tb via an S atom, and position 2 is attached to L₃.

In some embodiments, L₁-L₂ is selected from 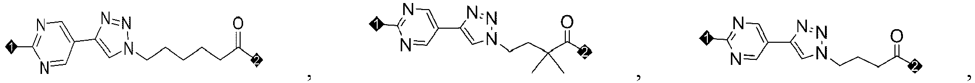 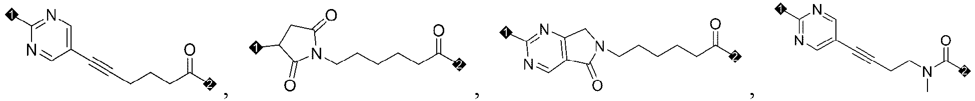 or 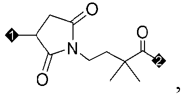 wherein position 1 is attached to Tb via an S atom, and position 2 is attached to L₃.

In some embodiments, L₁-L₂ is selected from 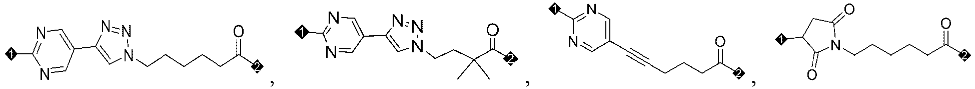 or 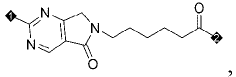 wherein position 1 is attached to Tb via an S atom, and position 2 is attached to L₃.

In some embodiments, L₃ is selected from an amino acid residue represented by AA¹ or a dipeptide, a tripeptide or a tetrapeptide comprising the amino acid residue represented by AA¹.

In some embodiments, L₃ is selected from an amino acid residue, a dipeptide or a tripeptide as shown below: AA¹, Val-Ala, Val-AA¹, Ala-AA¹, Gly-AA¹, AA¹-Gly, AA¹-Ala, Val-AA¹-Gly, Ala-AA¹-Gly, Gly-AA¹-Gly or Val-AA¹-Ala.

In some embodiments, L₃ is selected from an amino acid residue, a dipeptide or a tripeptide as shown below: AA¹, Val-AA¹, Ala-AA¹, Gly-AA¹, AA¹-Gly, AA¹-Ala, Val-AA¹-Gly, Ala-AA¹-Gly, Gly-AA¹-Gly or Val-AA¹-Ala.

In some embodiments, L₃ is selected from a dipeptide or a tripeptide as shown below: Val-AA¹, Val-AA¹-Ala or Val-AA¹-Gly.

In some embodiments, L₃ is a tripeptide represented by Val-AA¹-Gly.

In some embodiments, L₃ is selected from 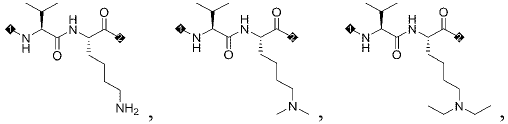 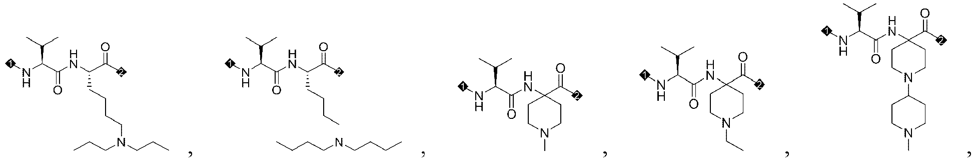 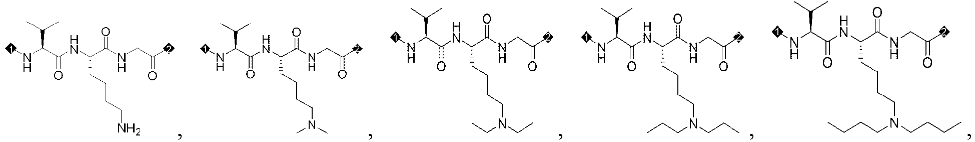 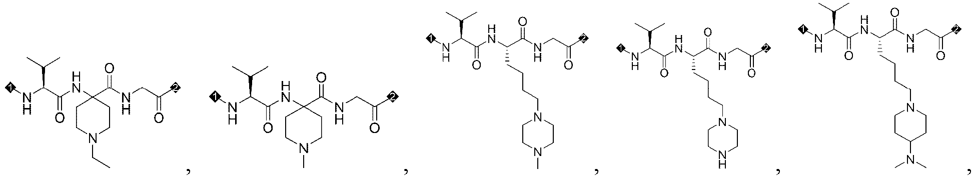 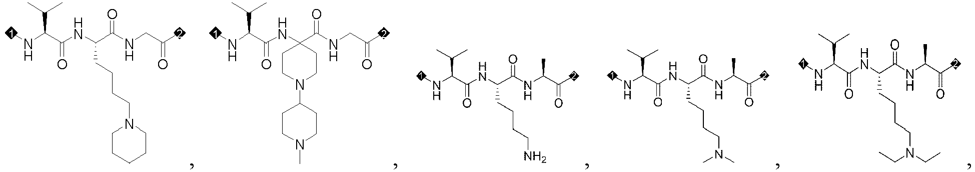 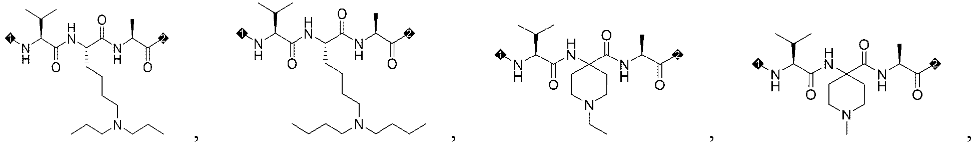 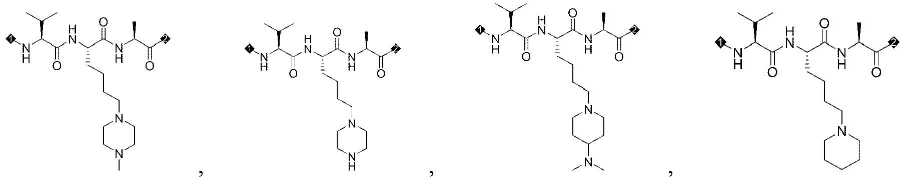 or 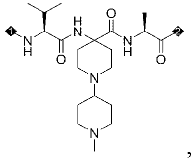 wherein position 1 is attached to L₂, and position 2 is attached to L₄ or D.

In some embodiments, L₃ is selected from 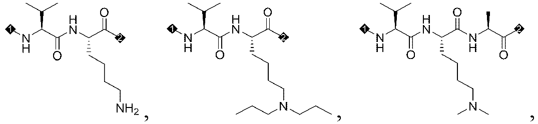 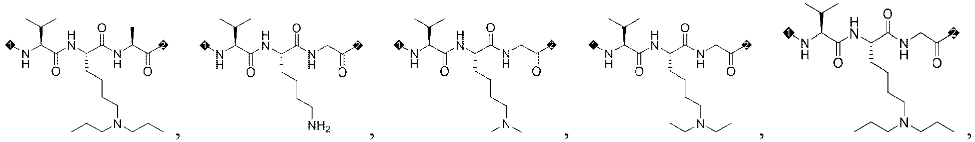 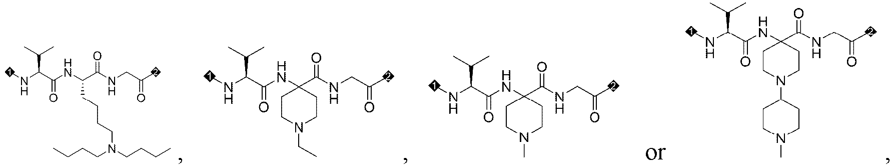 wherein position 1 is attached to L₂, and position 2 is attached to L₄ or D.

In some embodiments, L₃ is selected from 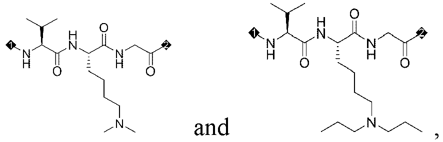 wherein position 1 is attached to L₂, and position 2 is attached to L₄ or D.

In some embodiments, one of R^{a} and R^{b} is hydrogen, and the other is

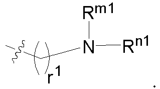

In some embodiments, R^{a} and R^{b}, together with the carbon atom to which they are both linked, form a 5-6 membered heterocycle substituted with R⁰.

In some embodiments, R^{a} and R^{b}, together with the carbon atom to which they are both linked, form a piperidine ring or piperazine ring substituted with R⁰.

In some embodiments, R^{a} and R^{b}, together with the carbon atom to which they are both linked, form a piperidine ring substituted with R⁰.

In some embodiments, R^{a} and R^{b}, together with the carbon atom to which they are both linked, form 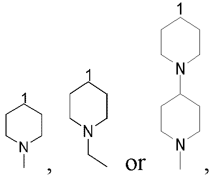 wherein the carbon atom at position 1 is the carbon atom to which R^{a} and R^{b} are both linked.

In some embodiments, r and r¹ are each independently selected from 0, 1, 2, 3, 4 or 5.

In some embodiments, r and r¹ are each independently selected from 0 or 4.

In some embodiments, r is 0, and r¹ is 4.

In some embodiments, R^{m1} and Rⁿ¹ are each independently selected from hydrogen or C1-6 alkyl.

In some embodiments, R^{m1} and Rⁿ¹ are each independently selected from C1-6 alkyl.

In some embodiments, R^{m1} and Rⁿ¹ are each independently selected from hydrogen, methyl, ethyl, *n*-propyl or *n*-butyl.

In some embodiments, R^{m1} and Rⁿ¹ are each independently selected from methyl, ethyl, *n*-propyl or n-butyl.

In some embodiments, R^{m1} and Rⁿ¹, together with the nitrogen atom to which they are both linked, form a 5-6 membered heterocycle optionally substituted with R^{0'}.

In some embodiments, R^{m1} and Rⁿ¹, together with the nitrogen atom to which they are both linked, form a piperidine ring or piperazine ring optionally substituted with R^{0'}.

In some embodiments, R^{m1} and Rⁿ¹, together with the nitrogen atom to which they are both linked, form 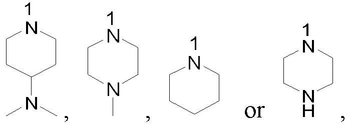 wherein the nitrogen atom at position 1 is the nitrogen atom to which R^{m1} and Rⁿ¹ are both linked.

In some embodiments, R⁰ and R^{0'} are each independently selected from C1-6 alkyl, -NR^{m2}Rⁿ², or 5-6 membered heterocyclyl optionally substituted with C1-6 alkyl.

In some embodiments, R⁰ is selected from C1-6 alkyl or 5-6 membered heterocyclyl substituted with C1-6 alkyl, and the 5-6 membered heterocyclyl is selected from piperidinyl and piperazinyl. In some embodiments, R⁰ is selected from methyl, ethyl or 5-6 membered heterocyclyl substituted with methyl, and the 5-6 membered heterocyclyl is piperidinyl.

In some embodiments, R⁰ is selected from methyl, ethyl or

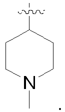

In some embodiments, R^{0'} is selected from C1-6 alkyl or -NR^{m2}Rⁿ².

In some embodiments, R^{0'} is selected from methyl or -NR^{m2}Rⁿ².

In some embodiments, R^{m2} and Rⁿ² are methyl.

In some embodiments, the amino acid residue represented by AA¹ has a structure shown below: 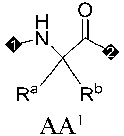 wherein:
one of R^{a} and R^{b} is H, the other is 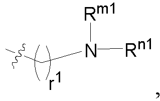 and r¹ is 4;
or R^{a} and R^{b}, together with the carbon atom to which they are both linked, form a 5-6 membered heterocycle substituted with R⁰;
R^{m1} and Rⁿ¹ are each independently selected from hydrogen, C1-6 alkyl or C3-6 cycloalkyl;
R⁰ is selected from C1-6 alkyl, C3-6 cycloalkyl, -NR^{m2}Rⁿ², or 5-6 membered heterocyclyl optionally substituted with C1-6 alkyl;
R^{m2} and Rⁿ² are each independently selected from hydrogen or C1-6 alkyl.

In some embodiments, the amino acid residue represented by AA¹ is selected from

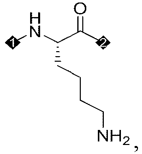

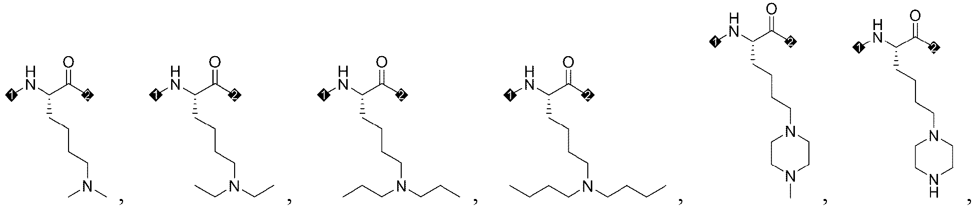

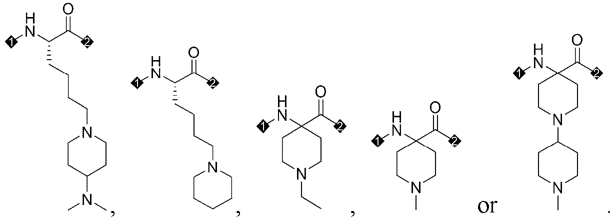

In some embodiments, the amino acid residue represented by AA¹ is selected from 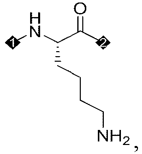 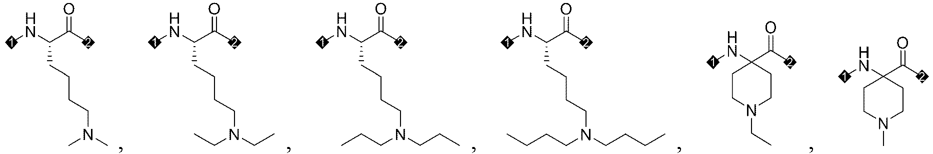 or

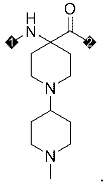

In some embodiments, the amino acid residue represented by AA¹ is selected from 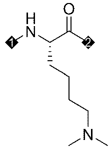 or

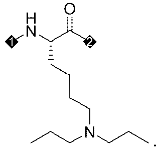

In some embodiments, L₄ is absent.

In some embodiments, L₄ is selected from 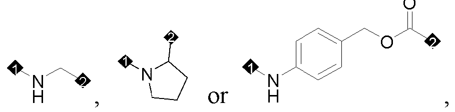 wherein position 1 is attached to L₃, and position 2 is attached to D.

In some embodiments, L₄ is selected from 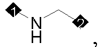 wherein position 1 is attached to L₃, and position 2 is attached to D.

In some embodiments, 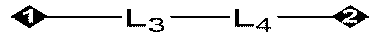 is selected from

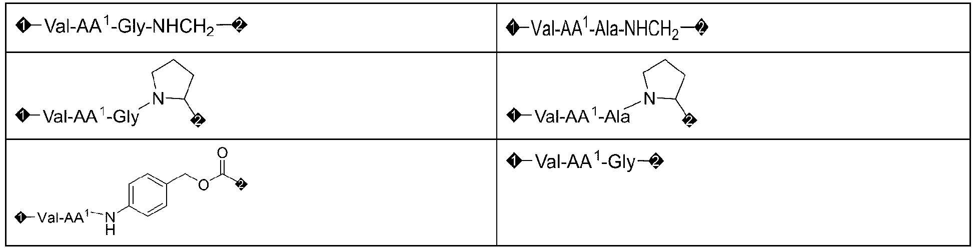

wherein position 1 is attached to L₂, position 2 is attached to D, and AA¹ is as defined in any one of the embodiments of the present disclosure.

In some embodiments, 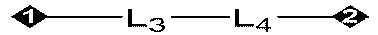 is selected from 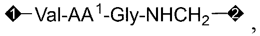 wherein position 1 is attached to L₂, position 2 is attached to D, and AA¹ is as defined in any one of the embodiments of the present disclosure.

In some embodiments, 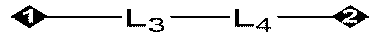 is selected from the following structures:

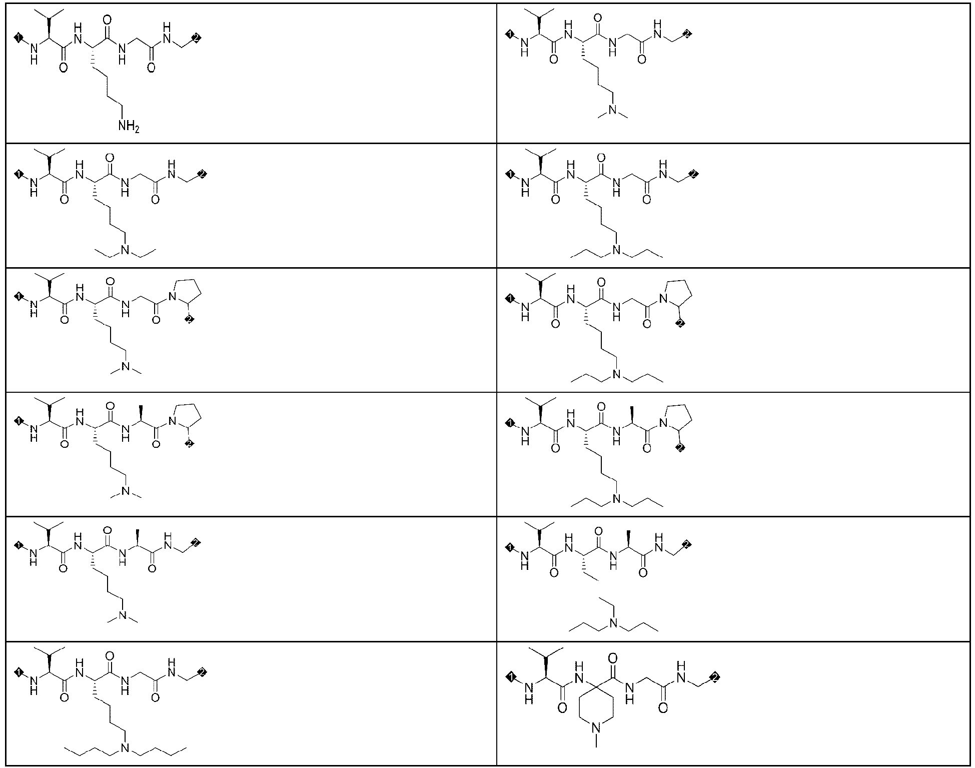

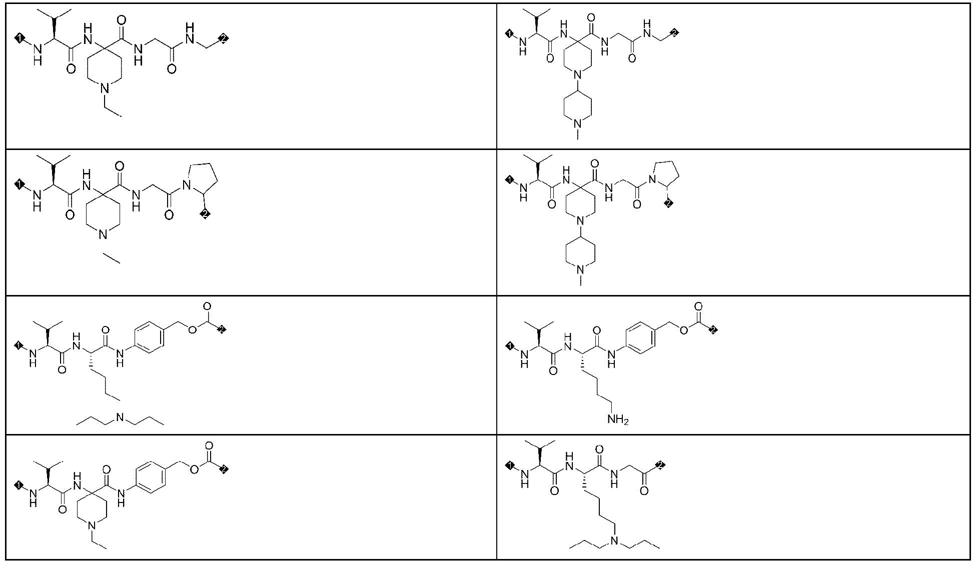

wherein position 1 is attached to L₂, and position 2 is attached to D.

In some embodiments, 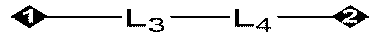 is selected from:

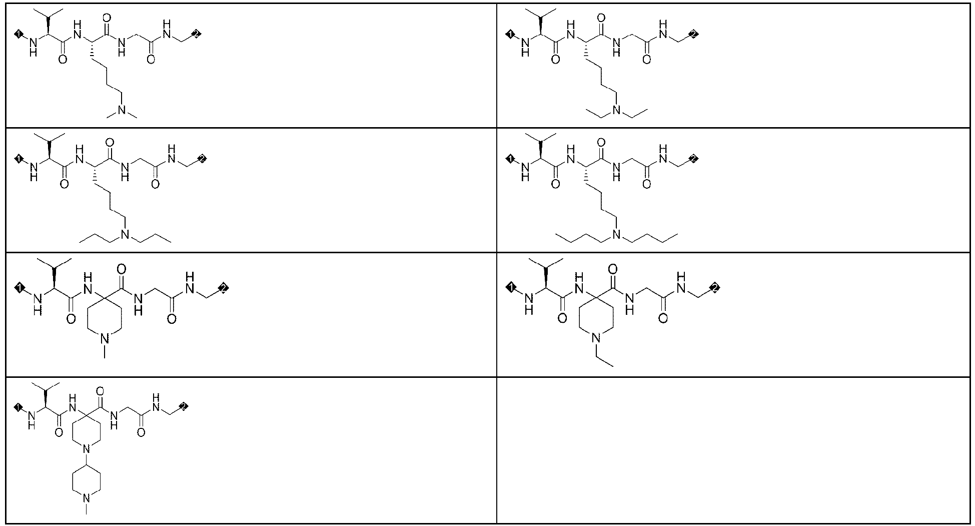

wherein position 1 is attached to L₂, and position 2 is attached to D.

In some embodiments, 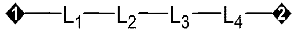 is selected from:

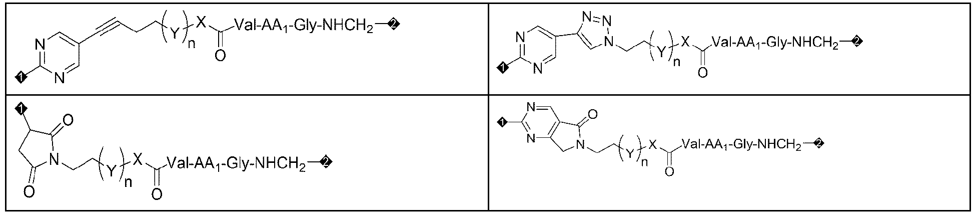

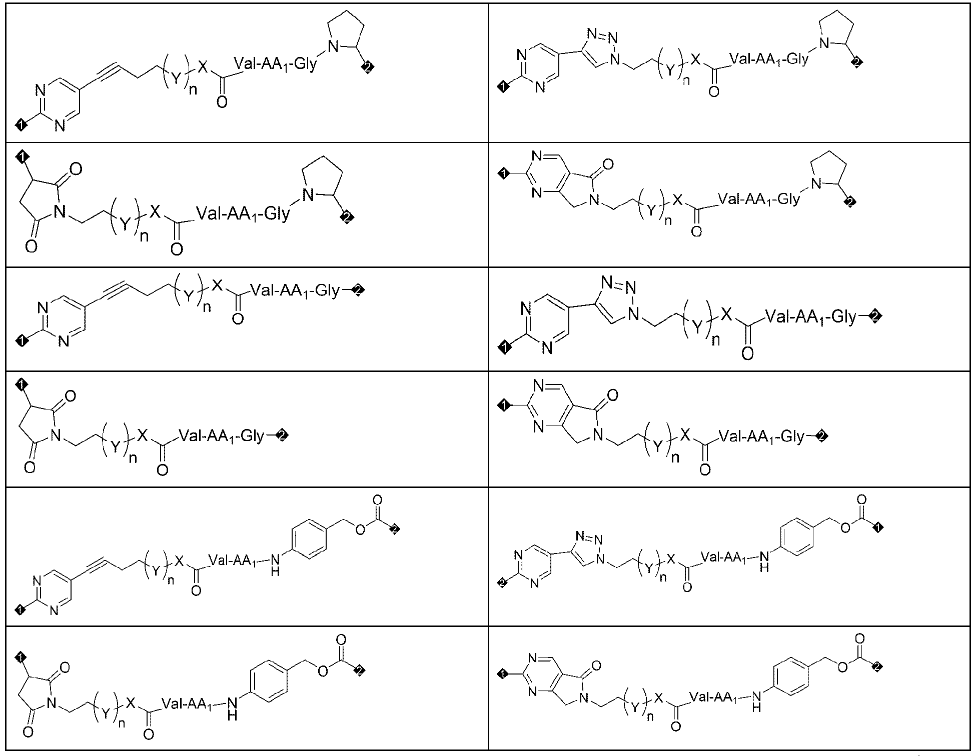

wherein position 1 is attached to Tb via an S atom, position 2 is attached to D, and Y, X, AA¹ and n are as defined in any one of the embodiments of the present disclosure.

In some embodiments, 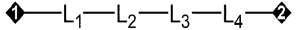 is selected from:

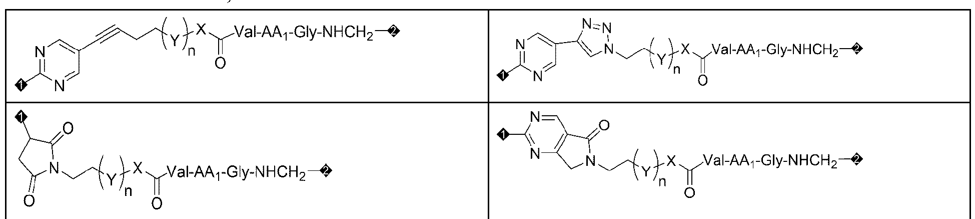

wherein position 1 is attached to Tb via an S atom, position 2 is attached to D, and Y, X, AA¹ and n are as defined in any one of the embodiments of the present disclosure.

In some embodiments, 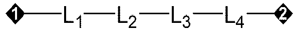 is selected from:

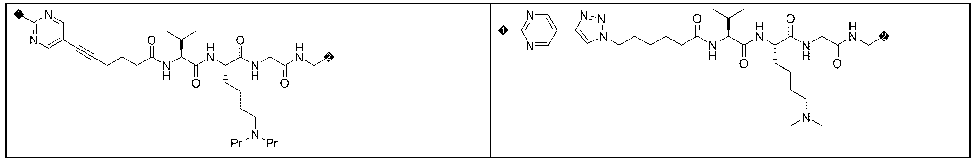

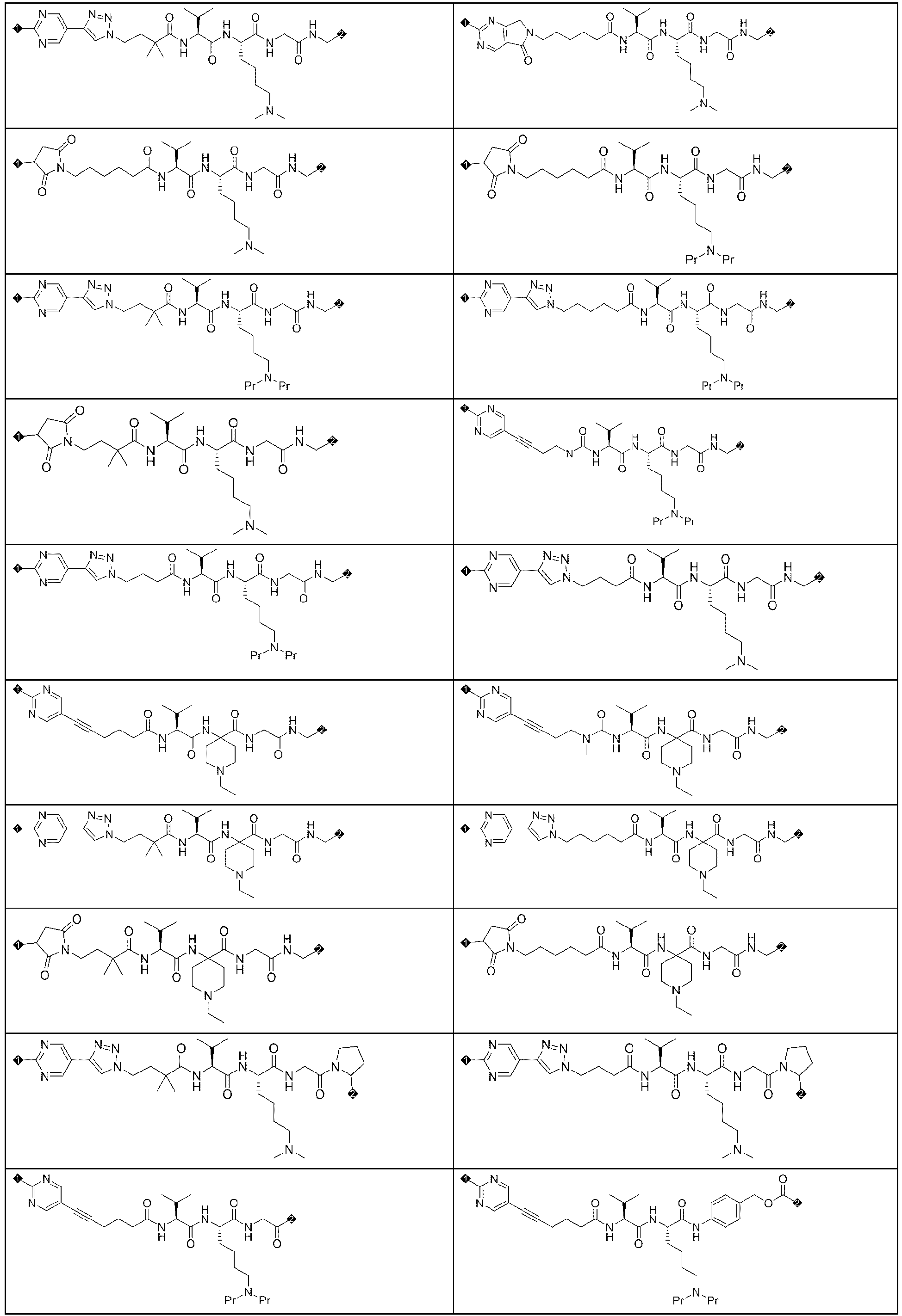

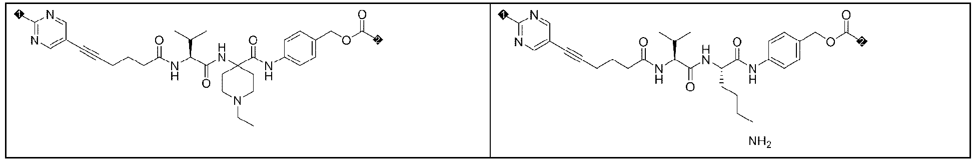

wherein position 1 is attached to Tb via an S atom, and position 2 is attached to D.

In some embodiments, 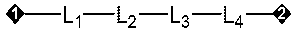 is selected from:

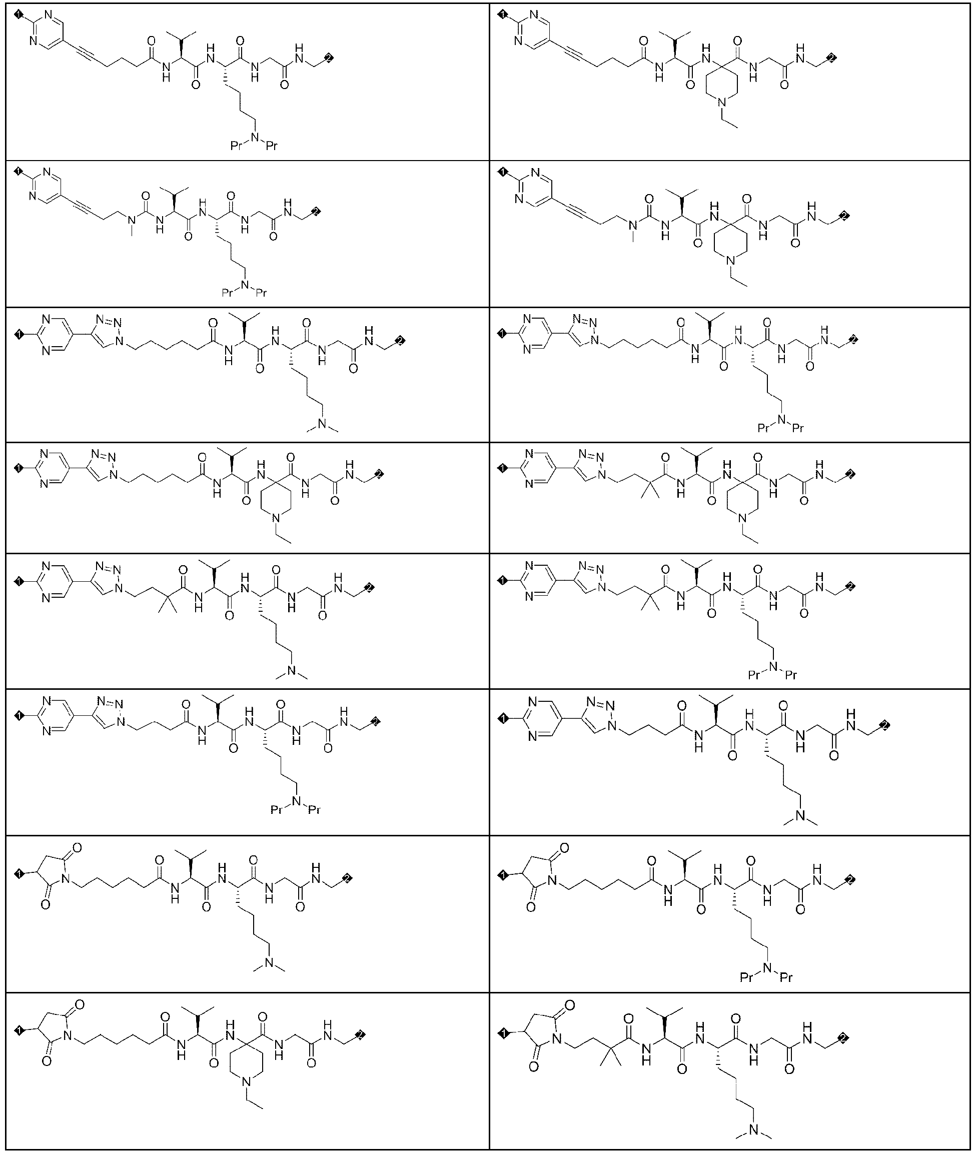

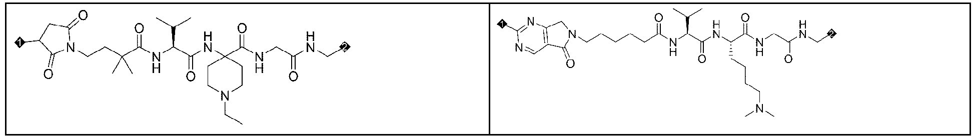

wherein position 1 is attached to Tb via an S atom, and position 2 is attached to D.

In some embodiments, D is a structural unit of formula II, and D is attached to L₄ or L₃ via A; 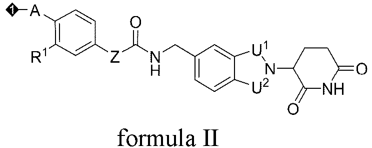 wherein,
R¹ is selected from hydrogen, deuterium, halogen, cyano, amino, nitro, C1-4 alkoxy or C1-4 alkyl;
Z is selected from -NH-, -CR^{2a}R^{3a}- or -C(O)-;
R^{2a} and R^{3a} are each independently selected from F or OH;
U¹ and U² are each independently selected from -CH₂- or -C(O)-, and U¹ and U² are not both -CH₂-;
A is selected from O, S, NR⁹, 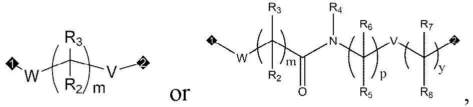 wherein position 1 is attached to L₄ or L₃, and position 2 is attached to a benzene ring; or position 1 is attached to a benzene ring, and position 2 is attached to L₄ or L₃;
R₂, R₃, R₄, R₅, R₆, R₇ and R₈ are each independently selected from hydrogen, deuterium, C1-4 alkyl, C3-6 cycloalkyl or 3-6 membered heterocyclyl;
or R₂ and R₃, together with the carbon atom to which they are linked, form a 3-7 membered carbocycle or a 3-7 membered heterocycle;
R₄ and R₅, together with the carbon atom and the nitrogen atom to which they are each linked, form a 4-7 membered heterocycle;
R₅ and R₆, together with the carbon atom to which they are linked, form a 3-7 membered carbocycle or a 3-7 membered heterocycle;
R₇ and R₈, together with the carbon atom to which they are linked, form a 3-7 membered carbocycle or a 3-7 membered heterocycle;
m, p and y are each independently selected from any integer between 0 and 8;
W and V are each independently selected from a direct bond, O, S or NR⁹;
R⁹ is selected from hydrogen, deuterium, C1-4 alkyl or C3-6 cycloalkyl.

In some embodiments, R¹ is selected from hydrogen, halogen or C1-4 alkyl.

In some embodiments, R¹ is halogen.

In some embodiments, R¹ is selected from hydrogen, methyl or chlorine.

In some embodiments, R¹ is chlorine.

In some embodiments, Z is -NH-.

In some embodiments, U¹ and U² are each independently selected from -CH₂- or -C(O)-, and U¹ and U² are different.

In some embodiments, U¹ is -CH₂-, and U² is -C(O)-.

In some embodiments, A is 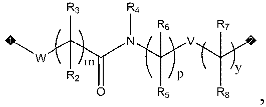 wherein position 1 is attached to L₄ or L₃, and position 2 is attached to a benzene ring; or position 1 is attached to a benzene ring, and position 2 is attached to L₄ or L₃; W, V, R₂, R₃, R₄, R₅, R₆, R₇, R₈, m, p and y are as defined in any one of the embodiments of the present disclosure.

In some embodiments, A is 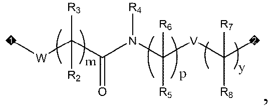 wherein position 1 is attached to L₄ or L₃, and position 2 is attached to a benzene ring; W, V, R₂, R₃, R₄, R₅, R₆, R₇, R₈, m, p and y are as defined in any one of the embodiments of the present disclosure.

In some embodiments, A is selected from O, S, -NH-, -N(CH₃)-, -O(CH₂)ₘ-, -S(CH₂)ₘ-, -NH (CH₂)ₘ-, -N(CH₃) (CH₂)ₘ-, -O(CH₂)ₘO-, -S(CH₂)ₘO-, -S(CH₂)ₘS-, -NH (CH₂)ₘO-, -N(CH₃) (CH₂)ₘO-, -NH (CH₂)ₘS-, -N(CH₃) (CH₂)ₘS-, -NH (CH₂)ₘNH-, -N(CH₃) (CH₂)ₘNH-, -O(CH₂)ₘC(O) NH (CH₂)_{P}-, -O(CH₂)ₘC(O) N(CH₃) (CH₂)_{P}-, -O(CH₂)ₘC(O) NH (CH₂)_{P}O-, -O(CH₂)ₘC(O) N(CH₃)(CH₂)_{P}O-, -S(CH₂)ₘC(O)NH (CH₂)_{P}-, -S(CH₂)ₘC(O)N(CH₃)(CH₂)_{P}-, -NH(CH₂)ₘC(O)NH (CH₂)_{P}-, -NH(CH₂)ₘC(O)N(CH₃)(CH₂)_{P}-, -O(CH₂)ₘC(O) NH (CH₂)_{P}O (CH₂)_{y}- or -O(CH₂)ₘC(O) N(CH₃)(CH₂)_{P}O(CH₂)_{y}-.

In some embodiments, A is selected from -O(CH₂)ₘC(O)NH (CH₂)_{P}-, -O(CH₂)ₘC(O) N(CH₃) (CH₂)_{P}-, -O(CH₂)ₘC(O) NH (CH₂)_{P}O-, -O(CH₂)ₘC(O) N(CH₃)(CH₂)_{P} O-, -S(CH₂)ₘC(O) NH (CH₂)_{P}-, -S(CH₂)ₘC(O)N(CH₃)(CH₂)_{P}-, -NH(CH₂)ₘC(O)NH (CH₂)_{P}-, -NH(CH₂)ₘC(O)N(CH₃) (CH₂)_{P}-, -O(CH₂)ₘC(O) NH (CH₂)_{P}O (CH₂)_{y}- or -O(CH₂)ₘC(O) N(CH₃)(CH₂)_{P}O(CH₂)_{y}-.

In some embodiments, A is selected from -N(CH₃) (CH₂)ₘ-, -O(CH₂)ₘC(O) N(CH₃) (CH₂)_{P}- or -O(CH₂)ₘC(O) N(CH₃) (CH₂)_{P}O(CH₂)_{y}-.

In some embodiments, A is selected from -O(CH₂)ₘC(O) N(CH₃) (CH₂)_{P}- or -O(CH₂)ₘC(O) N(CH₃) (CH₂)_{P}O(CH₂)_{y}-.

In some embodiments, A is selected from -N(CH₃) (CH₂)₃-, -OCH₂C(O) N(CH₃) (CH₂)₃- or -OCH₂C(O) N(CH₃) (CH₂)₂O(CH₂)₂-.

In some embodiments, A is selected from -OCH₂C(O) N(CH₃) (CH₂)₃- or -OCH₂C(O) N(CH₃) (CH₂)₂O(CH₂)₂-.

In some embodiments, R₂, R₃, R₄, R₅, R₆, R₇ and R₈ are each independently selected from hydrogen, deuterium or C1-4 alkyl.

In some embodiments, W is selected from O, S or NR⁹.

In some embodiments, W is O.

In some embodiments, V is selected from a direct bond, O, S or NR⁹.

In some embodiments, V is selected from a direct bond or O.

In some embodiments, m, p and y are each independently selected from 0, 1, 2, 3 or 4.

In some embodiments, m, p and y are each independently selected from 1, 2, 3 or 4.

In some embodiments, R₉ is selected from hydrogen, deuterium or methyl.

In some embodiments, D is preferably a structural unit of II-1, II-2 or II-3: 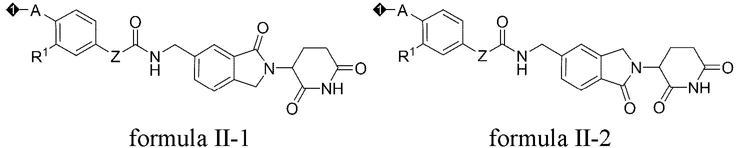 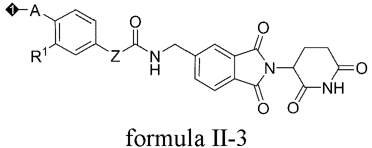 wherein,
R¹, A and Z are as defined in any one of the embodiments of the present disclosure, and position 1 is attached to L₄ or L₃.

In some embodiments, D is selected from the following structures, and position 1 is attached to L₄ or L₃:

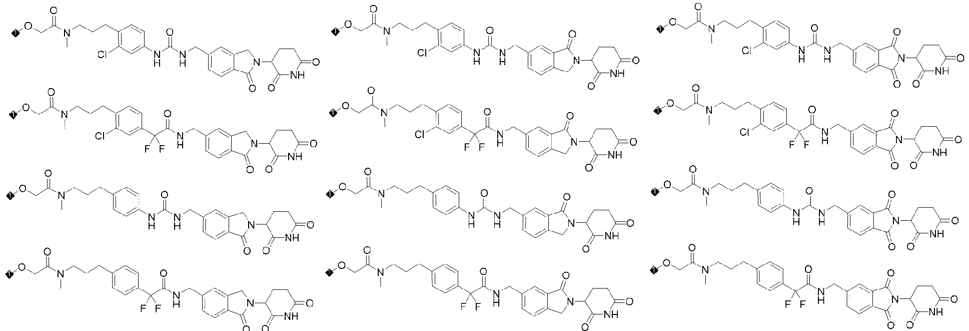

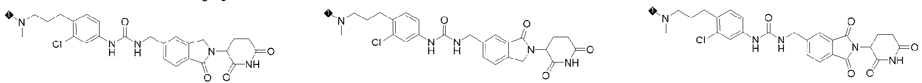

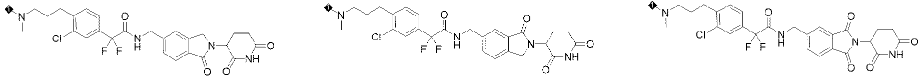

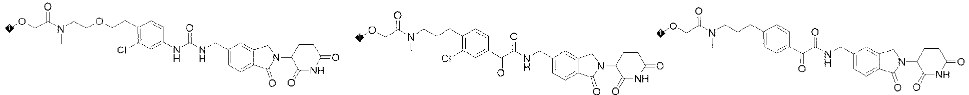

In some embodiments, D is selected from the following structures, and position 1 is attached to L₄ or L₃:

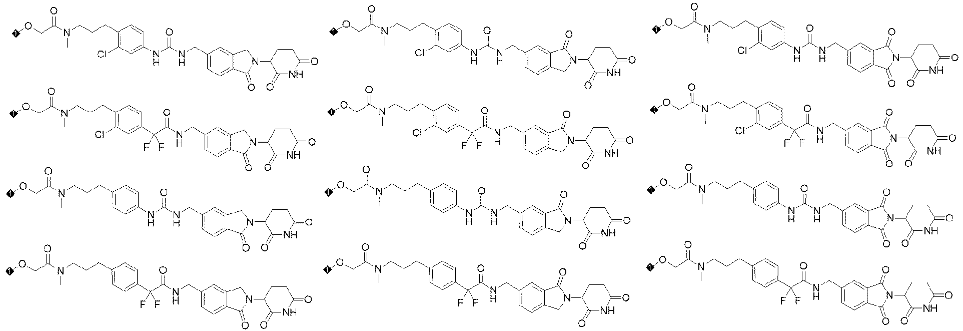

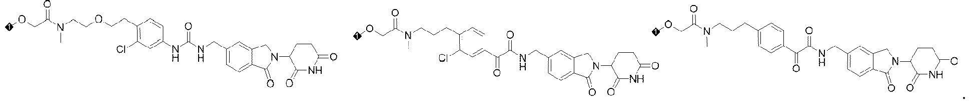

In some embodiments, the antibody-drug conjugate has a structure of formula III, 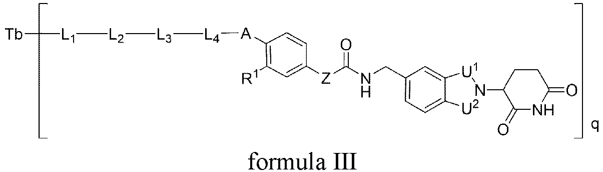 wherein Tb, q, L₁, L₂, L₃, L₄, R¹, A, Z, U¹ and U² are as defined in any one of the embodiments of the present disclosure.

In some embodiments, the antibody-drug conjugate has a structure of formula III-1: 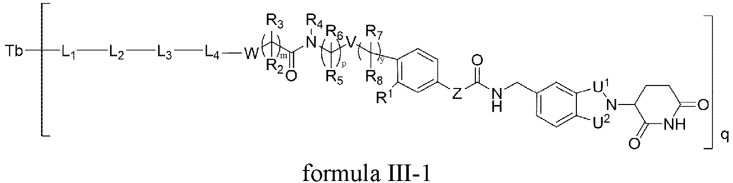 wherein Tb, L₁, L₂, L₃, L₄, W, V, R₂, R₃, R₄, R₅, R₆, R₇, R₈, Z, U¹, U², m, p, y and q are as defined in any one of the embodiments of the present disclosure.

In some embodiments, the antibody-drug conjugate is selected from:

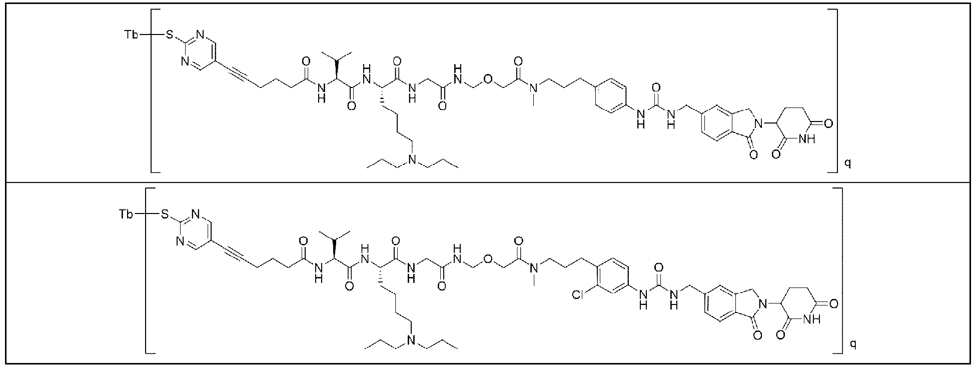

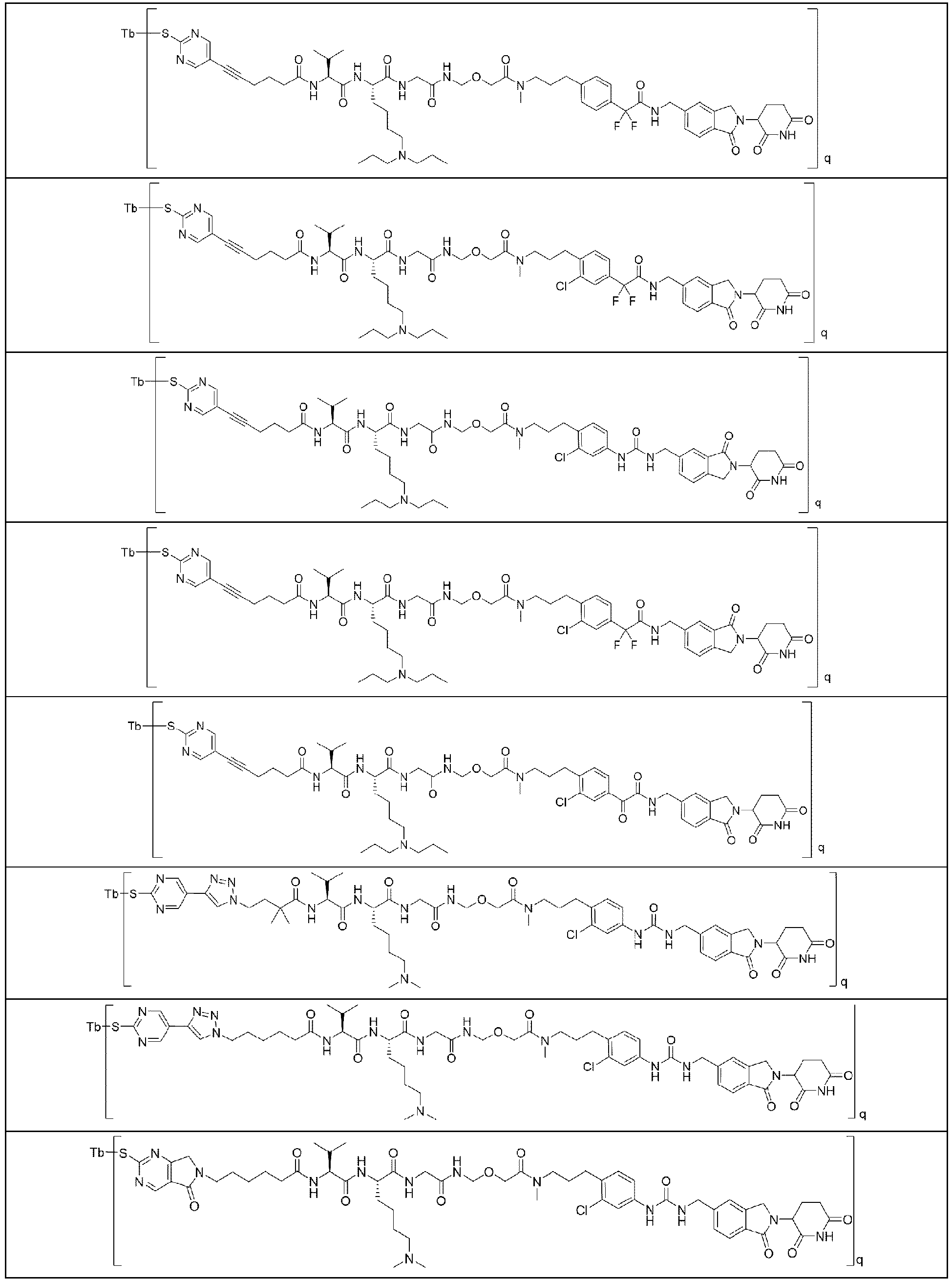

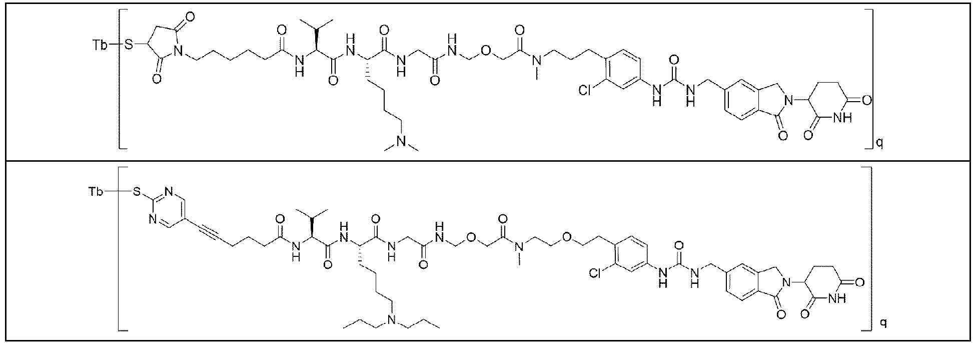

wherein q is selected from any value between 1.0 and 10.0.

In some preferred embodiments, the antibody-drug conjugate is selected from:

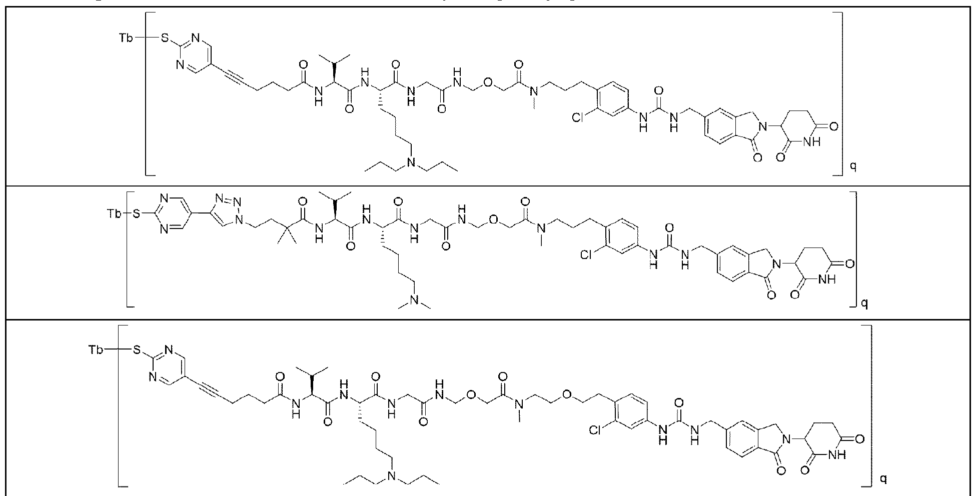

wherein q is selected from any value between 1.0 and 10.0.

In some preferred embodiments, q is selected from 1, 2, 3, 4, 5, 6, 7, 8, 9 or 10.

In some more preferred embodiments, q is selected from 2, 4, 6 or 8.

In some embodiments, Tb is a targeting moiety, such as an antibody and an antigen-binding fragment thereof, a ligand, a protein, a polypeptide, a non-protein reagent (e.g., a sugar, RNA or DNA), an antibody analog, etc.

In some embodiments, Tb is an antibody or an antigen-binding fragment thereof.

In some embodiments, Tb is an antibody or an antigen-binding fragment thereof with endocytic activity.

In some embodiments, Tb is an antibody or an antigen-binding fragment thereof with activity of binding to a tumor cell surface antigen.

In some embodiments, the antibody or the antigen-binding fragment thereof is an antibody or an antigen-binding fragment thereof with tumor cell surface antigen-binding activity and with tumor cell endocytic activity.

In some embodiments, the antibody or the antigen-binding fragment thereof is an antibody or an antigen-binding fragment thereof with antigen-binding activity and with no or weak tumor cell endocytic activity.

In some embodiments, the antibody or the antigen-binding fragment thereof is an antibody or an antigen-binding fragment thereof that binds to a tumor cell surface non-endocytic antigen.

In some embodiments, the antibody or the antigen-binding fragment thereof is an antibody or an antigen-binding fragment thereof without tumor cell endocytic activity.

In some preferred embodiments, the antibody or the antigen-binding fragment thereof is an antibody or an antigen-binding fragment thereof with activity of binding to a tumor cell surface antigen and with tumor cell endocytic activity.

In some embodiments, the antibody or the antigen-binding fragment thereof comprises a Fab, a Fab', a F(ab')2, an Fd, an Fv, a dAb, a complementarity determining region fragment, a single-chain antibody (e.g., scFv), a non-human antibody, a humanized antibody, a chimeric antibody, a fully human antibody, a probody, a bispecific antibody or a multispecific antibody.

In some embodiments, the antibody or the antigen-binding fragment thereof is a non-human antibody, a humanized antibody, a chimeric antibody or a fully human antibody.

In some embodiments, the antibody or the antigen-binding fragment thereof is a probody, a bispecific antibody or a multispecific antibody.

In some embodiments, the antibody or the antigen-binding fragment thereof comprises a Fab, a Fab', a F(ab')2, an Fd, an Fv, a dAb, a complementarity determining region fragment or a single-chain antibody (e.g., scFv).

In some embodiments, Tb is an anti-B7H3 antibody or an antigen-binding fragment thereof, an anti-Trop-2 antibody or an antigen-binding fragment thereof, an anti-Her 2 antibody or an antigen-binding fragment thereof, an anti-Her3 antibody or an antigen-binding fragment thereof, or an anti-EGFR antibody or an antigen-binding fragment thereof.

In some embodiments of the present disclosure, Tb is an anti-B7H3 monoclonal antibody or an antigen-binding fragment thereof. In some embodiments, the anti-B7H3 antibody includes all anti-B7H3 antibodies in the prior art, for example, see CN112521512, WO2021027674, WO2021021543, WO2021006619, CN111662384, CN111454357, WO2020151384, WO2020140094, WO2020103100, WO2020102779, WO2020063673, WO2020047257, WO2020041626, CN110684790, CN110642948, WO2019225787, WO2019226017, US20190338030, CN110305213, WO2018209346, WO2018177393, US9150656, WO2016106004, WO2016044383, WO2016033225, WO2015181267, US20120294796, WO2011109400, CN101104639, WO2004093894, WO2002010187 and WO2001018021.

In some embodiments, Tb is an anti-B7H3 antibody or an antigen-binding fragment thereof, such as 1D1-01, 2E3-02 antibody, enoblituzumab, mirzotamab, omburtamab, or an antigen-binding fragment thereof. In some preferred embodiments, Tb is 2E3-02 antibody.

In some embodiments, the VH sequence of 1D1-01 is set forth in SEQ ID NO: 1, and the VL sequence is set forth in SEQ ID NO: 2. In some embodiments, the heavy chain sequence of 1D1-01 is set forth in SEQ ID NO: 3, and the light chain sequence is set forth in SEQ ID NO: 4. In some embodiments, the VH sequence of 2E3-02 is set forth in SEQ ID NO: 5, and the VL sequence is set forth in SEQ ID NO: 6. In some embodiments, the heavy chain sequence of 2E3-02 is set forth in SEQ ID NO: 7, and the light chain sequence is set forth in SEQ ID NO: 8. In some embodiments, Tb is an anti-B7H3 antibody or an antigen-binding fragment thereof having a VH sequence set forth in SEQ ID NO: 1 and a VL sequence set forth in SEQ ID NO: 2.

In some preferred embodiments, Tb is an anti-B7H3 antibody or an antigen-binding fragment thereof having a heavy chain sequence set forth in SEQ ID NO: 3 and a light chain sequence set forth in SEQ ID NO: 4.

In some embodiments, Tb is an anti-B7H3 antibody or an antigen-binding fragment thereof having a VH sequence set forth in SEQ ID NO: 5 and a VL sequence set forth in SEQ ID NO: 6. In some preferred embodiments, Tb is an anti-B7H3 antibody or an antigen-binding fragment thereof having a heavy chain sequence set forth in SEQ ID NO: 7 and a light chain sequence set forth in SEQ ID NO: 8.

In some embodiments, Tb is an anti-Trop-2 antibody or an antigen-binding fragment thereof, such as datopotamab, sacituzumab, or an antigen-binding fragment thereof.

In some embodiments, Tb is an anti-Her 2 antibody or an antigen-binding fragment thereof, such as anbenitamab, coprelotamab, disitamab, gancotamab, margetuximab, pertuzumab, timigutuzumab, zanidatamab, Trastuzumab, Pertuzumab, or an antigen-binding fragment thereof. In some preferred embodiments, Tb is Trastuzumab or Pertuzumab.

In some embodiments, Tb is an anti-Her 2 antibody or an antigen-binding fragment thereof.

In some embodiments, Tb is an anti-Her 2 monoclonal antibody or an antigen-binding fragment thereof.

In some embodiments, Tb is a bispecific antibody or an antigen-binding fragment thereof with Her 2 binding activity.

In some embodiments, Tb is a multispecific antibody or an antigen-binding fragment thereof with Her 2 binding activity.

In some preferred embodiments, Tb is Trastuzumab or an antigen-binding fragment thereof.

In some preferred embodiments, Tb is Pertuzumab or an antigen-binding fragment thereof.

In some embodiments, Tb is an anti-Her3 antibody or an antigen-binding fragment thereof.

In some embodiments, Tb is an anti-EGFR antibody or an antigen-binding fragment thereof, such as demupitamab, depatuxizumab, futuximab, imgatuzumab, laprituximab, losatuxizumab, matuzumab, modotuximab, necitumumab, nimotuzumab, panitumumab, pimurutamab, serclutamab, tomuzotuximab, zalutumumab, Cetuximab, or an antigen-binding fragment thereof. In some embodiments, the antibody is an antibody with activity of binding to B7H3 antigen and without endocytic activity, such as INV721 and I7-01 in WO2021168379A1. More specifically, the anti-B7H3 antibody has a VH of SEQ ID NO: 2 and a VL of SEQ ID NO: 1 described in WO2021168379A1.

In some embodiments, the antibody is an antibody with activity of binding to CD20 antigen and without endocytic activity. Although so-called "type II" CD20-specific antibodies have been shown to be poorly internalized by CD20-positive target cells, other so-called "type I" CD20-specific antibodies have been found to be internalized and degraded to some extent, depending on activation and inhibition of Fc γR expression levels in the target cells with which they interact. In some embodiments, the antibody with the activity of binding to CD20 antigen and without endocytic activity is a "type II" CD20-specific antibody, such as obinutuzumab.

In some embodiments, the antibody is an antibody with activity of binding to a non-endocytic antigen (e.g., ALCAM/CD166). In some embodiments, the antibody is an antibody comprising a VH of SEQ ID NO: 73 and a VL of SEQ ID NO: 74, a VH of SEQ ID NO: 75 and a VL of SEQ ID NO: 76, a VH of SEQ ID NO: 77 and a VL of SEQ ID NO: 78, or a VH of SEQ ID NO: 79 and a VL of SEQ ID NO: 88 in EP3911682A1.

In some embodiments, the antibody-drug conjugate is: 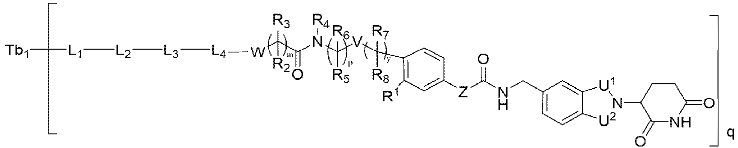 wherein Tb₁ is an anti-B7H3 antibody or an antigen-binding fragment thereof; L₁, L₂, L₃, L₄, W, V, R₂, R₃, R₄, R₅, R₆, R₇, R₈, Z, U¹, U², m, p, y and q are as defined in any one of the embodiments of the present disclosure.

In some preferred embodiments, Tb₁ is 2E3-02 antibody.

In some embodiments, the antibody-drug conjugate is: 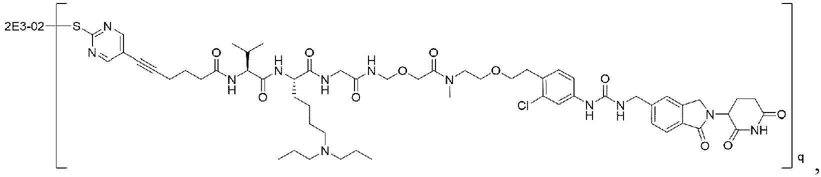 and the DAR value is 8.0.

In some embodiments, the antibody-drug conjugate is: 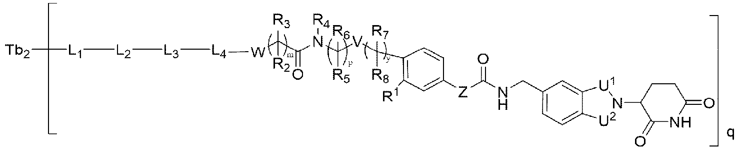 wherein Tb₂ is an anti-Her 2 antibody or an antigen-binding fragment thereof; L₁, L₂, L₃, L₄, W, V, R₂, R₃, R₄, R₅, R₆, R₇, R₈, Z, U¹, U², m, p, y and q are as defined in any one of the embodiments of the present disclosure.

In some preferred embodiments, Tb₂ is Trastuzumab.

In some embodiments, the antibody-drug conjugate is: 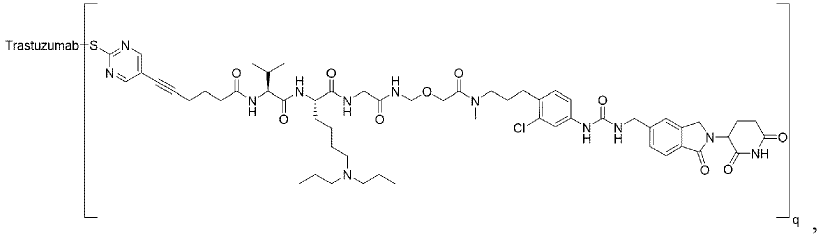 and the DAR value is 8.0.

In some embodiments, the antibody-drug conjugate is: 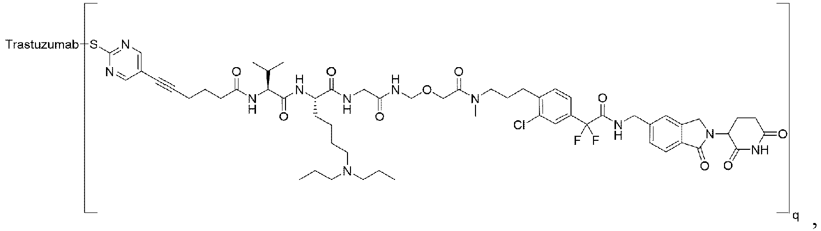 and the DAR value is 8.0.

In some embodiments, the antibody-drug conjugate is: 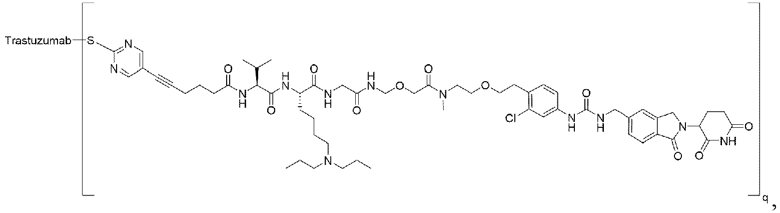 and the DAR value is 8.0.

In some embodiments, the antibody-drug conjugate is: 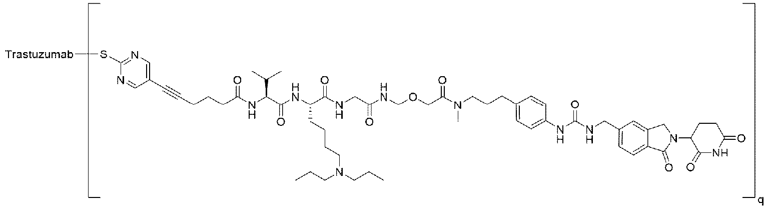 and the DAR value is 7.0.

In some embodiments, the antibody-drug conjugate is: 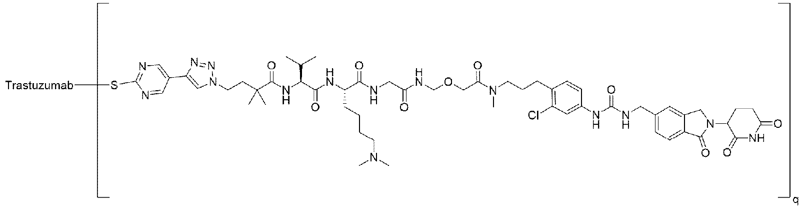 and the DAR value is 8.0.

In a second aspect of the present disclosure, the present disclosure provides a drug-linker conjugate of formula IV,

Lg-L₁-L₂-L₃-L₄-D formula IV

or a stereoisomer of the drug-linker conjugate, a prodrug thereof, a pharmaceutically acceptable salt thereof or a pharmaceutically acceptable solvate thereof,
wherein,
when L₁ is 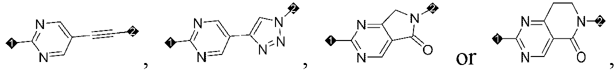 position 1 is attached to Lg, and position 2 is attached to L₂;
Lg is a leaving group when reacting with an antibody; L₂, L₃, L₄ and D are as defined in any one of the embodiments of the present disclosure;
when L₁ is 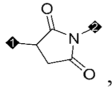 Lg-L₁ is 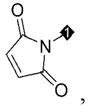 and L₂, L₃, L₄ and D are as defined in any one of the embodiments of the present disclosure.

In some embodiments, Lg is selected from halogen, sulfonyl, a tertiary amine salt group (Me₃N⁺ or Et₃N⁺), a diazonium salt group, -OMs, MeSO₂- or CF₃SO₃-.

In some embodiments, Lg is selected from F, Cl or MeSO₂-; more preferably, Lg is selected from F or MeSO₂-.

In some embodiments, the drug-linker conjugate has a structure of formula V, 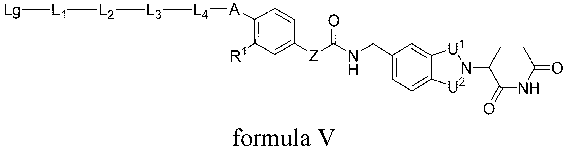 wherein Lg, L₁, L₂, L₃, L₄, R¹, A, Z, U¹ and U² are as defined in any one of the embodiments of the present disclosure.

In some embodiments, the drug-linker conjugate has a structure of formula V-1: 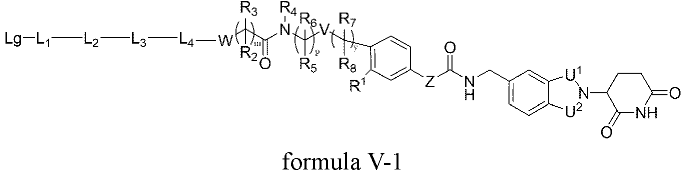 wherein Lg, L₁, L₂, L₃, L₄, W, V, R₂, R₃, R₄, R₅, R₆, R₇, R₈, Z, U¹, U², m, p and y are as defined in any one of the embodiments of the present disclosure.

In some embodiments, the drug-linker conjugate is selected from:

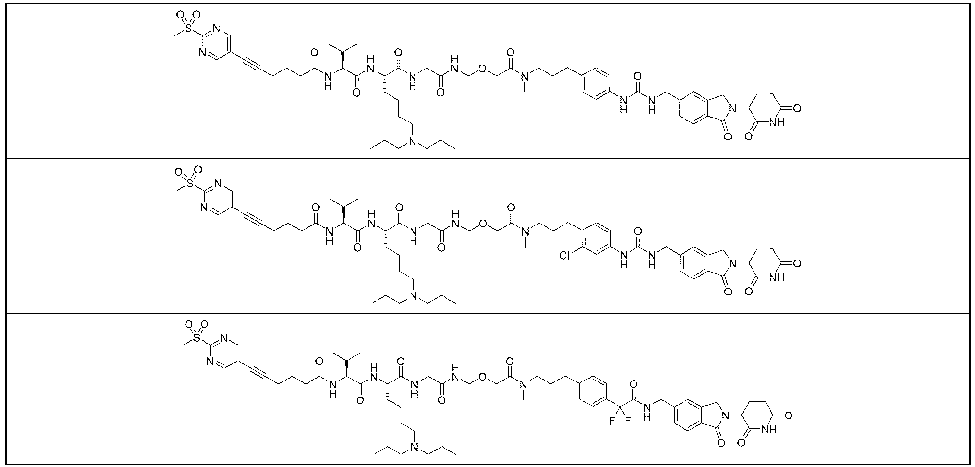

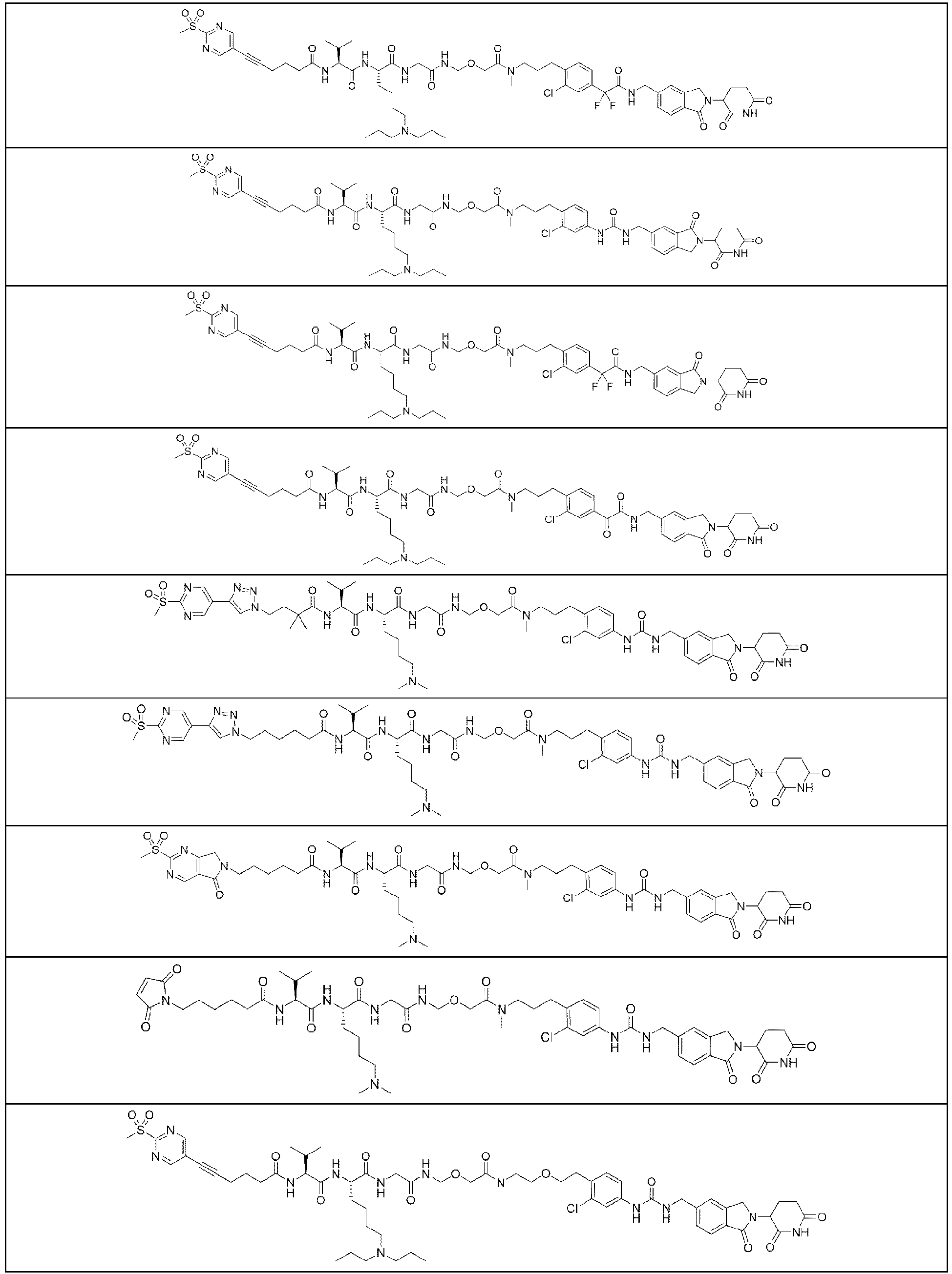

In a third aspect of the present disclosure, the present disclosure provides a protein degradation agent bioactive compound of formula IIA, 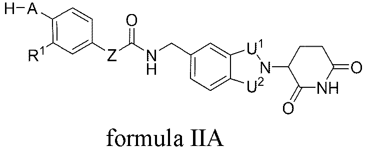
or a stereoisomer of the bioactive compound, a prodrug thereof, a pharmaceutically acceptable salt thereof or a pharmaceutically acceptable solvate thereof,
wherein R¹, A, Z, U¹ and U² are as defined in any one of the embodiments of the present disclosure.

In some embodiments, the protein degradation agent bioactive compound has a structure of formula IIA-1: 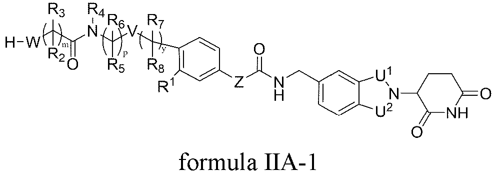 wherein W, V, R₂, R₃, R₄, R₅, R₆, R₇, R₈, Z, U¹, U², m, p and y are as defined in any one of the embodiments of the present disclosure.

In some embodiments, the bioactive compound is selected from:

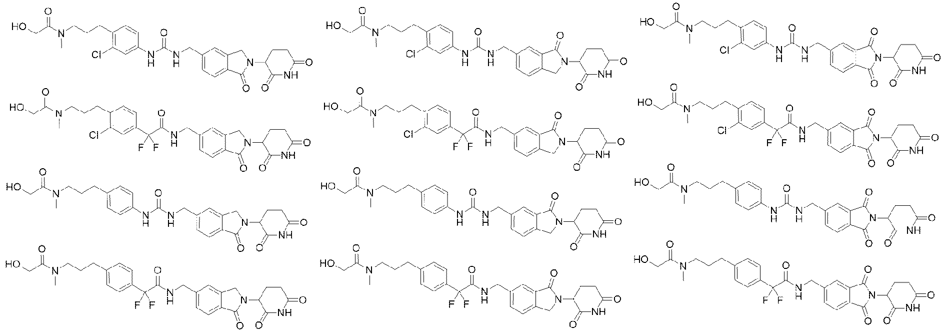

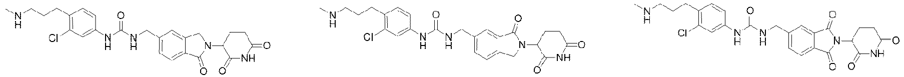

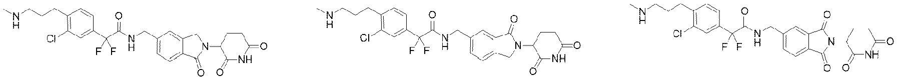

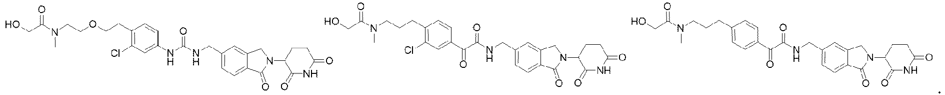

In a fourth aspect of the present disclosure, the present disclosure provides a linker in an antibody-drug conjugate, which has a structure shown below:

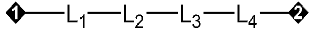

Wherein position 1 is attached to an antibody, and position 2 is attached to a protein degradation agent bioactive compound fragment; L₁, L₂, L₃ and L₄ are as defined in any one of the embodiments in the present disclosure;
preferably, the antibody and the protein degradation agent bioactive compound fragment are defined as Tb and D, respectively, in any one of the embodiments in the present disclosure.

In a fifth aspect of the present disclosure, the present disclosure provides a method for preparing an antibody-drug conjugate of formula I, comprising:
subjecting Tb and a drug-linker conjugate of formula IV Lg-L₁-L₂-L₃-L₄-D to a coupling reaction;
wherein,
Tb, L₁, L₂, L₃, L₄, Lg and D are as defined in any one of the embodiments in the present disclosure.

Specifically, the method comprises a step of subjecting Tb and the drug-linker conjugate of formula IV Lg-L₁-L₂-L₃-L₄-D to the coupling reaction in a suitable solvent and under suitable conditions to form a C-S bond.

In some embodiments, Tb and the drug-linker conjugate are in a molar ratio of 1:(1-20), such as 1:2, 1:4, 1:6, 1:8, 1:10, 1:12, 1:14, 1:16, 1:18, 1:(10-20), 1:(12-20), 1:(14-20), 1:(16-20) or 1:(18-20).

In some embodiments, the coupling reaction is carried out in water and/or an organic solvent.

In some embodiments, the organic solvent is selected from N,N-dimethylformamide, dimethyl sulfoxide, N-methylpyrrolidone, nitriles (e.g., acetonitrile), alcohols (e.g., methanol, ethanol) or any combination thereof.

In some embodiments, the method further comprises a step of purifying the coupled product.

In some embodiments, the coupled product is purified by chromatography.

In some embodiments, the chromatography comprises one or more of ion exchange chromatography, hydrophobic chromatography, reversed-phase chromatography or affinity chromatography.

In a sixth aspect of the present disclosure, the present disclosure provides a method for preparing a drug-linker conjugate of formula IV, comprising:
subjecting a compound of general formula IV-E-1 and a compound of general formula IV-E-2 to a reaction shown below; 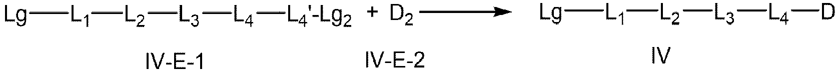
wherein L₄' is 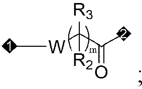 W, R₂, R₃ and m are as defined in any one of the embodiments in the present disclosure; position 1 is attached to L₄, and position 2 is attached to Lg₂;
Lg₂ is selected from hydroxy, halogen, activated hydroxy, such as hydroxy, chlorine, bromine, 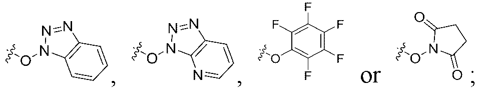
D₂ is a structure of formula VI, and R₄, R₅, R₆, R₇, R₈, V, Z, U¹, U², p and y are as defined in any one of the embodiments in the present disclosure.

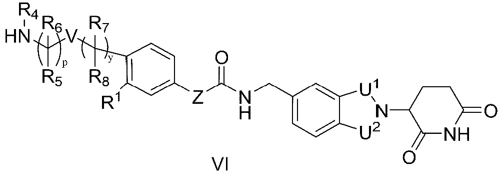

Lg, L₁, L₂, L₃ and L₄ are as defined in any one of the embodiments in the present disclosure.

In some embodiments, the present disclosure provides a method for preparing a drug-linker conjugate of formula V-1, comprising: subjecting a compound of general formula V-A-1 and a compound of general formula VI to a reaction shown below: 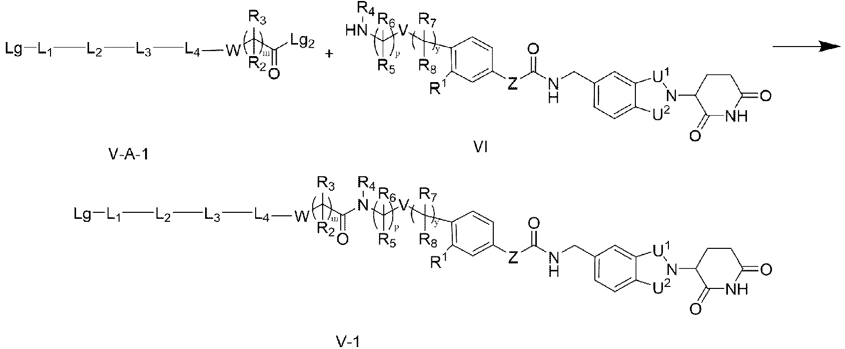 wherein Lg, Lg₂, L₁, L₂, L₃, L₄, W, V, R₂, R₃, R₄, R₅, R₆, R₇, R₈, V, Z, U¹, U², m, p and y are as defined in any one of the embodiments of the present disclosure.

In some embodiments, the present disclosure provides a method for preparing a drug-linker conjugate of formula DL-1.

Specifically,
a compound of general formula INT1 and compound DL-1-1 are subjected to an amide condensation reaction to give a target compound (DL-1). Drug-linker conjugates with various structures can be obtained by coupling analogs of INT1 to analogs of DL-1-1.

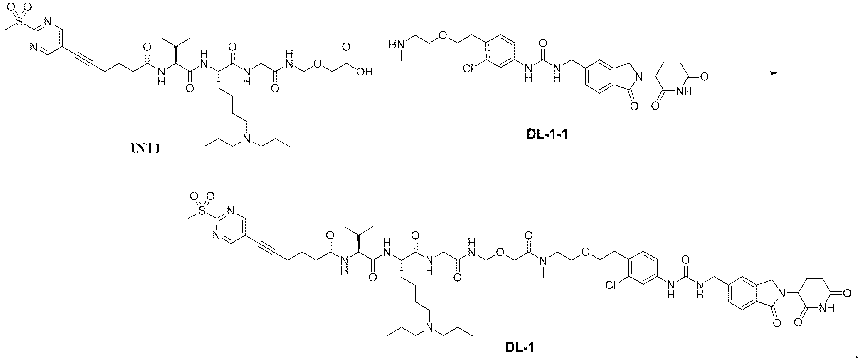

In a seventh aspect of the present disclosure, the present disclosure provides a linker of formula V-A-1, 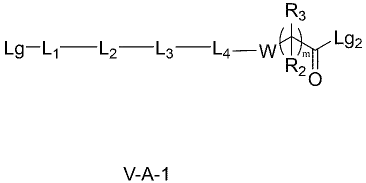 wherein Lg, L₁, L₂, L₃, L₄, W, R₂, R₃ and m are as defined in any one of the embodiments of the present disclosure, and Lg₂ is selected from hydroxy, halogen, activated hydroxy, such as hydroxy, chlorine, bromine,

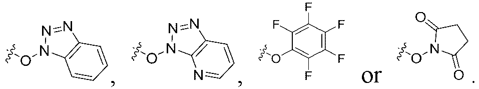

In some embodiments, the linker is selected from:

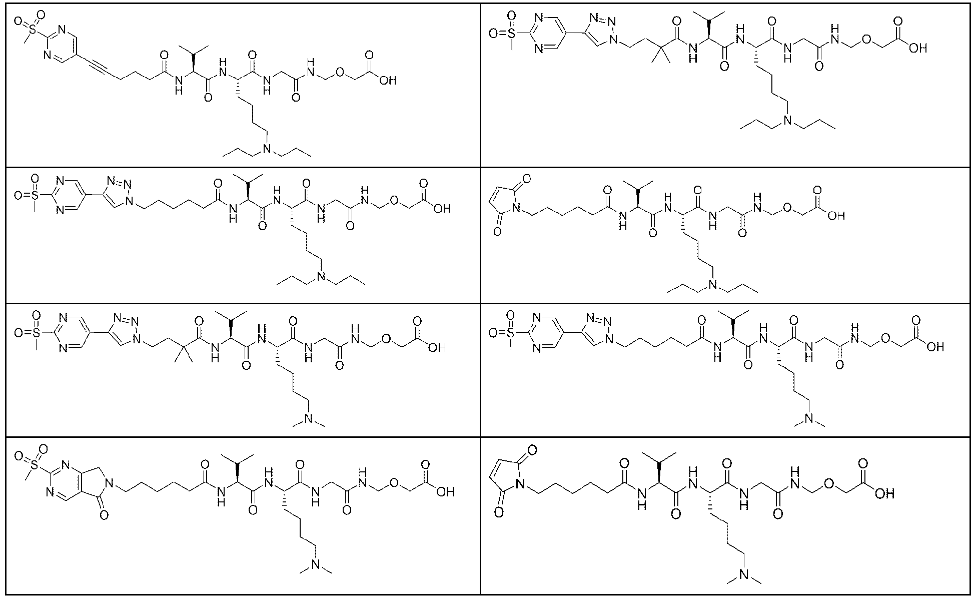

The present disclosure provides use of the aforementioned protein degradation agent bioactive compound or the stereoisomer of the compound, the prodrug thereof, the pharmaceutically acceptable salt thereof or the pharmaceutically acceptable solvate thereof in the preparation of an antibody-drug conjugate (ADC).

The present disclosure provides a pharmaceutical composition comprising the aforementioned antibody-drug conjugate or the stereoisomer of the antibody-drug conjugate, the prodrug thereof, the pharmaceutically acceptable salt thereof or the pharmaceutically acceptable solvate thereof; or the aforementioned protein degradation agent bioactive compound or the stereoisomer of the bioactive compound, the prodrug thereof, the pharmaceutically acceptable salt thereof or the pharmaceutically acceptable solvate thereof; or the aforementioned drug-linker conjugate or the stereoisomer of the drug-linker conjugate, the prodrug thereof, the pharmaceutically acceptable salt thereof or the pharmaceutically acceptable solvate thereof, and optionally one or more pharmaceutical adjuvants.

In some embodiments, the drug-to-antibody ratio (DAR) is any value between 1.0 and 12.0.

In some embodiments, the drug-to-antibody ratio (DAR) is any value between 1.0 and 10.0.

In some embodiments, the drug-to-antibody ratio (DAR) is any value between 1.0 and 9.0.

In some embodiments, the drug-to-antibody ratio (DAR) is any value between 1.0 and 8.5.

In some embodiments, the drug-to-antibody ratio (DAR) is any value between 1.5 and 8.5.

In some embodiments, the drug-to-antibody ratio (DAR) is any value between 2.0 and 8.0.

In some embodiments, the drug-to-antibody ratio (DAR) is any value within 2±0.4, 4±0.4, 6±0.4 or 8±0.4.

In some embodiments, the drug-to-antibody ratio (DAR) is about 2.0, 4.0, 6.0 or 8.0.

The term "drug-to-antibody ratio" or "DAR" refers to the number of drugs, e.g., small molecule toxins attached to an antibody of an ADC. The DAR of an ADC may be in the range of 1 to 16, but a higher load (e.g., 20) is also possible depending on the number of linking sites on the antibody. The term DAR may be used when referring to the number of drugs loaded onto a single antibody, or alternatively, when referring to an average or mean DAR for a set of ADCs.In some embodiments, the ADC comprises an ADC having a DAR distribution from 1 to 8, such as 1.5, 2, 4, 6 and 8 (i.e., drug-loaded species of 1.5, 2, 4, 6 and 8). Notably, degradation products may be produced, such that the ligand-drug conjugate may also comprise a DAR of 1, 3, 5 and 7. In addition, the ADC may also have a DAR greater than 8. The ADC is produced by the reduction of an inter-chain disulfide followed by coupling. In some embodiments, the ADC comprises the following two: an ADC with a DAR of 4 or less (i.e., drug-loaded species of 4 or less) and an ADC with a DAR of 6 or more (i.e., drug-loaded species of 6 or more).

The present disclosure provides use of the aforementioned antibody-drug conjugate or the stereoisomer of the antibody-drug conjugate, the prodrug thereof, the pharmaceutically acceptable salt thereof or the pharmaceutically acceptable solvate thereof; or the aforementioned protein degradation agent bioactive compound or the stereoisomer of the bioactive compound, the prodrug thereof, the pharmaceutically acceptable salt thereof or the pharmaceutically acceptable solvate thereof; or the aforementioned drug-linker conjugate or the stereoisomer of the drug-linker conjugate, the prodrug thereof, the pharmaceutically acceptable salt thereof or the pharmaceutically acceptable solvate thereof; or the aforementioned pharmaceutical composition in the preparation of a medicament for treating and/or preventing a disease associated with abnormal cell activity (e.g., a cancer disease).

The present disclosure provides the aforementioned antibody-drug conjugate or the stereoisomer of the antibody-drug conjugate, the prodrug thereof, the pharmaceutically acceptable salt thereof or the pharmaceutically acceptable solvate thereof; or the aforementioned protein degradation agent bioactive compound or the stereoisomer of the bioactive compound, the prodrug thereof, the pharmaceutically acceptable salt thereof or the pharmaceutically acceptable solvate thereof; or the aforementioned drug-linker conjugate or the stereoisomer of the drug-linker conjugate, the prodrug thereof, the pharmaceutically acceptable salt thereof or the pharmaceutically acceptable solvate thereof; or the aforementioned pharmaceutical composition for use in the treatment and/or prevention of a disease associated with abnormal cell activity (e.g., a cancer disease).

The present disclosure provides a method for preventing and/or treating a disease associated with abnormal cell activity (e.g., a cancer disease), comprising: administering to an individual in need thereof a prophylactically and/or therapeutically effective amount of the aforementioned antibody-drug conjugate or the stereoisomer of the antibody-drug conjugate, the prodrug thereof, the pharmaceutically acceptable salt thereof or the pharmaceutically acceptable solvate thereof; or the aforementioned protein degradation agent bioactive compound or the stereoisomer of the bioactive compound, the prodrug thereof, the pharmaceutically acceptable salt thereof or the pharmaceutically acceptable solvate thereof; or the aforementioned drug-linker conjugate or the stereoisomer of the drug-linker conjugate, the prodrug thereof, the pharmaceutically acceptable salt thereof or the pharmaceutically acceptable solvate thereof; or the aforementioned pharmaceutical composition.

In some embodiments, the cancer disease described herein is selected from esophageal cancer (e.g., esophageal adenocarcinoma or esophageal squamous cell carcinoma), brain tumor, lung cancer (e.g., small cell lung cancer or non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, urothelial cancer, solid tumors, non-Hodgkin's lymphoma, central nervous system tumors (e.g., neuroglioma, glioblastoma multiforme, glioma or sarcoma), prostate cancer or thyroid cancer.

In some embodiments, the cancer disease is a cancer disease associated with HER2, TROP2, B7H3, HER3 or EGFR.

In some embodiments, the cancer disease is a solid tumor or a hematological tumor.

In the present disclosure, unless otherwise indicated, scientific and technical terms used in the present disclosure have the meanings generally understood by those skilled in the art. In addition, the laboratory operations of cell culture, molecular genetics, nucleic acid chemistry and immunology used in the present disclosure are the conventional procedures widely used in the corresponding fields. Meanwhile, in order to better understand the present disclosure, definitions and explanations of related terms are provided below.

As used herein, the term "protein degradation agent" refers to a class of compounds that are capable of inducing and ubiquitinating proteins of interest with E3 ligases and then degrading the proteins with proteasomes.

As used herein, the term "proteolysis targeting chimera (PROTAC)" generally comprises three parts: an E3 ubiquitin ligase ligand, a target protein ligand, and a specially designed Linker structure for linking the two active ligands, wherein the E3 ubiquitin ligase is recruited on the target protein to ubiquitinate the target protein, thereby degrading the target protein.

As used herein, the term "molecular glue degrader (MGD)" refers to a class of small molecules that can induce a novel interaction between an E3 ubiquitin ligase and a target protein, leading to degradation of the target protein.

As used herein, examples of the term "pharmaceutically acceptable salt" are organic acid addition salts formed from organic acids that form pharmaceutically acceptable anions, including but not limited to formate, acetate, propionate, benzoate, maleate, fumarate, succinate, tartrate, citrate, ascorbate, α-ketoglutarate, α-glycerophosphate, alkylsulfonate or arylsulfonate; preferably, the alkylsulfonate is methylsulfonate or ethylsulfonate; the arylsulfonate is benzene sulfonate or p-toluenesulfonate. Suitable inorganic salts may also be formed, including but not limited to hydrochloride, hydrobromide, hydriodate, nitrate, bicarbonate and carbonate, sulfate or phosphate, etc.

Pharmaceutically acceptable salts may be obtained using standard procedures well known in the art, for example, by reacting a sufficient amount of an alkaline compound with a suitable acid providing a pharmaceutically acceptable anion.

As used herein, the term "stereoisomer" refers to an isomer formed due to at least one asymmetric center. In compounds having one or more (e.g., one, two, three or four) asymmetric centers, racemic mixtures, single enantiomers, mixtures of diastereoisomers and individual diastereomers may be produced. Certain individual molecules may also exist as geometric isomers (cis/trans). Similarly, the compound of the present invention may exist as a mixture of two or more structurally different forms in rapid equilibrium (commonly referred to as tautomers). Representative examples of tautomers include keto-enol tautomers, phenol-keto tautomers, nitroso-oxime tautomers, imine-enamine tautomers, etc. It should be understood that the scope of the present disclosure encompasses all such isomers or mixtures thereof in any proportion (e.g., 60%, 65%, 70%, 75%, 80%, 85%, 90%, 95%, 96%, 97%, 98% or 99%).

A solid line (-), a solid wedge or a dashed wedge may be used in the present disclosure to depict carbon-carbon bonds of the compound of the present invention. Using the solid line to depict a bond that is bonded to an asymmetric carbon atom is intended to indicate that all possible stereoisomers (e.g., particular enantiomers, racemic mixtures, etc.) at the carbon atom are included. Using the solid or dotted wedge to depict a bond that is bonded to an asymmetric carbon atom is intended to indicate that the stereoisomers shown are present. When present in a racemic mixture, the solid and dashed wedges are used to define the relative stereochemistry rather than the absolute stereochemistry. Unless otherwise indicated, the compound of the present invention is intended to be present in the form of stereoisomers (including cis and trans isomers, optical isomers (e.g., *R* and *S* enantiomers), diastereomers, geometric isomers, rotamers, conformers, atropisomers, and mixtures thereof). The compound of the present invention may exhibit more than one type of isomerism and consist of mixtures thereof (e.g., racemic mixtures and diastereomer pairs).

The compound of the present disclosure may be present in the form of a solvate (preferably hydrate), and the compound of the present disclosure contains a polar solvent as a structural element of the crystal lattice of the compound, particularly, for example, water, methanol or ethanol. The amount of the polar solvent, particularly water, may be present in a stoichiometric or non-stoichiometric ratio.

The present disclosure further comprises, within its scope, a prodrug of the compound of the present invention. Generally, such a prodrug will be a functional derivative of the compound which is readily converted into a desired therapeutically active compound *in vivo.* Thus, in these cases, the term "administering" or "administration" as used in the treatment method of the present disclosure shall include the treatment of various diseases or conditions with prodrug forms of one or more of the claimed compounds, but the prodrug forms are converted into the compounds described above *in vivo* upon administration to an individual. Conventional methods for selecting and preparing suitable prodrug derivatives are described, for example, in "Design of Prodrug", ed. H. Bundgaard, Elsevier, 1985.

In the present disclosure, the pharmaceutical adjuvants refer to excipients and additives used in the manufacture of a pharmaceutical product and in the formulation of a pharmaceutical formula, and refer to substances, other than the active ingredient, which have been rationally evaluated for safety and are contained in a pharmaceutical formulation. In addition to shaping, serving as carriers, and enhancing stability, pharmaceutical adjuvants also have such important functions as solubilization, aiding dissolution, sustaining or controlling release, etc. They are important ingredients which possibly affect the quality, safety and effectiveness of drugs. According to the origin, pharmaceutical adjuvants may be classified into natural products, semi-synthetic products and total synthetic products. According to the effect and use, pharmaceutical adjuvants may be classified into: solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adhesive agents, antioxidants, chelating agents, permeation promoters, pH regulators, buffers, plasticizers, surfactants, foaming agents, defoaming agents, thickeners, inclusion agents, humectants, absorbents, diluents, flocculants, deflocculants, filter aids, release retardants, etc; according to the administration route, pharmaceutical adjuvants may be classified into pharmaceutical adjuvants for oral administration, injection, mucosal administration, transdermal or local administration, nasal or oral inhalation administration, ocular administration, etc. The same pharmaceutical adjuvant may be used for pharmaceutical formulations with different administration routes, and have different effects and uses.

The pharmaceutical composition may be prepared into various suitable dosage forms according to the administration route, for example, tablets, capsules, granules, oral solutions, oral suspensions, oral emulsions, powders, tinctures, syrups, injections, suppositories, ointments, creams, pastes, ophthalmic formulations, pills, implants, aerosols, powder aerosols, sprays, etc. Among them, the pharmaceutical composition or suitable dosage form may contain 0.01 mg to 1000 mg of the compound of the present disclosure or the pharmaceutically acceptable salt or conjugate thereof, suitably 0.1 mg to 800 mg, preferably 0.5 mg to 500 mg, preferably 0.5 mg to 350 mg, and particularly preferably 1 mg to 250 mg.

The pharmaceutical composition may be administered in the form of an injection, including an injection, a sterile powder for injection and a concentrated solution for injection. The carriers and solvents that may be used include water, Ringer's solution and isotonic sodium chloride solution. In addition, sterilized non-volatile oil may also be used as a solvent or suspending medium, such as monoglyceride or diglyceride. The pharmaceutical composition may be administered in the form of an infusion.

The term "treat", "treating" or "treatment" as used herein usually refers to acquiring needed pharmacological effects and/or physiological effects. In terms of fully or partially preventing a disease or a symptom thereof, the effect may be preventive; and/or in terms of partially or fully stabilizing or curing the disease and/or a side effect of the disease, the effect may be therapeutic. As used herein, "treat", "treating" or "treatment" encompasses any treatment to a disease in a patient, including (a) preventing a disease or a symptom in a patient susceptible to the disease or the symptom that has not yet been diagnosed; (b) inhibiting a symptom of a disease, i.e., arresting its progression; or (c) relieving a symptom of a disease, i.e., causing regression of the disease or the symptom.

In the present disclosure, the term "individual" includes a human or non-human animal. Exemplary human individuals include human individuals with a disease (e.g., a disease described herein), which are referred to as patients, or normal individuals. In the present disclosure, the term "non-human animal" includes all vertebrates, such as non-mammals (e.g., birds, amphibians, reptiles) and mammals, such as non-human primates, livestock and/or domesticated animals (e.g., sheep, dogs, cats, cows, pigs, etc.).

In the present disclosure, the term "effective dose" refers to the amount of an antibody-drug conjugate, a drug-linker conjugate, a compound or a composition that can relieve one or more symptoms of a condition to be treated to some extent after administration.

In the present disclosure, the term "antibody-drug conjugate" or "ADC" refers to a substance obtained by linking a bioactive compound fragment (drug molecule) to a moiety of an antibody or an antigen-binding fragment thereof. In some embodiments of the present disclosure, the bioactive compound fragment is attached to the targeting moiety via a linker. The linker is capable of being cleaved in a particular environment (e.g., an intracellular low-pH environment) or under a particular action (e.g., the action of a lysosomal protease), so that the bioactive compound fragment is separated from the targeting moiety or the antibody or the antigen-binding fragment thereof. In some embodiments of the present disclosure, the linker comprises a cleavable or non-cleavable unit, such as a peptide or a disulfide bond. In some embodiments of the present disclosure, the bioactive compound fragment is directly attached to the targeting moiety or the antibody or the antigen-binding fragment thereof via a covalent bond that is capable of being cleaved in a particular environment or under a particular action, so that the bioactive compound fragment is separated from the antibody or the antigen-binding fragment thereof.

In the present disclosure, the term "drug" refers to a substance that inhibits or prevents cell functions and/or causes cell death or cell destruction.

In the present disclosure, referring to "position 1 of L1 is attached to Tb via an S atom", it will be understood by those skilled in the art that position 1 of L1 is attached to a sulfhydryl group contained in Tb (e.g., an antibody) itself after the disulfide bond is opened (for example, the disulfide bond may be opened by reducing the disulfide bond using a reducing agent TCEP to generate the sulfhydryl group -SH), that is, -S- between L1 and Tb is not an additional extraneous sulfur atom. For example, -S- is not an additional extraneous sulfur atom, but is -S- formed by linking the sulfhydryl group contained in Tb itself after the disulfide bond is opened to position 1 of L1, such as

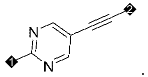

**In** the present disclosure, the term "linker" refers to a fragment that links a bioactive compound fragment (drug molecule) to an antibody moiety.

In the present disclosure, the ".... bioactive compound fragment" refers to a moiety (fragment or group) of an antibody-drug conjugate (or referred to as ADC) well known in the art, which is capable of forming a bioactive drug (e.g., a small-molecule cytotoxic drug, which comprises a group thereof after loss of an atom or an atomic group) or a derivative thereof (e.g., a precursor thereof) after cleavage/degradation/enzyme digestion of a linker among tumor tissues or inside tumor cells. To avoid ambiguity, "drug" does not refer to only a "drug" that has been approved by the pharmaceutical regulatory authority, but also includes any compound with potential therapeutic bioactivity in clinical practice or in research and development as well as in academic research.

In the present disclosure, the term "antibody" is interpreted in its broadest sense and includes intact monoclonal antibodies, polyclonal antibodies, and multispecific antibodies (e.g., bispecific antibodies) formed from at least two intact antibodies, so long as they possess the desired bioactivity. In the present disclosure, "antibody" and "immunoglobulin" may be used interchangeably.

In the present disclosure, the term "monoclonal antibody" refers to an antibody from a population of substantially homogeneous antibodies, i.e., the individual antibodies constituting the population are identical except for a small number of natural mutations that may be present. Monoclonal antibodies have high specificity for one determinant (epitope) of an antigen, while polyclonal antibodies in contrast thereto comprise different antibodies against different determinants (epitopes). In addition to specificity, monoclonal antibodies have the advantage that they can be synthesized without contamination by other antibodies. The modifier "monoclonal" used herein indicates that the antibody is characterized as being from a substantially homogeneous population of antibodies, and should not be construed as requiring a particular preparation method.

In some embodiments of the present disclosure, monoclonal antibodies further particularly include chimeric antibodies, i.e., a portion of the heavy chain and/or light chain is identical or homologous to a type, a class or a subclass of antibodies, and the remainder is identical or homologous to another type, another class or another subclass of antibodies, so long as they possess the desired bioactivity (see, e.g., US 4,816,567; and Morrison et al., 1984, PNAS, 81: 6851-6855). Chimeric antibodies that may be used in the present disclosure include primatized antibodies comprising variable region antigen-binding sequences from non-human primates (e.g., ancient monkeys, chimpanzees, etc.) and human constant region sequences.

The term "antibody fragment" refers to a portion of an antibody, preferably an antigen-binding region or a variable region. Examples of antibody fragments include a Fab, a Fab', a F(ab')₂, an Fd, an Fv, a dAb, a complementarity determining region fragment, a diabody, a linear antibody and a single-chain antibody molecule.

The term "bispecific antibody", also known as "bifunctional antibody conjugate", refers to a conjugate formed from a first antibody (fragment) and a second antibody (fragment) via a coupling arm, and the conjugate retains the activity of the respective antibody and thus has bifunctionality and bispecificity.

The term "multispecific antibody" includes, for example, a trispecific antibody and a tetraspecific antibody, the former being an antibody with three different kinds of antigen-binding specificity, and the latter being an antibody with four different kinds of antigen-binding specificity.

The term "intact antibody" refers to an antibody comprising antigen-binding variable regions, a light chain constant region (CL) and heavy chain constant regions (CH1, CH2 and CH3). The constant region may be a native sequence (e.g., a human native constant region sequence) or a variant of an amino acid sequence thereof. The intact antibody is preferably an intact antibody with one or more effector functions.

The term "probody" is a modified antibody, comprising an antibody or an antibody fragment, capable of specifically binding to its target and capable of being coupled to a masking group, wherein the masking group means that the cleavage constant of the binding capacity of the antibody or the antibody fragment to its target is at least 100 times or 1000 times or 10000 times greater than that of the binding capacity of the antibody or the antibody fragment without the masking group to its target.

In the present disclosure, "humanized" forms of non-human (e.g., murine) antibodies refer to chimeric antibodies that comprise minimal non-human immunoglobulin sequences. Most humanized antibodies are recipient immunoglobulins with hypervariable region residues that are replaced by non-human (e.g., mouse, rat, rabbit or non-human primate) hypervariable region residues having the desired specificity, affinity and function (donor antibody). In some embodiments, framework region (FR) residues of the human immunoglobulin are also replaced by non-human residues. Furthermore, humanized antibodies may further comprise residues that are not present in the recipient antibody or the donor antibody. These modifications are made to further optimize the properties of the antibody. Humanized antibodies generally comprise at least one, usually two, variable regions in which all or nearly all hypervariable loops correspond to those of non-human immunoglobulins, and FRs are completely or nearly completely the sequences of human immunoglobulins. The humanized antibody may further comprise at least one portion of an immunoglobulin constant region (Fc), typically a human immunoglobulin Fc. For details see, e.g., Jones et al., 1986, Nature, 321: 522-525; Riechmann et al., 1988, Nature, 332: 323-329; and Presta, 1992, Curr Op Struct Bwl 2: 593-596.

Intact antibodies may be divided into different "classes" according to the amino acid sequences of the heavy chain constant regions. The main five classes are IgA, IgD, IgE, IgG and IgM, several of which may also be divided into different "subclasses" (isotypes), such as IgG1, IgG2, IgG3, IgG4, IgA1 and IgA2. Different classes of heavy chain constant regions of antibodies are referred to as α, β, ε, γ and µ, respectively. Different classes of subunit structures and three-dimensional configurations of immunoglobulins are well known in the art.

In the present disclosure, although in most cases, amino acid substitutions in antibodies are substitutions with L-amino acids, it is not limited thereto. In some embodiments, one or more D-amino acids may be comprised in an antibody peptide chain. Peptides comprising D-amino acids are more stable and less susceptible to degradation in the oral cavity, intestine or plasma than peptides comprising only L-amino acids.

Monoclonal antibodies used in the present disclosure may be produced by a number of methods. For example, monoclonal antibodies used in the present disclosure may be obtained by the hybridoma method using a number of species including mouse, hamster, rat and human cells (see, e.g., Kohler et al., 1975, Nature, 256: 495), or prepared by recombinant DNA techniques (see, e.g., US 4,816,567), or isolated from phage antibody libraries (see, e.g., Clackson et al., 1991, Nature, 352: 624-628; and Marks et al., 1991, Journal of Molecular Biology, 222: 581-597).

In the present disclosure, unless otherwise expressly indicated, the description "each ... be independently selected from" and "... be each independently selected from" are used interchangeably throughout the present disclosure and should be understood in a broad sense, and it may mean that specific items expressed by the same or different symbols in different groups do not affect each other, or that specific items expressed by the same or different symbols in the same group do not affect each other.

In the structure of the amino acid residue represented by AA¹ 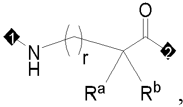 if r is 0, it will be understood by those skilled in the art that the structure of the amino acid residue represented by AA¹ will become

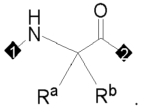

In the structure of the amino acid residue represented by AA¹ 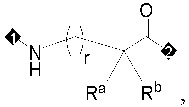 if R^{a} and R^{b}, together with the carbon atom to which they are both linked, form a 4-10 membered heterocycle, the 4-10 membered heterocycle is optionally substituted with one or more R⁰, wherein the term "the 4-10 membered heterocycle is optionally substituted with one or more R⁰" means that the 4-10 membered heterocycle may be unsubstituted or substituted with one or more R⁰, and in the more R⁰, the definition of each R⁰ may be identical or different. Other similar definitions may be understood with reference to the aforementioned content.

In each part of this specification, substituents for the compounds of the present disclosure are disclosed according to group types or ranges. It is specifically noted that each independent sub-combination of the various members of these group types and ranges is included in the present disclosure. For example, the term "C1-6 alkyl" refers specifically to independently disclosed methyl, ethyl, C3 alkyl, C4 alkyl, C5 alkyl and C6 alkyl.

In the present disclosure, the term "direct bond" means that the indicated substituent is absent and the two ends of the substituent are directly attached to form a bond.

In the present disclosure, the terms "include", "comprise", "have", "contain" or "involve", and other variant forms thereof herein, are inclusive or open-ended and do not exclude other unrecited elements or method steps.

In the present disclosure, the term "C1-6 alkyl" refers to linear or branched alkyl containing 1 to 6 carbon atoms, including, for example, "C1-3 alkyl" or "C1-4 alkyl", methyl, ethyl, etc, and specific examples include, but are not limited to: methyl, ethyl, *n*-propyl, isopropyl, *n*-butyl, isobutyl, *sec*-butyl, *tert-*butyl*,* pentyl and hexyl.

In the present disclosure, the term "C2-6 alkenyl" refers to linear, branched or cyclic alkenyl containing at least one double bond and 2 to 6 carbon atoms, including, for example, "C2-4 alkenyl" , etc. Examples of alkenyl include, but are not limited to: ethenyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, 1,4-cyclohexadienyl, etc.

In the present disclosure, the term "C2-6 alkynyl" refers to linear or branched alkynyl containing at least one triple bond and 2 to 6 carbon atoms, including, for example, "C2-4 alkynyl", etc. Examples of alkynyl include, but are not limited to: ethynyl, propynyl, 2-butynyl, 2-pentynyl, 3-pentynyl, 4-methyl-2-pentynyl, 2-hexynyl, 3-hexynyl, 5-methyl-2-hexynyl, etc.

In the present disclosure, the term "halogen" includes fluorine, chlorine, bromine and iodine.

In the present disclosure, the term "C3-6 cycloalkyl" refers to saturated cyclic alkyl containing 3 to 6 carbon atoms. Optionally, the carbon atom in the cyclic structure may be substituted with oxo. Specific examples include, but are not limited to, cyclopropanyl (i.e., cyclopropyl), cyclobutanyl (i.e., cyclobutyl), cyclopentanyl (i.e., cyclopentyl) and cyclohexyl.

In the present disclosure, the term "C1-6 alkoxy" refers to alkyl as defined above which is attached to the parent molecular moiety via an oxygen atom, including, for example, "C1-3 alkoxy" or "C1-4 alkoxy". Specific examples include, but are not limited to, methoxy, ethoxy, propoxy, isopropoxy, *n*-propoxy, isopropoxy, *n*-butoxy, isobutoxy, *tert*-butoxy, pentoxy, hexoxy, etc.

In the present disclosure, the term "C1-6 haloalkyl" refers to alkyl as defined above which is substituted with one or more halogens as defined above, including, for example, "C1-3 haloalkyl" or "C1-4 haloalkyl". Specific examples include, but are not limited to, chloromethyl, fluoroethyl, bromopropyl, etc.

In the present disclosure, the term "4-10 membered heterocycle" refers to a ring containing 4 to 10 ring atoms (at least one of which is a heteroatom, such as nitrogen atom, oxygen atom or sulfur atom). The term "3-6 membered heterocycle" refers to a ring containing 3 to 6 ring atoms (at least one of which is a heteroatom, such as nitrogen atom, oxygen atom or sulfur atom). The term "3-7 membered heterocycle" refers to a ring containing 3 to 7 ring atoms (at least one of which is a heteroatom, such as nitrogen atom, oxygen atom or sulfur atom). The term "5-6 membered heterocycle" refers to a ring containing 5 to 6 ring atoms (at least one of which is a heteroatom, such as nitrogen atom, oxygen atom or sulfur atom). Optionally, the ring atom (e.g., the carbon atom, the nitrogen atom or the sulfur atom) in the cyclic structure may be substituted with oxo, and specific examples include, but are not limited to, pyrrolidine, tetrahydrofuran, piperidine, piperazine, tetrahydropyran, pyrrolidone and other rings.

In the present disclosure, the term "3-10 membered carbocycle" refers to a ring having 3 to 10 ring atoms as carbon atoms. Optionally, the carbon atom in the cyclic structure may be substituted with oxo. Specific examples of, for example, 3-6 membered carbocycle, 3-7 membered carbocycle, 5-8 membered carbocycle, etc. include, but are not limited to, cyclopentane, cyclohexane, etc.

In the present disclosure, the term "4-10 membered heterocyclyl" refers to a cyclic group containing 4 to 10 ring atoms (at least one of which is a heteroatom, such as oxygen atom, nitrogen atom or sulfur atom). The term "4-6 membered heterocyclyl" refers to a cyclic group containing 4 to 6 ring atoms (at least one of which is a heteroatom, such as oxygen atom, nitrogen atom or sulfur atom). The term "3-6 membered heterocyclyl" refers to a cyclic group containing 3 to 6 ring atoms (at least one of which is a heteroatom, such as oxygen atom, nitrogen atom or sulfur atom). Optionally, the ring atom (e.g., the carbon atom, the nitrogen atom or the sulfur atom) in the cyclic structure may be substituted with oxo. "4-8 membered heterocyclyl" includes, for example, "4-8 membered nitrogen-containing heterocyclyl", "4-8 membered oxygen-containing heterocyclyl", "4-7 membered heterocyclyl", "4-7 membered oxygen-containing heterocyclyl", "4-7 membered heterocyclyl", "4-6 membered heterocyclyl", "5-7 membered heterocyclyl", "5-6 membered heterocyclyl", and "5-6 membered nitrogen-containing heterocyclyl", and specific examples include, but are not limited to, oxocyclobutanyl, pyrrolidinyl, tetrahydrofuranyl, piperidinyl, piperazinyl, tetrahydropyranyl, homopiperazinyl, etc.

In the present disclosure, the term "aryl" refers to a monocyclic or polycyclic hydrocarbon group which is aromatic, such as 6-10 membered aryl, 5-8 membered aryl, etc. Specific examples include, but are not limited to, phenyl, naphthyl, anthryl, phenanthryl, etc. The "6-10 membered aryl" refers to aryl containing 6 to 10 ring atoms. The "C6-10 aryl" refers to aryl containing 6 to 10 carbon atoms.

In the present disclosure, the term "heteroaryl" refers to a cyclic group which is aromatic, wherein at least one ring atom is a heteroatom, such as nitrogen atom, oxygen atom or sulfur atom. Optionally, the ring atom (e.g., the carbon atom, the nitrogen atom or the sulfur atom) in the cyclic structure may be substituted with oxo. Specific examples include, but are not limited to, 5-10 membered heteroaryl, 5-6 membered heteroaryl, 5-10 membered nitrogen-containing heteroaryl, 6-10 membered oxygen-containing heteroaryl, 6-8 membered nitrogen-containing heteroaryl, 5-8 membered oxygen-containing heteroaryl, etc, such as furanyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridinyl, 2-pyridonyl, 4-pyridonyl, pyrimidinyl, 1,4-dioxacyclohexadienyl, 2*H*-1,2-oxazinyl, 4*H*-1,2-oxazinyl, 6*H*-1,2-oxazinyl, 4*H*-1,3-oxazinyl, 6*H*-1,3-oxazinyl, 4*H*-1,4-oxazinyl, pyridazinyl, pyrazinyl, 1,2,3-triazinyl, 1,3,5-triazinyl, 1,2,4,5-tetrazinyl, azepinyl, 1,3-diazepinyl, azacyclooctatetraenyl, etc.

The bond in the structural formula represented by a wave line "~~" in the present disclosure is intended to represent that the structure represents a cis or trans isomer, or a mixture of cis and trans isomers in any proportion.

In the present disclosure, the term "linker unit" is a component of an antibody-drug conjugate or a drug-linker conjugate or a linker, which functions to link an antibody or an antigen-binding fragment thereof that binds to a target to the remainder of the antibody-drug conjugate. The linker unit is capable of linking a Tb unit to L₂, and specific examples include, but are not limited to (wherein position 1 is attached to the antibody or the antigen-binding fragment thereof, and position 2 is attached to L₂ or L₃): 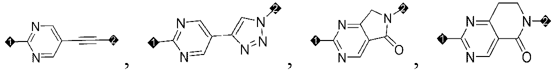 and

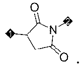

In the present disclosure, the term "connector unit" is a component of an antibody-drug conjugate or a drug-linker conjugate or a linker, which functions to bind the linker unit to an amino acid residue or a short peptide consisting of 2-10 amino acid residues. The connector unit, when present, is capable of linking L₁ to L₃. Specific examples include, but are not limited to (wherein position 1 is attached to the linker unit, and position 2 is attached to L₃):

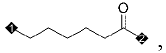

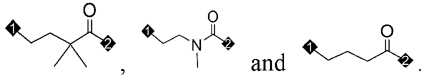

The preferred conditions described above may be combined arbitrarily to obtain preferred embodiments of the present invention without departing from the general knowledge in the art. The reagents and starting materials used in the present disclosure are commercially available.

### Beneficial effects of the invention

Through a large number of studies in the present disclosure, a GSPT1 protein degradation agent (bioactive compound or payload) with a novel structure and an antibody-drug conjugate (ADC) thereof are obtained, both of which have extremely strong bioactivity and can realize the following technical effects:
(1) The GSPT1 protein degradation agent shows extremely strong inhibitory activity on the proliferation of different cells, is significantly superior to a control compound (3-40 times), and has the potential of a strong bystander effect after forming an ADC. The molecule has no basic groups, so that in one aspect, the phenomenon of lysosome capture (i.e., molecules containing basic groups cannot play a role because of being bound in lysosomes with relatively strong acidity) can be reduced or eliminated, and in another aspect, the molecule cannot be rapidly distributed to other non-tumor tissues to cause "off-target" toxicity.
(2) The stability problem of the traditional maleimide coupling linker is overcome, the stability of the ADC in a circulatory system is high, and the shedding of bioactive compounds in non-target tissues can be reduced.
(3) The ADC can simultaneously release payload inside and outside tumor cells, so that the effective release of the bioactive compound at a tumor site is increased, and the bioactive compound has a higher intratumoral and blood concentration ratio, thereby achieving a higher therapeutic index.
(4) The ADC shows extremely strong inhibitory activity on the proliferation of cells, shows an anti-tumor effect that is significantly superior to that of a control compound in the aspect of *in vivo* efficacy, and has good safety because animals in each group have no significant weight loss and significant drug toxicity during the administration.

Therefore, the GSPT1 protein degradation agent and ADC of the present invention have high clinical application values.

### BRIEF DESCRIPTION OF THE DRAWINGS

FIG. 1A shows the results of ELISA binding assay of anti-B7H3 antibody 1D1-01 to human B7H3-4IgG-His protein;
FIG. 1B shows the results of ELISA binding assay of anti-B7H3 antibody 2E3-02 to human B7H3-4IgG-His protein;
FIG. 2A shows the results of ELISA binding assay of anti-B7H3 antibody 1D1-01 to monkey B7H3-4IgG-His protein;
FIG. 2B shows the results of ELISA binding assay of anti-B7H3 antibody 2E3-02 to monkey B7H3-4IgG-His protein;
FIG. 3A shows the results of flow cytometry binding assay of anti-B7H3 antibody 1D1-01 to MCF-7 tumor cells;
FIG. 3B shows the results of flow cytometry binding assay of anti-B7H3 antibody 2E3-02 to MCF-7 tumor cells;
FIG. 4A shows the results of flow cytometry binding assay of anti-B7H3 antibody 1D1-01 to A549 tumor cells;
FIG. 4B shows the results of flow cytometry binding assay of anti-B7H3 antibody 2E3-02 to A549 tumor cells;
FIG. 5 shows the results of flow cytometry binding assay of anti-B7H3 antibody 2E3-02 to PC-3 tumor cells;
FIG. 6A shows the results of endocytosis assay of the candidate antibody 1D1-01 by A549 tumor cells;
FIG. 6B shows the results of endocytosis assay of the candidate antibody 2E3-02 by A549 tumor cells;
FIG. 7A shows one of the assay results of anti-Her2-ADC inhibiting tumor growth in an NCI-N87 xenograft tumor model;
FIG. 7B shows the body weight changes of the test animals in each group at a dose of 1 mg/kg;
FIG. 8A shows the other assay results of anti-Her2-ADC inhibiting tumor growth in the NCI-N87 xenograft tumor model;
FIG. 8B shows the body weight changes of the test animals in each group at doses of 2 mg/kg and 6 mg/kg.

### DETAILED DESCRIPTION

The present disclosure is further illustrated by the following specific embodiments, which, however, are not intended to limit the present disclosure. Various modifications or improvements may be made by those skilled in the art in light of the teachings without departing from the basic spirit and scope of the present disclosure. Reagents or instruments without specified manufacturers used herein are conventional products that are commercially available.

The abbreviations in the present disclosure have the following meanings:

| | | | |
|---|---|---|---|
| OMs | Methanesulfonate group | FA | Formic acid |
| OTs | Trifluoromethanesulfon ate group | ACN | Acetonitrile |
| HCl | Hydrochloric acid | DCM | Dichloromethane |
| Na₂SO ₄ | Sodium sulfate | MeO H | Methanol |
| DMF | N,N-dimethylformamid e | Pd/C | Palladium on carbon |
| THF | Tetrahydrofuran | LCM S | High-performance liquid chromatography-mass spectrometry |
| TLC | Thin-layer chromatography | EA | Ethyl acetate |
| PE | Petroleum ether | DMS O | Dimethyl sulfoxide |
| OTf | *p*-Toluenesulfonate group | CCK8 reagen t | 2-(2-Methoxy-4-nitrophenyl)-3-(4-nitrophenyl)-5-(2,4-disulfophenyl)-2*H*-t etrazole monosodium salt |
| TBS | *tert*-Butyldimethylsilyl | FBS | Fetal bovine serum |
| MMT | *p*-Methoxytrityl | PB/ PBS | Phosphate buffer |

### Sequences and specific information

| **SEQ ID NO** | **Sequence name** | **Amino acid sequence** |
|---|---|---|
| 1 | 1D1-01 VH | EGQLVESGGGLVQPGGSLRLSCAASGFTISSYWMNWVRQAPGKGLEWVANIKQD GSEKYYVDSVQGRFTISRDNAKNSLYLQMNSLRAEDTAVYYCARRMYSSGWYWG AFDMWGQGTMVTVSS |
| 2 | 1D1-01 VL | EIVLTQSPGTLSLSPGERATLSCRASQSVSSIYLAWNQQKPGQAPRLLIYGTFKRATG IPDRFSGSGSGTDFTLTISSLEPEDFAVYYCQQYDTSSITFGQGTRLEIK |
| 3 | 1D1-01 HC, i.e., 1D1-01 heavy chain | EGQLVESGGGLVQPGGSLRLSCAASGFTISSYWMNWVRQAPGKGLEWVANIKQD GSEKYYVDSVQGRFTISRDNAKNSLYLQMNSLRAEDTAVYYCARRMYSSGWYWG AFDMWGQGTMVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNHY TQKSLSLSPGK |
| 4 | 1D1-01 LC, i.e., 1D1-01 light chain | EIVLTQSPGTLSLSPGERATLSCRASQSVSSIYLAWNQQKPGQAPRLLIYGTFKRATG IPDRFSGSGSGTDFTLTISSLEPEDFAVYYCQQYDTSSITFGQGTRLEIKRTVAAPSVFI FPPSDEQLKSGTASVVCLLNNFYPREAKVQWKVDNALQSGNSQESVTEQDSKDST YSLSSTLTLSKADYEKHKVYACEVTHQGLSSPVTKSFNRGEC |
| 5 | 2E3-02 VH | EVQLLESGGGLVQPGGSLRLSCAASGFTFSSYAMNWVRQAPGKGLEWVSGISGSG GSTYYADSVQGRFTISRDNSKNTLYLQMNSLRAEDTAVYYCAKARSGYDYFFDYW GQGTLVTVSS |
| 6 | 2E3-02 VL | EIVLTQSPGTLSLSPGERATLSCRASQRISSSFLAWYQQKPGQAPSLLIYGASSRATGI PDRFSGSGSGTDFTLTISSLEPEDFAVFYCQQYSNSPLTFGGGTKVEIK |
| 7 | 2E3-02 HC, i.e., 2E3-02 heavy chain | EVQLLESGGGLVQPGGSLRLSCAASGFTFSSYAMNWVRQAPGKGLEWVSGISGSG GSTYYADSVQGRFTISRDNSKNTLYLQMNSLRAEDTAVYYCAKARSGYDYFFDYW GQGTLVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSCD KTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPI EKTISKAKGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYTQKSLSLSP GK |
| 8 | 2E3-02 LC, i.e., 2E3-02 light chain | EIVLTQSPGTLSLSPGERATLSCRASQRISSSFLAWYQQKPGQAPSLLIYGASSRATGI PDRFSGSGSGTDFTLTISSLEPEDFAVFYCQQYSNSPLTFGGGTKVEIKRTVAAPSVFI FPPSDEQLKSGTASVVCLLNNFYPREAKVQWKVDNALQSGNSQESVTEQDSKDST YSLSSTLTLSKADYEKHKVYACEVTHQGLSSPVTKSFNRGEC |
| 9 | IgG1 CH, i.e., IgG1 constant region | |
| 10 | Kappa CL, i.e., Kappa constant region | |

### Preparation schemes

The structures of the compounds described in the following examples were determined by nuclear magnetic resonance (¹H NMR) and/or mass spectrometry (MS).

Nuclear magnetic resonance (¹H NMR) was determined using a Bruker 400 MHz nuclear magnetic resonance spectrometer, the solvents for the determination were deuterated methanol (CD₃OD), deuterated chloroform (CDCl₃) or hexadeuterodimethyl sulfoxide (DMSO-d₆), and the internal standard substance was tetramethylsilane (TMS).

The abbreviations in the nuclear magnetic resonance (NMR) spectra used in the examples are shown below:
s: singlet; d: doublet; t: triplet; q: quartet; dd: double doublet; qd: quartet doublet; ddd: double double doublet; ddt: double double triplet; dddd: double double double doublet; m: multiplet; br: broad; J: coupling constant; Hz: Hertz; DMSO-d₆: hexadeuterodimethyl sulfoxide. δ values were expressed in ppm.

Mass spectrometry (MS) was determined using an Agilent (ESI) mass spectrometer (model: Agilent 6120B).

Ultra-performance liquid chromatography (UPLC) was determined using an instrument from AB SCIEX with a model of ExionLC.

High-resolution mass spectrometry was determined using an instrument from AB SCIEX with a model of X500B.

### Example 1. Synthesis of Intermediates

### Example 1.1: Synthesis of (10S,13S)-10-(4-(dipropylamino)butyl)-13-isopropyl-20-(2-(methylsulfonyl)pyrimidin-5-yl)-6,9, 12,15-tetraoxo-3-oxa-5,8,11,14-tetraazaeicosan-19-ynoic acid (INT1)

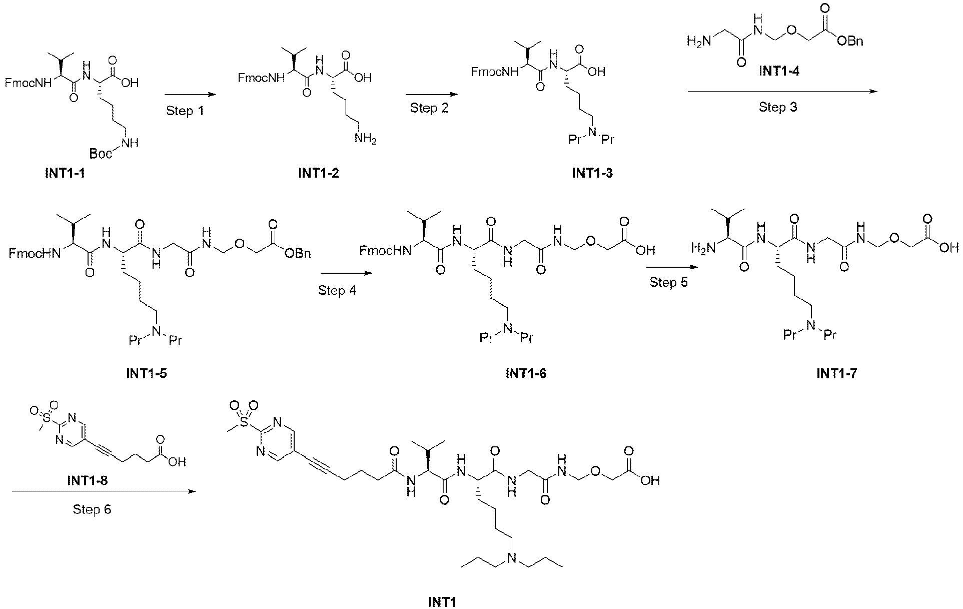

### Step 1:

Compound **INT1-1** (117 g, 206.2 mmol) was added to dioxane (1.5 L) and stirred for 10 min, and then HCl (0.75 L, 4 N in dioxane) was added. The mixture was reacted at room temperature overnight. To the reaction solution was added methyl *tert*-butyl ether (2 L). After being stirred for 30 min, the mixture was filtered, and the solid was transferred to an air blast dryer and dried to give the hydrochloride of the target compound **INT1-2** (94 g, yield: 91%, purity: 94%).

LCMS (ESI) [M+H]⁺ = 468.3.

¹H NMR (400 MHz, DMSO-*d₆*) δ 8.24 (d, *J* = 7.3 Hz, 1H), 8.07 (brs, 3H), 7.90 (d, *J* = 7.5 Hz, 2H), 7.77 (dd, *J* = 7.1, 3.4 Hz, 2H), 7.47 - 7.37 (m, 3H), 7.34 (td, *J* = 7.4, 1.7 Hz, 2H), 4.34 - 4.17 (m, 4H), 3.96 (dd, *J =* 8.6, 7.4 Hz, 1H), 2.83 - 2.65 (m, 2H), 2.04 (dd, *J =* 13.5, 6.7 Hz, 1H), 1.82- 1.52 (m, 4H), 1.51- 1.30 (m, 2H), 0.96- 0.84 (m, 6H).

### Step 2:

The hydrochloride of compound **INT1-2** (55.0 g, 109.1 mmol) and acetic acid (10 mL) were dissolved in methanol (500 mL), and *n*-propionaldehyde (25.35 g, 436.4 mmol) was added to the reaction solution in an ice-water bath. The mixture was stirred at room temperature for 30 min, and then sodium cyanoborohydride (27.43 g, 436.4 mmol) was added to the reaction solution under an ice-water bath. The mixture was stirred at room temperature for 1 h. Then, n-propionaldehyde (12.67 g, 218.2 mmol) and sodium cyanoborohydride (13.72 g, 218.2 mmol) were added to the reaction solution. The mixture was reacted for another 0.5 h. The reaction solution was filtered, and the filtrate was concentrated. Purified water (300 mL) was added to the concentrated solution, and the resulting mixture was extracted with DCM (300 mL × 2). The organic phase was washed with saturated brine, dried over anhydrous Na₂SO₄ and concentrated to give a crude product. The crude product was purified by column chromatography (eluent: MeOH and DCM, wherein MeOH:DCM (V/V) = 1:4 to 2:3) to give compound **INT1-3** (39 g, yield: 65%, purity: 98%).

LCMS (ESI) [M+H]⁺ = 552.3.

¹H NMR (400 MHz, DMSO-*d₆*) δ 7.95 - 7.85 (m, 3H), 7.76 (t, *J =* 7.3 Hz, 2H), 7.51 - 7.40 (m, 3H), 7.35 (t, *J =* 7.4 Hz, 2H), 4.34 - 4.22 (m, 3H), 4.13 (dd, *J =* 12.6, 7.2 Hz, 1H), 3.91 (dd, *J =* 8.8, 7.1 Hz, 1H), 2.52 - 2.41 (m, 6H), 2.03 (dt, *J =* 13.5, 6.7 Hz, 1H), 1.79 - 1.67 (m, 1H), 1.66 - 1.56 (m, 1H), 1.42-1.36 (m, 6H), 1.36 - 1.21 (m, 2H), 0.90 (t, *J =* 6.9 Hz, 6H), 0.82 (t, *J =* 6.9 Hz, 6H).

### Step 3:

Compound **INT1-3** (6.6 g, 12 mmol) was dissolved in DMF (50 mL), and compound **INT1-4** (3 g, 12 mmol), HATU (4 g, 11 mmol) and N,N-diisopropylethylamine (3.5 g, 30 mmol) were sequentially added. The mixture was stirred at room temperature for 1 h, and then water (100 mL) was added to the reaction solution. The resulting mixture was extracted with ethyl acetate (200 mL × 3). The organic phases were combined, washed with saturated brine (200 mL × 2), dried over anhydrous sodium sulfate and filtered. The filtrate was concentrated under reduced pressure to give a crude product. The crude product was purified by column chromatography (methanol:dichloromethane = 5%-10%) to give the target compound **INT1-5** (9 g).

LCMS (ESI) [M+H]⁺ = 787.0;

¹H NMR (400 MHz, DMSO-*d₆*) δ 8.87 (s, 1H), 8.70 (t, *J =* 6.5 Hz, 1H), 8.27 - 8.18 (m, 1H), 8.06 (d, *J =* 7.4 Hz, 1H), 7.90 (d, *J =* 7.5 Hz, 2H), 7.73 (d, *J =* 7.2 Hz, 2H), 7.47 - 7.40 (m, 3H), 7.38 - 7.30 (m, 6H), 5.15 (s, 2H), 4.71 - 4.55 (m, 2H), 4.37 - 4.27 (m, 2H), 4.26 - 4.19 (m, 2H), 4.15 (s, 2H), 3.92 - 3.83 (m, 1H), 3.73 (d, *J=* 5.5 Hz, 2H), 3.04 - 2.89 (m, 6H), 2.01 - 1.94 (m, 1H), 1.81 - 1.63 (m, 2H), 1.61 - 1.52 (m, 6H), 1.36 - 1.27 (m, 2H), 0.91 - 0.82 (m, 12H).

### Step 4:

Compound **INT1-5** (3 g, 3.8 mmol) was dissolved in a mixed solvent of ethanol (20 mL) and ethyl acetate (10 mL), and Pd/C (1.5 g) was added under nitrogen atmosphere. The reaction solution was purged three times with hydrogen, stirred at room temperature overnight under hydrogen atmosphere and filtered. The filtrate was concentrated under reduced pressure to give the target compound **INT1-6** (2.8 g), which was directly used in the next step.

LCMS (ESI) [M+H]⁺ = 696.4.

¹H NMR (400 MHz, DMSO-*d₆*) δ 8.67 (t, *J =* 6.5 Hz, 1H), 8.25 (t, *J =* 5.7 Hz, 1H), 8.06 (d, *J* = 7.5 Hz, 1H), 7.90 (d, *J =* 7.5 Hz, 2H), 7.73 (d, *J =* 7.3 Hz, 2H), 7.49 - 7.39 (m, 3H), 7.33 (t, *J =* 7.4 Hz, 2H), 4.65 - 4.55 (m, 2H), 4.35 - 4.26 (m, 2H), 4.24 - 4.14 (m, 2H), 3.97 (s, 2H), 3.92 - 3.83 (m, 1H), 3.73 (d, *J =* 5.6 Hz, 2H), 2.94 - 2.84 (m, 6H), 1.73 - 1.64 (m, 1H), 1.63 - 1.47 (m, 8H), 1.34 - 1.26 (m, 3H), 0.89 - 0.82 (m, 12H).

### Step 5:

Compound **INT1-6** (1 g, 1.43 mmol) was dissolved in THF (30 mL), and diethylamine (1.0 g, 14.3 mmol) was added. The reaction solution was stirred at room temperature for 1 h and concentrated under reduced pressure to give the target compound **INT1-7** (680 mg), which was directly used in the next step.

LCMS (ESI) [M+H]⁺ = 474.1.

### Step 6:

Compound **INT1-8** (340 mg, 1.27 mmol) was dissolved in DMF (50 mL), and HATU (4 g, 11 mmol) and N,N-diisopropylethylamine (3.5 g, 30 mmol) were sequentially added. The mixture was stirred at room temperature for 30 min, and compound **INT1-7** (0.6 g, 1.27 mmol) was added. After the addition, the reaction solution was stirred at room temperature for 1 h and purified by preparative high-performance liquid chromatography (acetonitrile:water = 5%-50%) to give the target compound INT1 (200 mg).

LCMS (ESI) [M+H]⁺ = 724.8;

¹H NMR (400 MHz, DMSO-*d₆*) δ 9.13 (s, 2H), 8.72 - 8.67 (m, 1H), 8.20 (t, *J =* 5.1 Hz, 1H), 8.11 (d, *J =* 7.3 Hz, 1H), 8.01 (d, *J =* 8.7 Hz, 1H), 4.62 (d, *J =* 6.2 Hz, 2H), 4.26 - 4.21 (m, 1H), 4.18 - 4.14 (m, 1H), 3.90 (s, 2H), 3.70 (d, *J =* 5.6 Hz, 2H), 3.42 (s, 3H), 3.17 (s, 2H), 2.42 - 2.32 (m, 4H), 2.05 - 1.94 (m, 3H), 1.87 - 1.78 (m, 2H), 1.75 - 1.67 (m, 2H), 1.63 - 1.53 (m, 2H), 1.48 - 1.40 (m, 9H), 0.87 - 0.84 (m, 12H).

### Example 1.2: Synthesis of 1-(3-chloro-4-(3-(methylamino)propyl)phenyl)-3-((2-(2,6-dioxopiperidin-3-yl)-1-oxoisoindolin-5 -yl)methyl)urea (INT2)

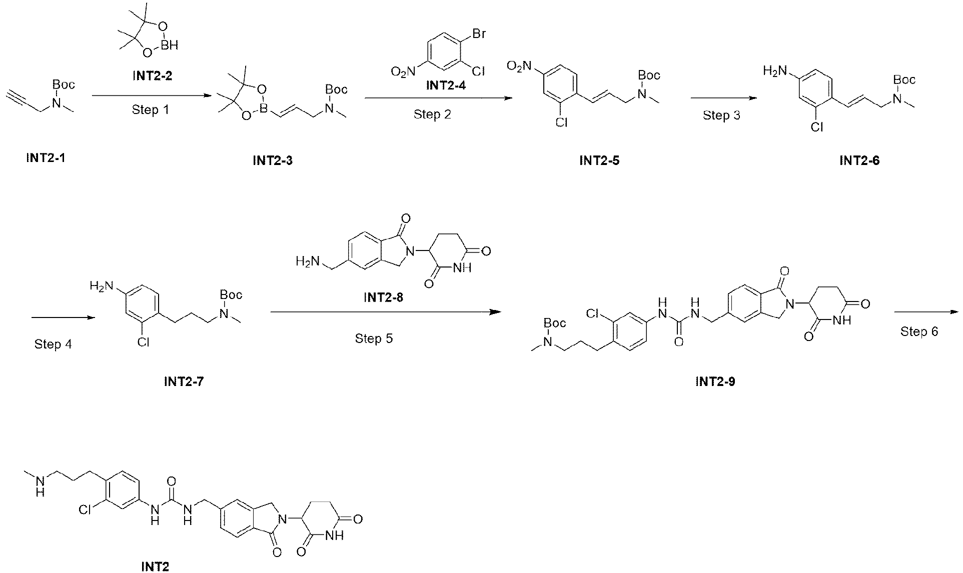

### Step 1:

Compound **INT2-1** (4.0 g, 23.65 mmol) and triethylamine (0.33 mL, 2.36 mmol) were dissolved in compound **INT2-2** (3.18 g, 24.84 mmol), and bis(3-cyclopentadienyl)chlorohydrurozirconium (691 mg, 2.36 mmol) was added. The reaction solution was stirred at 60 °C for 8 h under nitrogen atmosphere. The reaction was monitored by LCMS. The reaction solution was directly concentrated under reduced pressure to give a crude product. The crude product was purified through a chromatographic column (petroleum ether:ethyl acetate = 40:1) to give the target compound **INT2-3** (2.92 g).

¹H NMR (400 MHz, DMSO-*d₆*) δ 6.38 (dt, *J =* 18.0, 4.3 Hz, 1H), 5.32 (d, *J =* 18.1 Hz, 1H), 3.83 (d, *J* = 3.7 Hz, 2H), 2.74 (s, 3H), 1.39 (s, 9H), 1.20 (s, 12H).

### Step 2:

Compound **INT2-4** (1.86 g, 7.88 mmol), compound **INT2-3** (2.34 g, 7.88 mmol), potassium phosphate (5.02 g, 23.64 mmol) and 2-dicyclohexylphosphonium-2',6'-diisopropoxy-1,1'-biphenyl (368 mg, 0.788 mmol) were dissolved in a mixed solution of 1,4-dioxane and water (V:V = 5:1, 50 mL), and palladium acetate (88 mg, 0.394 mmol) was added. The reaction solution was stirred at 80 °C for 5 h under nitrogen atmosphere. The reaction was monitored by LCMS. The reaction solution was directly concentrated under reduced pressure to give a crude product. The crude product was purified through a chromatographic column (petroleum ether:ethyl acetate = 40:1) to give the target compound **INT2-5** (1.0 g).

¹H NMR (400 MHz, DMSO-*d₆*) δ 8.29 (d, *J =* 2.1 Hz, 1H), 8.16- 8.14 (m, 1H), 8.02 (d, *J =* 8.7 Hz, 1H), 6.79 (d, *J =* 16.0 Hz, 1H), 6.19 - 6.05 (m, 1H), 3.78 (d, *J =* 5.7 Hz, 2H), 2.84 (s, 3H), 1.39 (s, 9H).

### Step 3:

Compound **INT2-5** (500 mg, 1.53 mmol) was dissolved in a mixed solution of tetrahydrofuran, anhydrous methanol and water (V:V:V = 2:2:1, 6 mL), and ammonium chloride (821 mg, 15.30 mmol) and zinc powder (997 mg, 15.30 mmol) were sequentially added. The mixture was stirred at 50 °C for 2 h, and the reaction was monitored by LCMS. The reaction solution was filtered, and the filtrate was concentrated under reduced pressure to give a crude product. The crude product was purified through a chromatographic column (petroleum ether:ethyl acetate = 10:1) to give the target compound **INT2-6** (320 mg).

### LCMS (ESI) [M+H -56]⁺ = 241.0.

¹H NMR (400 MHz, DMSO-*d₆*) δ 7.36 (d, *J =* 8.5 Hz, 1H), 6.62-6.58 (m, 2H), 6.51 (d, *J =* 8.5 Hz, 1H), 5.95-5.93 (m, 1H), 5.50 (s, 2H), 3.89 (d, *J =* 5.4 Hz, 2H), 2.77 (s, 3H), 1.40 (s, 9H). Step 4:
Compound **INT2-6** (300 mg, 1.01 mmol) was dissolved in THF (10 mL), and platinum dioxide (50 mg) was added to the reaction solution. The reaction solution was stirred for 1 h under hydrogen atmosphere. The reaction was monitored by LCMS. The reaction solution was filtered, and the filtrate was concentrated under reduced pressure to give the target compound **INT2-7** (200 mg), which was directly used in the next step.

LCMS (ESI) [M+H-100]⁺= 199.0.

¹H NMR (400 MHz, DMSO-*d₆*) δ 6.95 (d, *J =* 8.2 Hz, 1H), 6.59 (d, *J =* 2.2 Hz, 1H), 6.45 (dd, *J =* 8.2, 2.2 Hz, 1H), 5.16 (s, 2H), 3.17 (t, *J =* 7.1 Hz, 2H), 2.77 (s, 3H), 2.48 - 2.43 (m, 2H), 1.66 (s, 2H), 1.37 (s, 9H).

### Step 5:

Compound **INT2-7** (200 mg, 0.67 mmol) was dissolved in tetrahydrofuran (5 mL), and trichloromethyl chloroformate (265 mg, 0.14 mmol) was added. The reaction solution was stirred at room temperature for 1.5 h. The reaction solution was concentrated under reduced pressure to give a crude product. The crude product was dissolved in DMF (5 mL), and a solution of the hydrochloride of compound **INT2-8** (221 mg, 0.88 mmol) and triethylamine (670 mg, 6.70 mmol) in DMF was added. The reaction was monitored by LCMS. Water (50 mL) was added to the reaction solution, and the resulting mixture was extracted with ethyl acetate (25 mL × 3). The organic phases were combined, washed with saturated brine (30 mL × 3), dried over anhydrous Na₂SO₄ and filtered. The filtrate was concentrated under reduced pressure to give a crude product. The crude product was purified by column chromatography (DCM:MeOH = 10:1) to give the target compound **INT2-9** (150 mg).

LCMS (ESI) [M+H-100]⁺= 498.2.

¹H NMR (400 MHz, DMSO-*d₆*) δ 10.98 (s, 1H), 8.81 (s, 1H), 7.69 (d, *J =* 7.9 Hz, 1H), 7.66 (s, 1H), 7.51 (s, 1H), 7.44 (d, *J =* 8.2 Hz, 1H), 7.20 - 7.14 (m, 2H), 6.84 (t, *J =* 5.7 Hz, 1H), 5.17 - 5.03 (m, 1H), 4.43 - 4.39 (m, 2H), 4.34 - 4.27 (m, 1H), 3.21 - 3.17 (m, 2H), 2.96 - 2.86 (m, 1H), 2.77 (s, 3H), 2.64 - 2.52 (m, 4H), 2.43 - 2.33 (m, 1H), 2.03 - 1.98 (m, 1H), 1.76 - 1.67 (m, 2H), 1.37 (s, 9H).

### Step 6:

Compound **INT2-9** (120 mg, 0.20 mmol) was dissolved in DCM (6 mL), and trifluoroacetic acid (2 mL) was added. The reaction solution was stirred at room temperature for 1 h. The reaction was monitored by LCMS. The reaction solution was concentrated under reduced pressure to give the trifluoroacetate of the target compound **INT2** (100 mg).

LCMS (ESI) [M+H]⁺=498.1.

### Example 1.3: Synthesis of 2-(3-chloro-4-(3-(methylamino)propyl)phenyl)-N-((2-(2,6-dioxopiperidin-3-yl)-1-oxoisoindolin-5-yl)methyl)-2,2-difluoroacetamide (INT3)

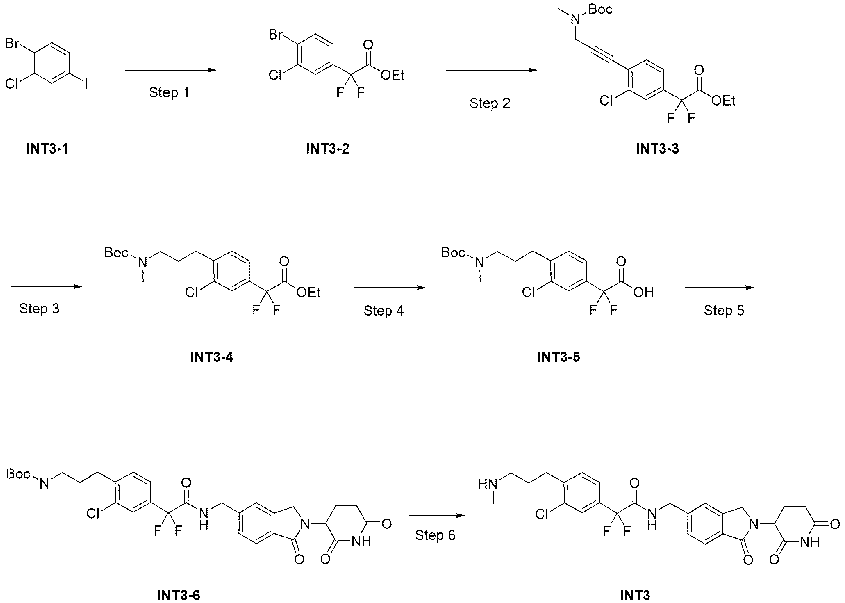

### Step 1:

Under nitrogen atmosphere, compound **INT3-1** (10.0 g, 31.50 mmol) was dissolved in dimethyl sulfoxide (70 mL), and ethyl difluorobromoacetate (12.79 g, 63.00 mmol) and copper powder (4.0 g, 63.00 mmol) were sequentially added to the reaction solution. The reaction solution was stirred at room temperature for 48 h, and the reaction was monitored by TLC. The reaction solution was slowly poured into saturated aqueous ammonium chloride solution (200 mL), and the resulting mixture was extracted with petroleum ether (200 mL × 2). The organic phases were combined, washed with saturated brine (200 mL × 2), dried over anhydrous Na₂SO₄ and filtered under vacuum. The filtrate was concentrated under reduced pressure to give a crude product. The crude product was purified through a chromatographic column (petroleum ether:ethyl acetate = 10:1) to give the target compound **INT3-2** (7.4 g).

¹H NMR (400 MHz, DMSO-*d₆*) δ 7.98 (d, *J =* 8.3 Hz, 1H), 7.83 (s, 1H), 7.51 (d, *J =* 8.3 Hz, 1H), 4.35 - 4.30 (m, 2H), 1.24 (t, *J =* 7.1 Hz, 3H).

### Step 2:

Under nitrogen atmosphere, compound **INT3-2** (3.0 g, 9.57 mmol) was dissolved in DMF (50 mL), and tert-butyl methyl(propyl-2-yn-1-yl)carbamate (3.2 g, 19.14 mmol), triethylamine (4.8 g, 47.84 mmol), cuprous iodide (182 mg, 0.96 mmol) and bis(triphenylphosphine)palladium dichloride (672 mg, 0.96 mmol) were sequentially added. The reaction solution was stirred at 60 °C for 4 h. The reaction was monitored by LCMS. The reaction solution was cooled to room temperature, water (150 mL) was added to the reaction solution, and the resulting mixture was extracted with ethyl acetate (150 mL × 3). The organic phases were combined, washed with saturated brine (200 mL × 3), dried over anhydrous Na₂SO₄ and filtered under vacuum. The filtrate was concentrated under reduced pressure to give a crude product. The crude product was purified through a chromatographic column (petroleum ether:ethyl acetate = 10:1) to give the target compound **INT3-3** (2.0 g).

LCMS (ESI) [M+H-56]⁺= 346.0.

¹H NMR (400 MHz, DMSO-*d₆*) δ 7.75 (d, *J =* 7.4 Hz, 2H), 7.58 (d, *J =* 8.2 Hz, 1H), 4.38 - 4.29 (m, 4H), 2.91 (s, 3H), 1.43 (s, 9H), 1.24 (t, *J=* 7.1 Hz, 3H).

### Step 3:

Compound **INT3-3** (1.2 g, 2.99 mmol) was dissolved in methanol (10 mL), and Raney nickel (300 mg) was added under nitrogen atmosphere. The reaction solution was purged three times with hydrogen and stirred at room temperature for 6 h under hydrogen atmosphere. The reaction was monitored by LCMS. The reaction solution was filtered through celite, and the filtrate was concentrated under reduced pressure to give the target compound **INT3-4** (930 mg), which was directly used in the next step.

LCMS (ESI) [M+H-56]⁺ = 350.0.

¹H NMR (400 MHz, DMSO-*d₆*) δ 7.64 - 7.54 (m, 2H), 7.52 (s, 1H), 4.32 - 4.30 (m, 2H), 3.29 - 3.16 (m, 2H), 2.78 (s, 3H), 2.74 - 2.67 (m, 2H), 1.87 - 1.70 (m, 2H), 1.41 (s, 9H), 1.27 - 1.22 (m, 3H).

### Step 4:

Compound **INT3-4** (900 mg, 2.22 mmol) was dissolved in a mixed solution of methanol (15 mL) and water (3 mL), and lithium hydroxide monohydrate (212 mg, 8.87 mmol) was added. The reaction solution was stirred at room temperature for 2 h. The reaction was monitored by LCMS. Water (20 mL) was added to the reaction solution. The reaction solution was concentrated under reduced pressure to remove methanol, the pH was adjusted to 4 with 1 N aqueous hydrochloric acid solution, and the resulting mixture was extracted with ethyl acetate (20 mL × 2). The organic phases were combined, washed with saturated brine (20 mL × 3), dried over anhydrous Na₂SO₄ and filtered under vacuum. The filtrate was concentrated under reduced pressure to give the target compound **INT3-5** (700 mg), which was directly used in the next step.

LCMS (ESI) [M+H-56]⁺ = 322.2.

¹H NMR (400 MHz, DMSO-*d₆*) δ 7.49 (s, 1H), 7.40 (s, 2H), 3.23-3.20 (m, 2H), 2.78 (s, 3H), 2.68-2.64 (m, 2H), 1.77-1.73 (m, 2H), 1.36 (s, 9H).

### Step 5:

Compound **INT3-5** (670 mg, 1.77 mmol) and 3-(6-aminomethylene-3-oxo-1H-isoindol-2-yl)piperidine-2,6-dione hydrochloride (500 mg, 1.61 mmol) were dissolved in dichloromethane (20 mL), and triethylamine (765 mg, 7.57 mmol) and a solution of propylphosphonic anhydride in ethyl acetate (2.87 g, 4.51 mmol, mass fraction 50%) were added. The reaction solution was stirred at room temperature for 2 h. The reaction was monitored by LCMS. The reaction solution was diluted with water (20 mL) and extracted with ethyl acetate (30 mL × 3). The organic phases were combined, washed with saturated brine (20 mL × 2), dried over anhydrous Na₂SO₄ and filtered under vacuum. The filtrate was concentrated under reduced pressure to give a crude product. The crude product was purified through a chromatographic column (petroleum ether:ethyl acetate = 1:1) to give the target compound **INT3-6** (270 mg).

LCMS (ESI) [M+Na]⁺ = 655.6.

¹H NMR (400 MHz, DMSO-*d₆*) δ 10.99 (s, 1H), 9.68 (t, *J* = 5.9 Hz, 1H), 7.68 (d, *J* = 7.8 Hz, 1H), 7.60 (s, 1H), 7.52 (d, *J =* 11.0 Hz, 2H), 7.43 (s, 1H), 7.37 (d, *J =* 7.8 Hz, 1H), 5.13 - 5.09 (m, 1H), 4.45 - 4.27 (m, 4H), 3.22 - 3.20 (m, 3H), 2.94 - 2.91 (m, 1H), 2.78 (s, 3H), 2.69 (d, *J* = 7.8 Hz, 2H), 2.67 - 2.62 (m, 1H), 2.42 - 2.35 (m, 1H), 1.77 (s, 2H), 1.33 (s, 9H).

### Step 6:

Compound **INT3-6** (205 mg, 0.32 mmol) was dissolved in HCl/1,4-dioxane solution (5 mL). The reaction solution was stirred at room temperature for 2 h. The reaction was monitored by LCMS. The reaction solution was concentrated under reduced pressure to give the hydrochloride of the target compound **INT3** (165 mg).

LCMS (ESI) [M+H]⁺ = 532.9.

### Example 1.4: Synthesis of 1-(3-chloro-4-(3-(methylamino)propyl)phenyl)-3-((2-(2,6-dioxopiperidin-3-yl)-1,3-dioxoisoindol in-5-yl)methyl)urea (INT4)

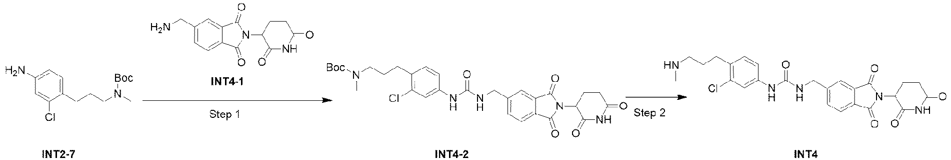

### Step 1:

Compound **INT2-7** (200 mg, 0.67 mmol) was dissolved in THF (5 mL), and trichloromethyl chloroformate (265 mg, 1.34 mmol) was added. The reaction solution was stirred at room temperature for 1 h. The reaction solution was concentrated under reduced pressure to give a crude intermediate. The crude intermediate was dissolved in DMF (5 mL), and a solution of compound **INT4-1** (193 mg, 0.67 mmol) and triethylamine (670 mg, 6.70 mmol) in DMF (5 mL) was added. The reaction was monitored by LCMS. Water (25 mL) was added to the reaction solution, and the resulting mixture was extracted with EA (30 mL × 3). The organic phases were combined, washed with saturated brine (30 mL × 2), dried over anhydrous Na₂SO₄ and filtered. The filtrate was concentrated under reduced pressure, and the crude product was purified by column chromatography (dichloromethane:methanol = 10:1) to give the target compound **INT4-2** (200 mg).

LCMS (ESI) [M+H-100]⁺ =512.3.

¹H NMR (400 MHz, DMSO-*d₆*) δ 11.12 (s, 1H), 8.86 (s, 1H), 7.89 (d, *J =* 7.7 Hz, 1H), 7.85-7.75 (m, 2H), 7.64 (s, 1H), 7.18 (s, 2H), 6.91 (t, *J =* 6.1 Hz, 1H), 5.14 (dd, *J =* 12.9, 5.3 Hz, 1H), 4.46 (d, *J* = 6.1 Hz, 2H), 3.18 (t, *J =* 7.1 Hz, 2H), 2.94-2.85 (m, 1H), 2.77 (s, 3H), 2.64-2.52 (m, 4H), 2.09-2.03 (m, 1H), 1.77-1.64 (m, 2H), 1.36 (s, 9H).

### Step 2:

Compound **INT4-2** (120 mg, 0.20 mmol) was dissolved in DCM (6 mL), and trifluoroacetic acid (2 mL) was added. The reaction solution was stirred at room temperature for 1 h. The reaction was monitored by LCMS. The reaction solution was concentrated under reduced pressure to give the trifluoroacetate of the target compound **INT4** (100 mg).

LCMS (ESI) [M+H]⁺=512.3.

¹H NMR (400 MHz, DMSO-*d₆*) δ 11.12 (s, 1H), 8.98 (s, 1H), 8.34 (s, 2H), 7.90 (d, *J =* 7.7 Hz, 1H), 7.85-7.78 (m, 2H), 7.67 (d, *J =* 1.7 Hz, 1H), 7.23-7.17 (m, 2H), 7.04 (t, *J =* 6.0 Hz, 1H), 5.15 (dd, *J* = 12.8, 5.3 Hz, 1H), 4.47 (d, *J* = 5.8 Hz, 2H), 2.95-2.85 (m, 3H), 2.70-2.57 (m, 4H), 2.57-2.54 (m, 3H), 2.10-2.01 (m, 1H), 1.87-1.79 (m, 2H).

### Example 1.5: Synthesis of (5S,8S)-8-(4-(dimethylamino)butyl)-1-(9H-fluoren-9-yl)-5-isopropyl-3,6,9,12-tetraoxo-2,15-diox a-4,7,10,13-tetraazahetadecan-17-oic acid (INT5)

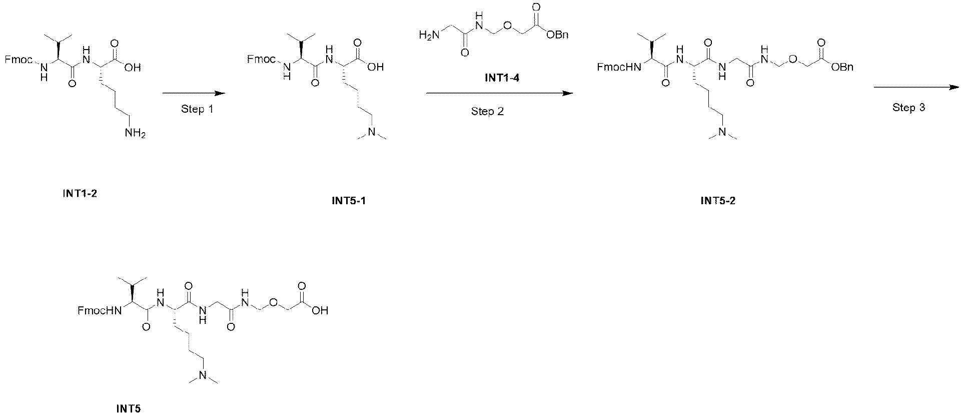

### Step 1:

Compound **INT1-2** (50.0 g, 107.07 mmol) was dissolved in MeOH (1000 mL), sodium acetate (17.6 g, 214.1 mmol) was added, and paraformaldehyde (57.8 g, 642.4 mmol) was added in an ice bath. The mixture was stirred for 30 min, and sodium cyanoborohydride (13.5 g, 214.1 mmol) was added to the reaction system. After the addition, the reaction system was returned to room temperature, and the reaction solution was stirred at room temperature overnight. The reaction was monitored by LCMS. The reaction solution was concentrated under reduced pressure, and the crude product was directly purified by preparative high-performance liquid chromatography (acetonitrile:H₂O containing 0.05% FA = 5%-70%) to give the target compound **INTS-1** (25 g).

LCMS (ESI) [M+H]⁺ = 496.6.

¹H NMR (400 MHz, DMSO-*d₆*) δ 9.96 (s, 1H), 8.19 (d, *J =* 7.5 Hz, 1H), 7.89 (d, *J =* 7.5 Hz, 2H), 7.75 (d, *J =* 7.4 Hz, 2H), 7.42-7.40 (m, 3H), 7.37-7.27 (m, 2H), 4.33-4.25 (m, 1H), 4.24-4.18 (m, 2H), 3.94-3.85 (m, 1H), 3.01-2.89 (m, 2H), 2.68 (d, *J =* 4.7 Hz, 6H), 2.06-1.91 (m, 2H), 1.78-1.68 (m, 1H), 1.65-1.58 (m, 2H), 1.38-1.28 (m, 2H), 1.27-1.22 (m, 1H), 0.93-0.83 (m, 6H).

### Step 2:

Compound **INT5-1** (4.7 g, 9.5 mmol) and compound **INT1-4** (2.4 g, 9.5 mmol) were dissolved in DMF (50 mL), and 2-(7-azabenzotriazol-1-yl)-N,N,N',N'-tetramethyluronium hexafluorophosphate (3.2 g, 8.6 mmol) and N,N-diisopropylethylamine (3.7 g, 28.5 mmol) were added. The reaction solution was stirred at room temperature for 1 h. The reaction was monitored by LCMS. Water (200 mL) was added to the reaction solution, and the resulting mixture was extracted with EA (200 mL × 3). The organic phases were combined, washed with saturated brine (150 mL × 3), dried over anhydrous Na₂SO₄ and filtered. The filtrate was concentrated under reduced pressure, and the crude product was purified by column chromatography (DCM:MeOH = 10:1) to give the target compound **INT5-2** (1.5 g).

LCMS (ESI) [M+H]⁺ = 730.4.

¹H NMR (400 MHz, DMSO-*d₆*) δ 8.70 (t, *J =* 6.6 Hz, 1H), 8.25 (t, *J =* 6.2 Hz, 1H), 8.08 (d, *J =* 7.3 Hz, 1H), 7.90 (d, *J =* 7.5 Hz, 2H), 7.74 (d, *J =* 7.3 Hz, 2H), 7.43-7.32 (m, 10H), 5.15 (s, 2H), 4.66-4.60 (m, 2H), 4.33-4.24 (m, 2H), 4.24-4.19 (m, 2H), 4.16 (s, 2H), 3.92-3.86 (m, 1H), 3.73 (d, *J* = 5.9 Hz, 2H), 2.91-2.82 (m, 2H), 2.66 (s, 6H), 2.02-1.94 (m, 1H), 1.74-1.64 (m, 1H), 1.60-1.51 (m, 3H), 1.31-1.26 (m, 2H), 0.85 (t, *J =* 6.0 Hz, 6H).

### Step 3:

Compound **INT5-2** (1.3 g, 1.7 mmol) was dissolved in a mixed solvent of EtOH (40 mL) and EA (20 mL), and Pd/C (400 mg) was added. The reaction solution was purged three times with hydrogen, and stirred for 16 h under hydrogen atmosphere. The reaction was monitored by LCMS. The reaction solution was filtered, and the filtrate was concentrated under reduced pressure to give the target compound **INT5** (780 mg).

LCMS (ESI) [M+H]⁺=640.4.

### Example 1.6: Synthesis of 1-((2-(2,6-dioxopiperidin-3-yl)-1-oxoisoindol-5-yl)methyl)-3-(4-(3-(methylamino)propyl)phenyl) urea (INT6)

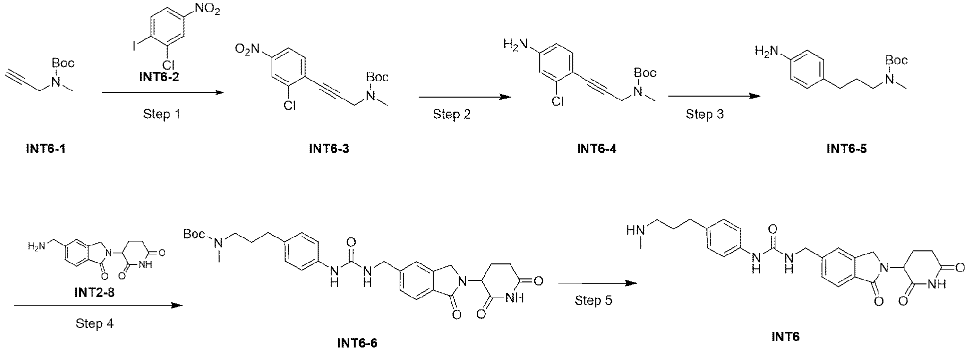

### Step 1:

Compound **INT6-1** (3.0 g, 17.60 mmol) was dissolved in DMF (100 mL), and **INT6-2** (5.0 g, 17.60 mmol), triethylamine (8.9 g, 88.20 mmol), cuprous iodide (335 mg, 1.76 mmol) and triphenylphosphine palladium dichloride (1.2 g, 1.76 mmol) were sequentially added to the reaction solution. The reaction solution was stirred at room temperature for 4 h under nitrogen atmosphere. The reaction was monitored by TLC. The reaction solution was quenched by adding water (100 mL) and extracted with ethyl acetate (100 mL × 3). The organic phases were combined, washed with saturated brine (100 mL × 2), washed with water (100 mL × 2), dried over anhydrous Na₂SO₄ and filtered. The filtrate was concentrated under reduced pressure, and the crude product was purified by column chromatography (petroleum ether:ethyl acetate = 10:1) to give the target compound **INT6-3** (3.5 g).

¹H NMR (400 MHz, DMSO-*d₆*) δ 8.39 (d, *J =* 2.3Hz, 1H), 8.18 (dd, *J =* 8.8 Hz, 2.4 Hz, 1H), 7.84 (d, *J=* 8.6 Hz, 1H), 4.38 (s, 2H), 2.91 (s, 3H), 1.42 (s, 9H).

### Step 2:

Compound **INT6-3** (1.0 g, 3.09 mmol) was dissolved in anhydrous MeOH (20 mL), and THF (20 mL), distilled water (10 mL), zinc powder (2.0 g, 30.86 mmol) and ammonium chloride (1.6 g, 30.86 mmol) were sequentially added to the reaction solution. The reaction solution was stirred at 50 °C for 4 h. The reaction was monitored by LCMS. The reaction solution was filtered, the filtrate was concentrated under reduced pressure, and then EA was added for extraction (50 mL × 3). The organic phases were combined, washed with saturated brine (50 mL × 2), washed with water (50 mL × 2), dried over anhydrous Na₂SO₄ and filtered. The filtrate was concentrated under reduced pressure, and the crude product was purified through a chromatographic column (petroleum ether:ethyl acetate = 5:1) to give the target compound **INT6-4** (900 mg).

LCMS (ESI) [M+H-56]⁺ = 239.0.

¹H NMR (400 MHz, DMSO*-d₆*) δ 7.16 (d, *J =* 8.4 Hz, 1H), 6.64 (d, *J=* 2.1 Hz, 1H), 6.45 (dd, *J =* 8.4 Hz, 2.0 Hz, 1H), 5.78 (s, 2H), 4.22 (s, 2H), 2.86 (s, 3H), 1.41 (s, 9H).

### Step 3:

Compound **INT6-4** (5.0 g, 17.00 mmol) was dissolved in MeOH (50 mL), and ammonium formate (5.36 g, 85.00 mmol) and palladium on carbon (360 mg, 3.40 mmol) were sequentially added. The reaction solution was purged three times with hydrogen and reacted at 60 °C overnight under hydrogen atmosphere. The reaction was monitored by LCMS. The reaction solution was filtered through celite, the filtrate was concentrated under reduced pressure, and the crude product was purified by preparative high-performance liquid chromatography (acetonitrile:H₂O containing 0.05% FA= 5%-50%) to give the target compound **INT6-5** (2.6 g).

LCMS (ESI) [M+H-100]⁺ = 165.1.

¹H NMR (400 MHz, CDCl₃) δ 6.97 (d, *J =* 8.2 Hz, 2H), 6.62 (d, *J =* 8.3 Hz, 2H), 3.18-3.23 (m, 2H), 2.83 (s, 3H), 2.52-2.44 (m, 2H), 1.83-1.72 (m, 2H), 1.45 (s, 9H).

### Step 4:

Compound **INT6-5** (1.0 g, 3.78 mmol) was dissolved in THF (20 mL), and triphosgene (1.50 g, 7.56 mmol) was added. After the addition, the mixture was stirred at room temperature for 30 min. The formation of an intermediate state during the reaction was detected by LCMS. The reaction solution was concentrated under reduced pressure, and DMF (5 mL) was added and dissolved for later use. The hydrochloride of compound **INT2-8** (1.04 g, 3.78 mmol) and triethylamine (3.03 g, 30.04 mmol) were dissolved in DMF (5 mL), and the resulting mixture was slowly added dropwise to the above prepared reaction solution. After the addition, the mixture was stirred at room temperature for 2 h. The reaction was monitored by LCMS. The reaction solution was quenched by adding water (10 mL) and extracted with EA (10 mL × 3). The organic phases were combined, washed with saturated brine (10 mL × 3), dried over anhydrous Na₂SO₄ and filtered. The filtrate was concentrated under reduced pressure, and the crude product was purified through a chromatographic column (methanol:dichloromethane = 0%-7%) to give the target compound **INT6-6** (800 mg).

LCMS (ESI) [M+H-100]⁺ = 464.3.

¹H NMR (400 MHz, CDCl₃) δ 8.97 (s, 1H), 7.57-7.45 (m, 2H), 7.29 (s, 1H), 7.26-7.21 (m, 2H), 7.18 (d, *J =* 7.8 Hz, 1H), 7.04 (d, *J =* 7.7 Hz, 2H), 5.95 (s, 1H), 5.04 (dd, *J =* 13.1, 4.8 Hz, 1H), 4.43-4.30 (m, 2H), 4.24-4.11 (m, 2H), 3.21 (t, *J =* 7.0 Hz, 2H), 2.81 (s, 3H), 2.80-2.63 (m, 2H), 2.50 (t, *J =* 7.1 Hz, 2H), 2.27-2.05 (m, 2H), 1.79-1.74 (m, 2H), 1.43 (s, 9H).

### Step 5:

Compound **INT6-6** (200 mg, 0.35 mmol) was dissolved in DCM (5 mL), and trifluoroacetic acid (1 mL) was slowly added dropwise to the reaction solution. After the addition, the mixture was stirred at room temperature for 30 min. The reaction was monitored by LCMS. The reaction solution was concentrated under reduced pressure to give the trifluoroacetate of the target compound **INT6** (150 mg).

LCMS (ESI) [M+H]⁺ = 464.2.

¹H NMR (400 MHz, DMSO*-d₆*) δ 10.90 (s, 1H), 8.54 (s, 1H), 8.25 (s, 2H), 7.63 (d, *J =* 7.8 Hz, 1H), 7.44 (s, 1H), 7.37 (d, *J =* 7.9 Hz, 1H), 7.27 (d, *J =* 8.4 Hz, 2H), 7.00 (d, *J =* 8.4 Hz, 2H), 6.69 (t, *J =* 6.0 Hz, 1H), 5.03 (dd, *J =* 13.3, 4.9 Hz, 1H), 4.42-4.24 (m, 4H), 2.90-2.83 (m, 2H), 2.82-2.75 (m, 2H), 2.62-2.47 (m, 5H), 2.37-2.24 (m, 1H), 1.96-1.90 (m, 1H), 1.81-1.70 (m, 2H).

### Example 1.7: Synthesis of 2,2-dimethyl-4-(4-(2-(methylsulfonyl)pyrimidin-5-yl)-1H-1,2,3-triazol-1-yl)butanoic acid (INT7)

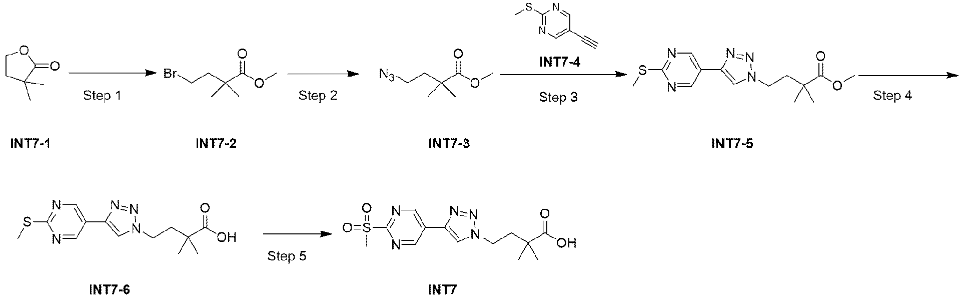

### Step 1:

Compound **INT7-1** (500 mg, 4.39 mmol) was dissolved in extra-dry DCM (5 mL), and boron tribromide (1.15 g, 4.6 mmol) was added dropwise at 0 °C. After the addition, the reaction solution was heated to room temperature and stirred for 24 h. Then, the reaction solution was cooled to 0 °C, and methanol (5 mL) was added dropwise to the reaction solution to quench the reaction. The reaction solution was stirred at room temperature for another 24 h. Saturated aqueous sodium bicarbonate solution (30 mL) was added to the reaction solution, and the resulting mixture was extracted with DCM (30 mL × 3). The organic phases were combined, dried over anhydrous Na₂SO₄ and filtered. The filtrate was concentrated under reduced pressure to give the target compound **INT7-2** (830 mg).

¹H NMR (400 MHz, CDCl₃) δ 3.69 (s, 3H), 3.40-3.27 (m, 2H), 2.20-2.09 (m, 2H), 1.21 (s, 6H).

### Step 2:

Compound **INT7-2** (650 mg, 3.13 mmol) was dissolved in DMF (10 mL), and sodium azide (1.02 g, 15.63 mmol) was added. The reaction solution was heated to 80 °C, stirred overnight and then cooled to room temperature. Water (30 mL) was added to the reaction solution, and the resulting mixture was extracted with MTBE (30 mL × 3). The organic phases were combined, dried over anhydrous Na₂SO₄ and filtered. The filtrate was concentrated under increased pressure to give the target compound **INT7-3** (470 mg).

¹H NMR (400 MHz, CDCl₃) δ 3.69 (s, 3H), 3.31-3.23 (m, 2H), 1.89-1.81 (m, 2H), 1.22 (s, 6H).

### Step 3:

Compounds **INT7-3** (365 mg, 2.13 mmol) and **INT7-4** (320 mg, 2.13 mmol) were dissolved in *tert*-butanol (5 mL) and water (5 mL), and sodium ascorbate (84 mg, 0.43 mmol) and anhydrous copper sulfate (34 mg, 0.21 mmol) were added. The reaction solution was stirred at room temperature for 1 h. Water (25 mL) was added to the reaction solution, and the resulting mixture was extracted with ethyl acetate (30 mL × 3). The organic phases were combined, dried over anhydrous Na₂SO₄ and filtered. The filtrate was concentrated under reduced pressure to evaporate the solvent, and the crude product was purified by column chromatography (PE:EA = 3:1) to give the target compound **INT7-5** (600 mg).

LCMS (ESI) [M+H]⁺ = 322.1.

¹H NMR (400 MHz, CDCl₃) δ 8.95 (s, 2H), 7.85 (s, 1H), 4.53-4.39 (m, 2H), 3.69 (s, 3H), 2.61 (s, 3H), 2.29-2.17 (m, 2H), 1.31 (s, 6H).

### Step 4:

Compound **INT7-5** (500 mg, 1.60 mmol) was dissolved in THF (5 mL) and water (5 mL), and lithium hydroxide (112 mg, 4.70 mmol) was added. The reaction solution was stirred at room temperature for 5 h. 1 N hydrochloric acid was added to the reaction solution to adjust the pH to 3-4. A large amount of white solid was precipitated. The mixture was filtered, and the filter cake was dried to give the target compound **INT7-6** (450 mg).

LCMS (ESI) [M+H]⁺ = 308.0.

¹H NMR (400 MHz, DMSO*-d₆*) δ 12.43 (s, 1H), 9.06 (s, 2H), 8.76 (s, 1H), 4.55-4.31 (m, 2H), 2.56 (s, 3H), 2.15-2.04 (m, 2H), 1.20 (s, 6H).

### Step 5:

Compound **INT7-6** (1.5 g, 4.90 mmol) was dissolved in THF (10 mL) and water (10 mL), and Oxane (15.0 g, 24.5 mmol) was added. The reaction solution was stirred at room temperature overnight. The reaction solution was poured into water (200 mL) and filtered, and the filter cake was dried to give the target compound **INT7** (1.6 g).

LCMS (ESI) [M+H]⁺ = 340.1.

¹H NMR (400 MHz, DMSO*-d₆*) δ 9.48 (s, 2H), 8.98 (s, 1H), 4.54-4.48 (m, 2H), 3.44 (s, 3H), 2.16-2.09 (m, 2H), 1.21 (s, 6H).

### Example 2. Synthesis of Bioactive Compounds (Payloads)

### Example 2.1: Synthesis of N-(3-(2-chloro-4-(3-((2-(2,6-dioxopiperidin-3-yl)-1-oxoisoindolin-5-yl)methyl)ureido)phenyl)pro pyl)-2-hydroxy-N-methylacetamide (PL-1)

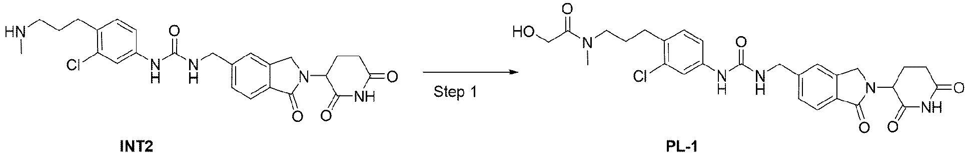

### Step 1:

The trifluoroacetate of compound **INT2** (20 mg, 0.04 mmol) and glycolic acid (3 mg, 0.04 mmol) were dissolved in DMF (3 mL), and triethylamine (12 mg, 0.12 mmol) and 4-(4,6-dimethoxytriazin-2-yl)-4-methylmorpholine hydrochloride (17 mg, 0.06 mmol) were added. The reaction solution was stirred at room temperature for 2 h. The reaction was monitored by LCMS. The reaction solution was directly purified by preparative high-performance liquid chromatography (acetonitrile:H₂O containing 0.05% FA = 5%-50%) to give the target compound **PL-1** (10 mg).

LCMS (ESI) [M+H]⁺ =556.2;

¹H NMR (400 MHz, DMSO*-d₆*) δ 10.97 (s, 1H), 8.95 - 8.86 (m, 1H), 7.69 (d, *J =* 7.8 Hz, 1H), 7.66 (s, 1H), 7.51 (s, 1H), 7.44 (d, *J* = 7.7 Hz, 1H), 7.22 - 7.15 (m, 2H), 6.98 - 6.90 (m, 1H), 5.18 - 5.03 (m, 1H), 4.46 - 4.36 (m, 4H), 4.33- 4.28 (m, 1H), 4.09 - 4.01 (m, 2H), 3.24 - 3.17 (m, 1H), 2.96 - 2.86 (m, 1H), 2.84 (s, 3H), 2.64 - 2.58 (m, 1H), 2.58 - 2.54 (m, 2H), 2.44 - 2.32 (m, 1H), 2.02 - 1.97 (m, 1H), 1.80 - 1.66 (m, 2H).

### Example 2.2: Synthesis of 2-(3-chloro-4-(3-(2-hydroxy-N-methylacetamido)propyl)phenyl)-N-(2-(2,6-dioxopiperidin-3-yl)-1-oxoisoindolin-5-yl)methyl)-2,2-difluoroacetamide (PL-2)

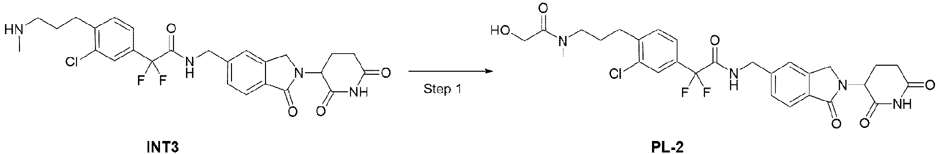

### Step 1:

The hydrochloride of compound **INT3** (40 mg, 0.08 mmol) and glycolic acid (6 mg, 0.08 mmol) were dissolved in DMF (2 mL), and triethylamine (24 mg, 0.24 mmol) and 4-(4,6-dimethoxytriazin-2-yl)-4-methylmorpholine hydrochloride (33 mg, 0.12 mmol) were added. The reaction solution was stirred at room temperature for 2 h. The reaction was monitored by LCMS. The reaction solution was directly purified by preparative high-performance liquid chromatography (CH₃CN:H₂O containing 0.05% FA = 5%-50%) to give the target compound **PL-2** (15 mg).
LCMS (ESI) [M+H]⁺ = 591.2;
¹H NMR (400 MHz, DMSO*-d₆*) δ 10.98 (s, 1H), 9.69 (t, *J =* 5.8 Hz, 1H), 7.68 (d, *J =* 7.8 Hz, 1H), 7.59 (s, 1H), 7.56 (d, *J =* 8.1 Hz, 1H), 7.49 (d, *J =* 8.0 Hz, 1H), 7.42 (s, 1H), 7.37 (d, *J =* 7.9 Hz, 1H), 5.16 - 5.05 (m, 1H), 4.45 (d, *J =* 6.1 Hz, 2H), 4.41 - 4.38 (m, 1H), 4.40 - 4.30 (m, 1H), 4.07 (t, *J =* 4.8 Hz, 2H), 3.30 - 3.23 (m, 2H), 2.97 - 2.88 (m, 1H), 2.86 (s, 3H), 2.74 - 2.67 (m, 2H), 2.64 - 2.57 (m, 1H), 2.44 - 2.32 (m, 1H), 2.03 - 1.98 (m, 1H), 1.84 - 1.72 (m, 2H).

### Example 2.3: Synthesis of N-(3-(2-chloro-4-(3-((2-(2,6-dioxopiperidin-3-yl)-1,3-dioxoisoindol-5-yl)methyl)ureido)phenyl)p ropyl)-2-hydroxy-N-methylacetamide (PL-3)

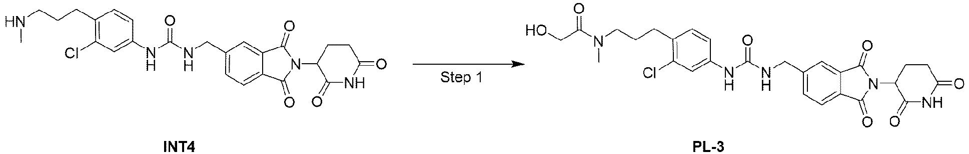

### Step 1:

Compound **INT4** (50.0 mg, 0.10 mmol) was dissolved in DMF (1 mL), and glycolic acid (7.0 mg, 0.10 mmol), 2-(7-azabenzotriazol-1-yl)-N,N,N',N'-tetramethyluronium hexafluorophosphate (38 mg, 0.10 mmol) and N,N-diisopropylethylamine (32 mg, 0.25 mmol) were sequentially added. The reaction solution was stirred at room temperature for 1 h. The reaction was monitored by LCMS. The reaction solution was directly purified by preparative high-performance liquid chromatography (acetonitrile:H₂O containing 0.05% FA = 5%-50%) to give the target compound **PL-3** (15 mg).
LCMS (ESI) [M+H]⁺ =570.3;
¹H NMR (400 MHz, DMSO*-d₆*) δ 11.11 (s, 1H), 8.86 (d, *J =* 5.0 Hz, 1H), 7.89 (d, *J =* 7.7 Hz, 1H), 7.82 (s, 1H), 7.79 (d, *J =* 7.7 Hz, 1H), 7.63 (d, *J =* 1.6 Hz, 1H), 7.24-7.15 (m, 2H), 6.92 (t, *J* = 6.4 Hz, 1H), 5.14 (dd, *J =* 13.0, 5.4 Hz, 1H), 4.51-4.44 (m, 2H), 4.44-4.32 (m, 1H), 4.08-3.99 (m, 2H), 3.37-3.35 (m, 1H), 3.25-3.16 (m, 1H), 2.95-2.86 (m, 1H), 2.84 (d, *J =* 5.1 Hz, 3H), 2.65 (d, *J* = 19.8 Hz, 1H), 2.59-2.53 (m, 3H), 2.11-2.01 (m, 1H), 1.81-1.65 (m, 2H).

### Example 2.4: Synthesis of N-(2-chloro-4-(3-(2-(2,6-dioxopiperidin-3-yl)-1-oxoisoindolin-5-methyl)ureido)phenethoxy)ethyl -2-hydroxy-N-methylacetamide (PL-4)

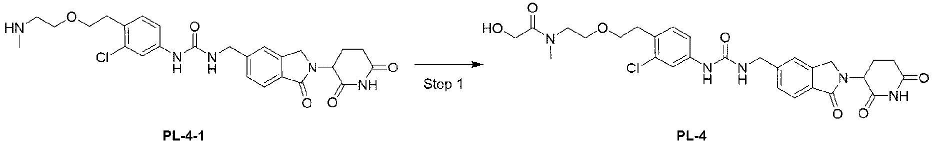

### Step 1:

Compound **PL-4-1** (50.0 mg, 0.09 mmol, prepared with reference to the patent document WO2021198965) was dissolved in DMF (1 mL), and glycolic acid (6.9 mg, 0.09 mmol), 2-(7-azabenzotriazol-1-yl)-N,N,N',N'-tetramethyluronium hexafluorophosphate (37 mg, 0.09 mmol) and N,N-diisopropylethylamine (31 mg, 0.22 mmol) were sequentially added. The reaction solution was stirred at room temperature for 1 h. The reaction was monitored by LCMS. The reaction solution was directly purified by preparative high-performance liquid chromatography (acetonitrile:H₂O containing 0.05% FA = 5%-50%) to give the target compound **PL-4** (23 mg).
LCMS (ESI) [M+H]⁺ = 586.4;
¹H NMR (400 MHz, DMSO*-d₆*) δ 10.97 (s, 1H), 8.77 (s, 1H), 7.68 (dd, *J =* 9.5, 4.8 Hz, 2H), 7.51 (s, 1H), 7.44 (d, *J =* 7.9 Hz, 1H), 7.20-7.11 (m, 2H), 6.80 (t, *J =* 5.8 Hz, 1H), 5.10 (dd, *J =* 13.3, 5.1 Hz, 1H), 4.48-4.26 (m, 4H), 4.05 (d, *J =* 17.8 Hz, 2H), 3.57-3.53 (m, 2H), 3.52-3.48 (m, 2H), 3.46-3.41 (m, 2H), 3.36-3.30 (m, 1H), 2.96-2.84 (m, 2H), 2.83 (d, *J =* 4.6 Hz, 3H), 2.60-2.51 (m, 1H), 2.45-2.30 (m, 1H), 2.02-1.95 (m, 1H).

### Example 2.5: Synthesis of N-(3-(4-(3-(2-(2,6-dioxopiperidin-3-yl)-1-oxoisoindolin-5-methyl)ureido)phenyl)propyl)-2-hydro xy-N-methylacetamide (PL-5)

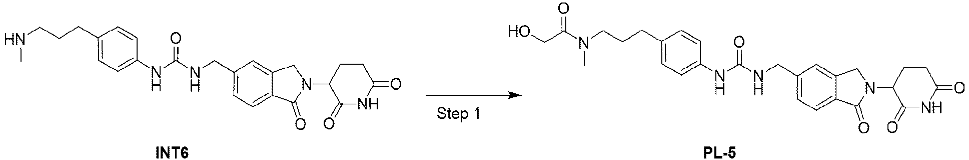

### Step 1:

The trifluoroacetate of compound **INT6** (50.0 mg, 0.10 mmol) was dissolved in DMF (1 mL), and glycolic acid (8.0 mg, 0.10 mmol), 2-(7-azabenzotriazol-1-yl)-N,N,N',N'-tetramethyluronium hexafluorophosphate (41 mg, 0.10 mmol) and N,N-diisopropylethylamine (35 mg, 0.25 mmol) were sequentially added. The reaction solution was stirred at room temperature for 1 h. The reaction was monitored by LCMS. The reaction solution was directly purified by preparative high-performance liquid chromatography (acetonitrile:H₂O containing 0.05% FA = 5%-50%) to give the target compound **PL-5** (18 mg).

LCMS (ESI) [M+H]⁺ = 522.4.

¹H NMR (400 MHz, DMSO*-d₆*) δ 10.97 (s, 1H), 8.52 (d, *J =* 5.7 Hz, 1H), 7.69 (d, *J =* 7.8 Hz, 1H), 7.51 (s, 1H), 7.44 (d, *J =* 7.8 Hz, 1H), 7.35-7.27 (m, 2H), 7.07 (t, *J =* 6.8 Hz, 2H), 6.68 (t, *J* = 5.6 Hz, 1H), 5.10 (dd, *J =* 13.3, 5.0 Hz, 1H), 4.48-4.27 (m, 4H), 4.03-3.99 (m, 2H), 3.85 (s, 1H), 3.31 (t, *J =* 7.3 Hz, 1H), 3.20-3.10 (m, 1H), 2.97-2.86 (m, 1H), 2.83 (s, 3H), 2.61-2.57 (m, 1H), 2.49-2.42 (m, 2H), 2.41-2.30 (m, 1H), 2.04-1.93 (m, 1H), 1.82-1.67 (m, 2H).

### Example 3. Synthesis of Toxin-Linkers

### Example 3.1: Synthesis of N-((165,19S)-1-(2-chloro-4-(3-((2-(2,6-dioxopiperidin-3-yl)-1-oxoisoindolin-5-yl)methyl)ureido) phenyl)-16-(4-(dipropylamino)butyl)-6,20-dimethyl-7,12,15,18-tetraoxo-3,9-dioxa-6,11,14,17-tet raazaheneicosan-19-yl)-6-(2-(methylsulfonyl)pyrimidin-5-yl)hex-5-ynamide (DL-1)

### Method I:

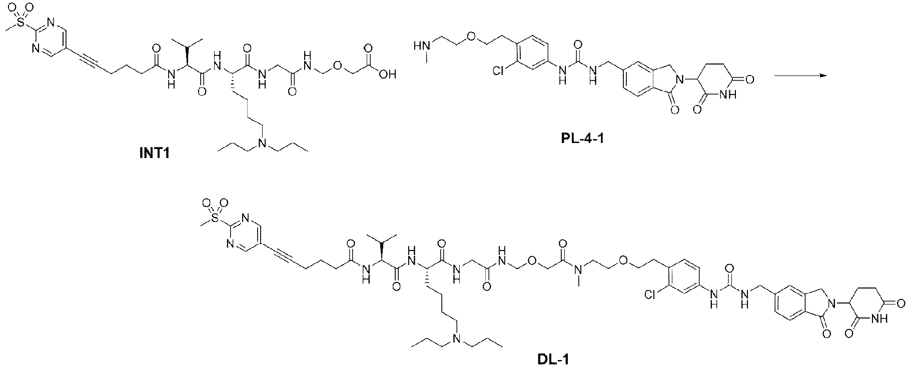

Compound **INT1** (22 mg) was dissolved in DMF (1 mL), and HATU (15 mg) and N,N-diisopropylethylamine (100 µL) were sequentially added. The mixture was stirred at room temperature for 5 min, and compound **PL-4-1** (16 mg) was added. After the addition, the reaction solution was stirred at room temperature for 1 h and purified by preparative high-performance liquid chromatography (acetonitrile:water = 5%-50%) to give the formate of the target compound **DL-1** (18 mg).

### LCMS (ESI) [M+H]⁺ = 1233.6.

¹H NMR (400 MHz, DMSO-*d6*) δ 10.97 (s, 1H), 9.11 (s, 2H), 8.82 (s, 1H), 8.63-8.57 (m, 1H), 8.16 (s, 2H), 8.03 (d, *J =* 7.2 Hz, 1H), 7.93 (d, *J =* 8.4 Hz, 1H), 7.70-7.66 (m, 2H), 7.51 (s, 1H), 7.44 (d, *J* = 8.1 Hz, 1H), 7.19-7.14 (m, 2H), 6.85 (s, 1H), 5.10 (dd, *J* = 13.3, 5.0 Hz, 1H), 4.60-4.55 (m, 2H), 4.48-4.38 (m, 4H), 4.34-4.28 (m, 1H), 4.24-4.16 (m, 2H), 4.11 (d, *J =* 15.5 Hz, 2H), 3.74-3.68 (m, 2H), 3.56-3.45 (m, 6H), 3.41 (s, 3H), 2.96-2.89 (m, 1H), 2.87-2.78 (m, 6H), 2.62 (s, 1H), 2.59-2.51 (m, 4H), 2.42-2.29 (m, 4H), 2.02-1.96 (m, 2H), 1.85-1.78 (m, 2H), 1.70-1.65 (m, 1H), 1.59-1.53 (m, 1H), 1.45-1.38 (m, 7H), 1.33-1.27 (m, 2H), 0.85-0.81 (m, 12H).

### Method II:

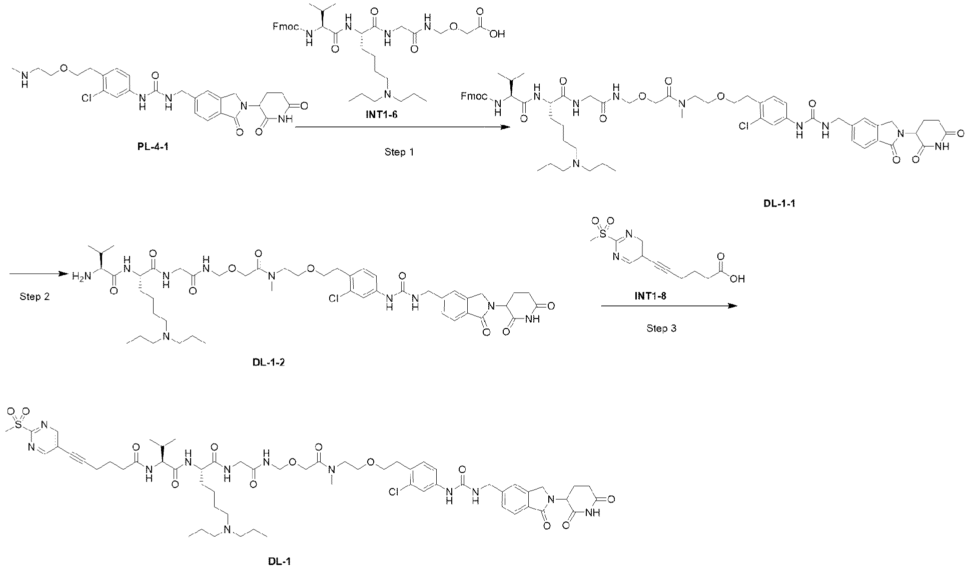

### Step 1:

Compound **PL-4-1** (200 mg, 0.38 mmol) was dissolved in DMF (2 mL), and compound **IN1-6** (263 mg, 0.38 mmol), 2-(7-azabenzotriazol-1-yl)-N,N,N',N'-tetramethyluronium hexafluorophosphate (144 mg, 0.38 mmol) and N,N-diisopropylethylamine (122 mg, 0.95 mmol) were sequentially added. After the addition, the reaction solution was stirred at room temperature for 1 h. The reaction was monitored by LCMS. Methyl tert-butyl ether (80 mL) was slowly added to the reaction solution for dilution, the mixture was left to stand for 10 min, and then the supernatant was removed. The residue was directly concentrated under reduced pressure using an oil pump to remove excess solvent, and the crude product was purified by preparative TLC (dichloromethane:methanol = 8:1) to give the target compound **DL-1-1** (280 mg).

LCMS (ESI) [M+H]⁺ = 1205.5.

¹H NMR (400 MHz, DMSO*-d₆*) δ 10.99 (s, 1H), 8.81 (s, 1H), 8.65 (t, *J =* 8.3 Hz, 1H), 8.30-8.18 (m, 1H), 8.04 (d, *J =* 7.7 Hz, 1H), 7.89 (d, *J =* 7.5 Hz, 2H), 7.74-7.70 (m, 2H), 7.67 (d, *J =* 5.7 Hz, 2H), 7.51 (s, 1H), 7.47-7.39 (m, 4H), 7.32 (t, *J =* 7.4 Hz, 2H), 7.20-7.13 (m, 2H), 6.83 (t, *J=* 5.4 Hz, 1H), 5.10 (dd, *J =* 13.3, 5.1 Hz, 1H), 4.61-4.54 (m, 2H), 4.43-4.39 (m, 2H), 4.35-4.29 (m, 2H), 4.21 (d, *J =* 6.8 Hz, 2H), 4.11 (d, *J =* 15.8 Hz, 2H), 3.92-3.85 (m, 1H), 3.72 (t, *J =* 6.0 Hz, 2H), 3.57-3.45 (m, 6H), 2.96-2.87 (m, 2H), 2.86 (s, 2H), 2.83 (d, *J =* 6.2 Hz, 2H), 2.78 (s, 2H), 2.61 (d, *J* = 4.5 Hz, 1H), 2.59-2.56 (m, 1H), 2.42-2.32 (m, 2H), 2.04-1.94 (m, 3H), 1.74-1.51 (m, 4H), 1.50-1.37 (m, 6H), 1.32-1.25 (m, 3H), 0.87-0.81 (m, 12H).

### Step 2:

Compound **DL-1-1** (100 mg, 0.08 mmol) was dissolved in DMF (2 mL), and diethylamine (25.6 mg, 0.32 mmol) was added to the reaction solution. The reaction solution was stirred at room temperature for 1 h. The reaction was monitored by LCMS. The reaction solution was directly placed under an oil pump and concentrated under reduced pressure to give the target compound **DL-1-2** (60 mg), which was directly used in the next step.

LCMS (ESI) [M+H]⁺ = 983.5.

### Step 3:

Compound **DL-1-2** (60 mg, 0.06 mmol), compound **INT1-8** (18 mg, 0.06 mmol), 2-(7-azabenzotriazol-1-yl)-N,N,N',N'-tetramethyluronium hexafluorophosphate (25 mg, 0.06 mmol) and N,N-diisopropylethylamine (21 mg, 0.15 mmol) were dissolved in DMF (3 mL). The reaction solution was stirred at room temperature for 1 h. The reaction was monitored by LCMS. The reaction solution was directly purified by preparative high-performance liquid chromatography (acetonitrile:H₂O containing 0.05% FA = 5%-50%) to give the formate of the target compound **DL-1** (23 mg).

LCMS (ESI) [M+H]⁺ = 1233.9.

¹H NMR (400 MHz, DMSO*-d₆*) δ 10.97 (s, 1H), 9.11 (s, 2H), 8.82 (s, 1H), 8.63-8.57 (m, 1H), 8.16 (s, 2H), 8.03 (d, *J =* 7.2 Hz, 1H), 7.93 (d, *J =* 8.4 Hz, 1H), 7.70-7.66 (m, 2H), 7.51 (s, 1H), 7.44 (d, *J* = 8.1 Hz, 1H), 7.19-7.14 (m, 2H), 6.85 (s, 1H), 5.10 (dd, *J* = 13.3, 5.0 Hz, 1H), 4.60-4.55 (m, 2H), 4.48-4.38 (m, 4H), 4.34-4.28 (m, 1H), 4.24-4.16 (m, 2H), 4.11 (d, *J =* 15.5 Hz, 2H), 3.74-3.68 (m, 2H), 3.56-3.45 (m, 6H), 3.41 (s, 3H), 2.96-2.89 (m, 1H), 2.87-2.78 (m, 6H), 2.62 (s, 1H), 2.59-2.51 (m, 4H), 2.42-2.29 (m, 4H), 2.02-1.96 (m, 2H), 1.85-1.78 (m, 2H), 1.70-1.65 (m, 1H), 1.59-1.53 (m, 1H), 1.45-1.38 (m, 7H), 1.33-1.27 (m, 2H), 0.85-0.81 (m, 12H).

### Example 3.2: Synthesis of N-(14S,17S)-1-(2-chloro-4-(3-(2-(2,6-dioxopiperidin-3-yl)-1-oxoisoindolin-5-yl)methyl)ureido)p henyl)-14-(4-(dipropylamino)butyl)-4,18-dimethyl-5,10,13,16-tetraoxo-7-oxa-4,9,12,15-tetraazan onadecan-17-yl)-6-(2-(methylsulfonyl)pyrimidin-5-yl)hex-5-hexanamide (DL-2)

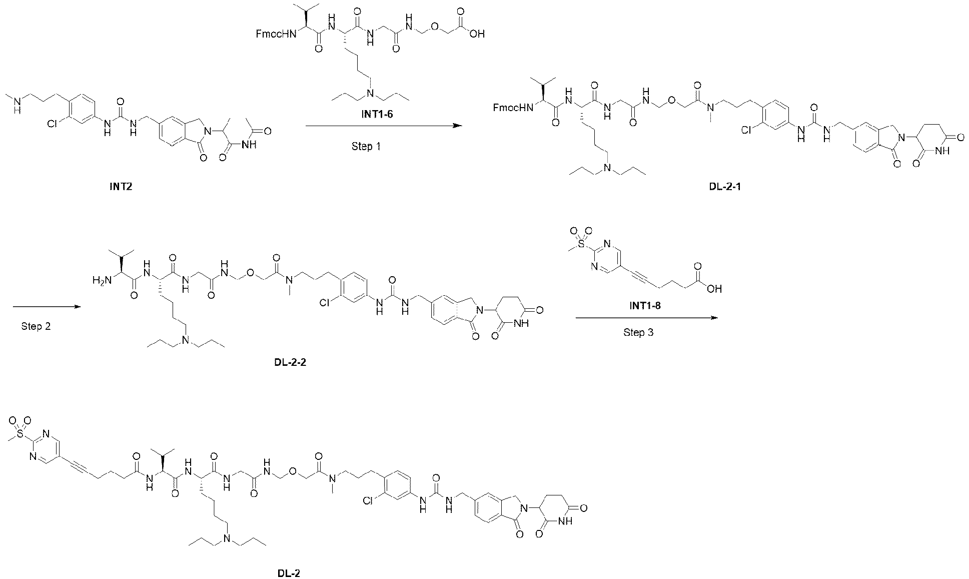

### Step 1:

The trifluoroacetate of compound **INT2** (100 mg, 0.20 mmol) and compound **INT1-6** (139 mg, 0.20 mmol) were dissolved in DMF (5 mL), and N,N-diisopropylethylamine (77 mg, 0.60 mmol) and 2-(7-azabenzotriazol-1-yl)-N,N,N',N'-tetramethyluronium hexafluorophosphate (76 mg, 0.20 mmol) were added. The reaction solution was stirred at room temperature for 1 h. The reaction was monitored by LCMS. Water (45 mL) was added to the reaction solution, and the resulting mixture was extracted with ethyl acetate (25 mL × 3). The organic phases were combined, washed with saturated brine (30 mL × 3), dried over anhydrous Na₂SO₄ and filtered. The filtrate was concentrated under reduced pressure to give a crude product. The crude product was purified by preparative thin-layer chromatography (DCM:MeOH = 10:1) to give the target compound **DL-2-1** (100 mg).

LCMS (ESI) [M+H]⁺ =1175.3.

¹H NMR (400 MHz, DMSO*-d₆*) δ 10.98 (s, 1H), 8.85 - 8.73 (m, 1H), 8.68 - 8.56 (m, 1H), 8.31 - 8.18 (m, 1H), 8.07 - 7.97 (m, 1H), 7.88 (d, *J =* 7.5 Hz, 2H), 7.74 - 7.64 (m, 4H), 7.51 (s, 1H), 7.46 - 7.39 (m, 4H), 7.31 (t, *J =* 6.2 Hz, 2H), 7.20 - 7.15 (m, 2H), 6.86 - 6.77 (m, 1H), 5.17 - 5.04 (m, 1H), 4.61 - 4.54 (m, 2H), 4.47 - 4.38 (m, 4H), 4.34 - 4.27 (m, 2H), 4.23 (d, *J =* 6.8 Hz, 2H), 4.10 (s, 2H), 3.92 - 3.84 (m, 1H), 3.76 - 3.67 (m, 2H), 2.97 - 2.86 (m, 2H), 2.81 (d, *J* = 21.5 Hz, 4H), 2.63 - 2.53 (m, 6H), 2.40 - 2.33 (m, 2H), 2.05 - 1.94 (m, 4H), 1.77 - 1.64 (m, 4H), 1.60 - 1.48 (m, 2H), 1.47 - 1.34 (m, 6H), 0.87 - 0.83 (m, 6H), 0.83 - 0.77 (m, 6H).

### Step 2:

Compound **DL-2-1** (50 mg, 0.04 mmol) was dissolved in DMF (2 mL), and diethylamine (15 mg, 0.20 mmol) was added. The reaction solution was stirred at room temperature for 2 h. The reaction was monitored by LCMS. The reaction solution was concentrated under reduced pressure to give the target compound **DL-2-2** (50 mg), which was directly used in the next step.

LCMS (ESI) [M+H]⁺ = 953.5.

### Step 3:

Compound **DL-2-2** (50 mg, 0.04 mmol) and compound **INT1-8** (14 mg, 0.04 mmol) were dissolved in DMF (3 mL), and N,N-diisopropylethylamine (15 mg, 0.12 mmol) and 2-(7-azabenzotriazol-1-yl)-N,N,N',N'-tetramethyluronium hexafluorophosphate (15 mg, 0.04 mmol) were added. The reaction solution was stirred at room temperature for 1 h. The reaction was monitored by LCMS. The reaction solution was directly purified by preparative high-performance liquid chromatography (CH₃CN:H₂O containing 0.05% FA = 5%-50%) to give the formate of the target compound **DL-2** (10 mg).

LCMS (ESI) [M+H]⁺ = 1203.6.

¹H NMR (400 MHz, DMSO*-d₆*) δ 10.97 (s, 1H), 9.11 (s, 2H), 8.80 (d, *J =* 5.4 Hz, 1H), 8.61 (t, *J* = 6.5 Hz, 1H), 8.20 - 8.15 (m, 2H), 8.05 - 8.03 (m, 1H), 7.93 (d, *J =* 8.3 Hz, 1H), 7.69 (d, *J =* 7.8 Hz, 1H), 7.65 (s, 1H), 7.51 (s, 1H), 7.44 (d, *J =* 7.9 Hz, 1H), 7.21 - 7.16 (m, 2H), 6.86 - 6.80 (m, 1H), 5.10 (dd, *J =* 13.4, 5.0 Hz, 1H), 4.61 - 4.56 (m, 2H), 4.47 - 4.29 (m, 4H), 4.22 - 4.14 (m, 2H), 4.10 (s, 2H), 3.72 - 3.69 (m, 2H), 3.41 (s, 3H), 2.95 - 2.88 (m, 2H), 2.86 (s, 2H), 2.79 (s, 1H), 2.63 - 2.54 (m, 12H), 2.41 - 2.31 (m, 4H), 2.02 - 1.97 (m, 2H), 1.84 - 1.78 (m, 2H), 1.70 - 1.66 (m, 2H), 1.59 - 1.54 (m, 1H), 1.47 - 1.42 (m, 6H), 1.31 - 1.26 (m, 2H), 0.86 - 0.82 (m, 12H).

### Example 3.3: Synthesis of N-((14S,17S)-1-(2-chloro-4-(2-(((2-(2,6-dioxopiperidin-3-yl)-1-oxoisoindolin-5-yl)methyl)amino )-1,1-difluoro-2-oxoethyl)phenyl)-14-(4-(dipropylamino)butyl)-4,18-dimethyl-5,10,13,16-tetraox o-7-oxa-4,9,12,15-tetraazanonadecan-17-yl)-6-(2-(methylsulfonyl)pyrimidin-5-yl)hex-5-ynamide (DL-3)

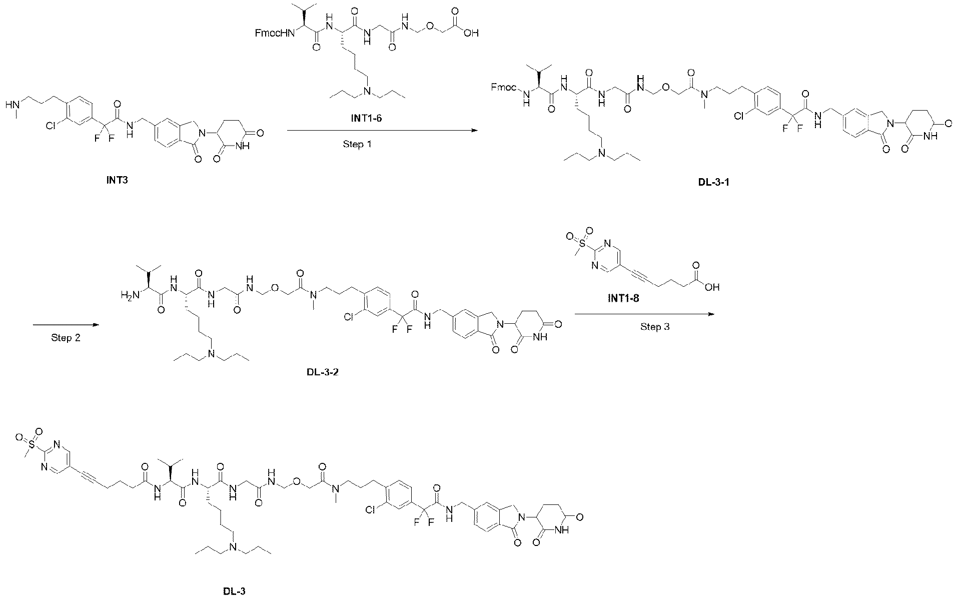

### Step 1:

The hydrochloride of compound **INT3** (165 mg, 0.31 mmol) was dissolved in DMF (8 mL), and compound **INT1-6** (216 mg, 0.31 mmol), N,N-diisopropylethylamine (80 mg, 0.62 mmol) and 4-(4,6-dimethoxytriazin-2-yl)-4-methylmorpholine hydrochloride (129 mg, 0.47 mmol) were sequentially added. The reaction solution was stirred at room temperature for 1 h. The reaction was monitored by LCMS. Water (45 mL) was added to the reaction solution, and the resulting mixture was extracted with ethyl acetate (25 mL × 3). The organic phases were combined, washed with saturated brine (30 mL × 3), dried over anhydrous Na₂SO₄ and filtered. The filtrate was concentrated under reduced pressure to give a crude product. The crude product was purified by preparative thin-layer chromatography (DCM:MeOH = 10:1) to give the target compound **DL-3-1** (150 mg).

LCMS (ESI) [M+H]⁺ = 1210.2.

¹H NMR (400 MHz, DMSO*-d₆*) δ 10.98 (s, 1H), 9.68 (t, *J =* 6.0 Hz, 1H), 8.70 - 8.58 (m, 1H), 8.29 - 8.19 (m, 1H), 8.02 (t, *J =* 9.1 Hz, 1H), 7.88 (d, *J =* 7.6 Hz, 2H), 7.75 - 7.70 (m, 2H), 7.67 (d, *J* = 7.9 Hz, 1H), 7.58 (s, 1H), 7.56 - 7.52 (m, 1H), 7.51 - 7.44 (m, 2H), 7.44 - 7.37 (m, 4H), 7.31 (t, *J =* 7.0 Hz, 2H), 5.33 (t, *J =* 4.6 Hz, 1H), 5.13 - 5.06 (m, 1H), 4.61 - 4.55 (m, 2H), 4.45 (d, *J =* 5.9 Hz, 2H), 4.30 (s, 1H), 4.23 - 4.20 (m, 2H), 4.11 - 4.09 (m, 1H), 3.87 (t, *J =* 8.2 Hz, 1H), 3.77 - 3.65 (m, 2H), 2.94 - 2.89 (m, 1H), 2.87 - 2.79 (m, 3H), 2.69 - 2.66 (m, 2H), 2.64 - 2.60 (m, 1H), 2.59 - 2.56 (m, 1H), 2.43 - 2.35 (m, 2H), 2.34 - 2.32 (m, 1H), 2.28 - 2.22 (m, 2H), 2.05 - 1.93 (m, 6H), 1.78 - 1.71 (m, 2H), 1.68 - 1.61 (m, 2H), 1.56 - 1.52 (m, 1H), 1.47 - 1.44 (m, 1H), 1.37 - 1.26 (m, 8H), 0.86 - 0.78 (m, 12H).

### Step 2:

Compound **DL-3-1** (100 mg, 0.08 mmol) was dissolved in DMF (2 mL), and diethylamine (2 mg, 0.24 mmol) was added. The reaction solution was stirred at room temperature for 2 h. The reaction was monitored by LCMS. The reaction solution was concentrated under reduced pressure to give the target compound **DL-3-2** (70 mg), which was directly used in the next step.

LCMS (ESI) [M+H]⁺ = 988.3.

### Step 3:

Compound **DL-3-2** (70 mg, 0.06 mmol) and compound **INT1-8** (15 mg, 0.06 mmol) were dissolved in DMF (2 mL), and N,N-diisopropylethylamine (23 mg, 0.18 mmol) and 2-(7-azabenzotriazol-1-yl)-N,N,N',N'-tetramethyluronium hexafluorophosphate (23 mg, 0.06 mmol) were added. The reaction solution was stirred at room temperature for 1 h. The reaction was monitored by LCMS. The reaction solution was purified by preparative high-performance liquid chromatography (CH₃CN:H₂O containing 0.05% FA = 5%-50%) to give the formate of the target compound **DL-3** (15 mg).

LCMS (ESI) [M+H]⁺ = 1238.4.

¹H NMR (400 MHz, DMSO*-d₆*) δ 10.98 (s, 1H), 9.67 (t, *J =* 6.4 Hz, 1H), 9.11 (s, 2H), 8.59 (t, *J* = 6.0 Hz, 1H), 8.23 (s, 1H), 8.17 (t, *J* = 5.8 Hz, 1H), 8.02 (t, *J* = 7.2 Hz, 1H), 7.94 (d, *J =* 8.5 Hz, 1H), 7.68 (d, *J =* 7.8 Hz, 1H), 7.60 - 7.57 (m, 1H), 7.57 - 7.53 (m, 1H), 7.52 - 7.47 (m, 1H), 7.42 (d, *J* = 5.9 Hz, 1H), 7.37 (d, *J =* 7.9 Hz, 1H), 5.16 - 5.04 (m, 1H), 4.62 - 4.55 (m, 2H), 4.47 - 4.39 (m, 3H), 4.29 - 4.25 (m, 1H), 4.20 - 4.14 (m, 2H), 4.12 (d, *J =* 5.5 Hz, 2H), 3.75 - 3.66 (m, 2H), 3.41 (s, 3H), 2.96 - 2.89 (m, 1H), 2.87 (s, 3H), 2.72 - 2.69 (m, 1H), 2.68 - 2.66 (m, 1H), 2.64 - 2.58 (m, 1H), 2.57 - 2.52 (m, 4H), 2.42 - 2.36 (m, 2H), 2.35 - 2.31 (m, 2H), 2.31 - 2.25 (m, 6H), 2.04 - 1.96 (m, 3H), 1.84 - 1.72 (m, 4H), 1.40 - 1.28 (m, 8H), 0.85 - 0.78 (m, 12H).

### Example 3.4: Synthesis of N-((13S,16S)-1-(2-chloro-4-(3-((2-(2,6-dioxopiperidin-3-yl)-1-oxoisoindolin-5-yl)methyl)ureido) phenoxy)-13-(4-(dipropylamino)butyl)-3,17-dimethyl-4,9,12,15-tetraoxo-6-oxa-3,8,11,14-tetraaz anonadecan-16-yl)-6-(2-(methylsulfonyl)pyrimidin-5-yl)hex-5-ynamide (DL-4)

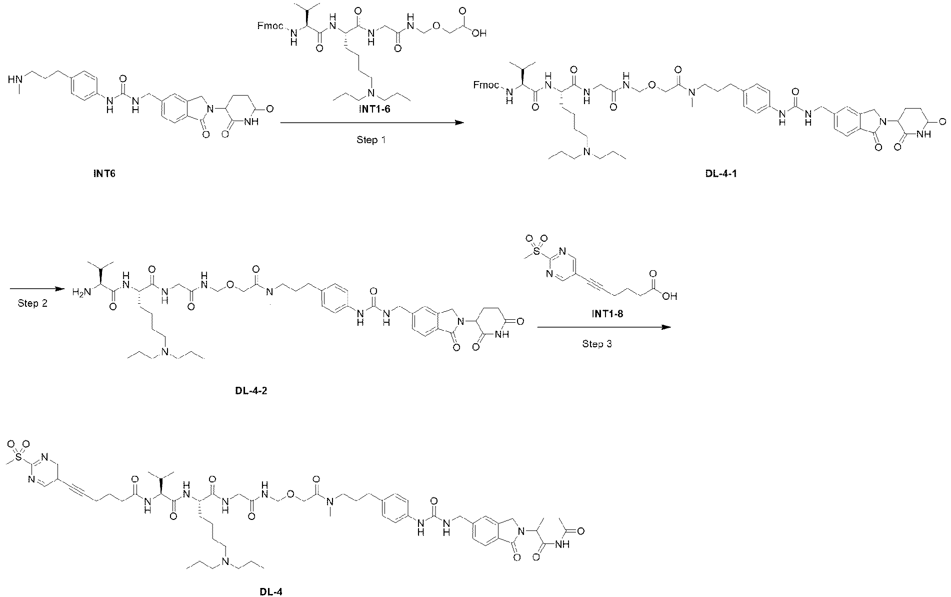

### Step 1:

Compound **INT6** (150 mg, 0.32 mmol) was dissolved in DMF (2 mL), and compound **INT1-6** (192 mg, 0.32 mmol), 2-(7-azabenzotriazol-1-yl)-N,N,N',N'-tetramethyluronium hexafluorophosphate (123 mg, 0.32 mmol) and N,N-diisopropylethylamine (104 mg, 0.80 mmol) were sequentially added. After the addition, the reaction solution was stirred at room temperature for 1 h. The reaction was monitored by LCMS. Methyl *tert*-butyl ether (80 mL) was slowly added to the reaction solution for dilution, the mixture was left to stand for 10 min, and then the supernatant was removed. The residue was directly concentrated under reduced pressure to remove excess solvent, and the crude product was purified by preparative TLC (dichloromethane:methanol = 8:1) to give the target compound **DL-4-1** (250 mg).

LCMS (ESI) [M+H]⁺ = 1141.7.

¹H NMR (400 MHz, DMSO*-d₆*) δ 10.98 (s, 1H), 8.65 (t, *J =* 6.2 Hz, 1H), 8.54 (d, *J =* 5.3 Hz, 1H), 8.27-8.19 (m, 1H), 8.08-8.01 (m, 1H), 7.89 (d, *J =* 7.6 Hz, 2H), 7.75-7.66 (m, 3H), 7.51 (s, 1H), 7.46-7.39 (m, 4H), 7.35-7.29 (m, 4H), 7.07 (t, *J =* 9.1 Hz, 2H), 6.74-6.67 (m, 1H), 5.10 (dd, *J =* 13.3, 5.0 Hz, 1H), 4.63-4.53 (m, 2H), 4.41 (d, *J =* 6.2 Hz, 2H), 4.35-4.20 (m, 5H), 4.11-4.05 (m, 2H), 3.88 (t, *J =* 8.2 Hz, 1H), 3.73 (d, *J =* 2.5 Hz, 2H), 3.31-3.24 (m, 4H), 3.21-3.15 (m, 2H), 2.88-2.85 (m, 1H), 2.80-2.77 (m, 3H), 2.64-2.56 (m, 2H), 2.48-2.42 (m, 2H), 2.40-2.33 (m, 1H), 2.04-1.93 (m, 3H), 1.82-1.60 (m, 4H), 1.56-1.42 (m, 7H), 1.33-1.27 (m, 2H), 0.88-0.83 (m, 12H). Step 2:
Compound **DL-4-1** (100 mg, 0.08 mmol) was dissolved in DMF (2 mL), and diethylamine (25.6 mg, 0.32 mmol) was added to the reaction solution. The reaction solution was stirred at room temperature for 1 h. The reaction was monitored by LCMS. The reaction solution was concentrated under reduced pressure to give the target compound **DL-4-2** (55 mg), which was directly used in the next step.

LCMS (ESI) [M+H]⁺ = 919.6.

### Step 3:

Compound **DL-4-2** (55 mg, 0.06 mmol), compound **INT1-8** (18 mg, 0.06 mmol), 2-(7-azabenzotriazol-1-yl)-N,N,N',N'-tetramethyluronium hexafluorophosphate (25 mg, 0.06 mmol) and N,N-diisopropylethylamine (21 mg, 0.15 mmol) were added to DMF (2 mL). The reaction solution was stirred at room temperature for 1 h. The reaction was monitored by LCMS. The reaction solution was directly purified by preparative high-performance liquid chromatography (acetonitrile:H₂O containing 0.05% FA = 5%-50%) to give the formate of the target compound **DL-4** (15 mg).

LCMS (ESI) [M+H]⁺ = 1169.7.

¹H NMR (400 MHz, DMSO*-d₆*) δ 10.97 (s, 1H), 9.11 (s, 2H), 8.67-8.57 (m, 2H), 8.23-8.17 (m, 2H), 8.09-8.03 (m, 1H), 7.95 (d, *J =* 8.5 Hz, 1H), 7.69 (d, *J =* 7.8 Hz, 1H), 7.51 (s, 1H), 7.44 (d, *J* = 7.9 Hz, 1H), 7.35-7.29 (m, 2H), 7.07 (t, *J =* 8.6 Hz, 2H), 6.85-6.78 (m, 1H), 5.10 (dd, *J =* 13.2, 5.0 Hz, 1H), 4.62-4.54 (m, 2H), 4.44-4.28 (m, 4H), 4.23-4.14 (m, 2H), 4.10-4.07 (m, 2H), 3.74-3.69 (m, 4H), 3.41 (s, 3H), 3.30-3.23 (m, 2H), 3.20-3.14 (m, 1H), 2.96-2.89 (m, 1H), 2.81-2.73 (m, 3H), 2.60-2.57 (m, 1H), 2.56-2.52 (m, 6H), 2.48-2.43 (m, 2H), 2.38-2.32 (m, 2H), 2.02-1.95 (m, 2H), 1.86-1.75 (m, 3H), 1.72-1.66 (m, 2H), 1.60-1.52 (m, 1H), 1.48-1.40 (m, 6H), 1.33-1.25 (m, 2H), 0.84 (t, *J =* 6.8 Hz, 12H).

### Example 3.5: Synthesis of (2S)-N-(2-(((2-((3-(2-chloro-4-(3-((2-(2,6-dioxopiperidin-3-yl)-1-oxoisoindolin-5-yl)methyl)urei do)phenyl)propyl)(methyl)amino)-2-oxoethoxy)methyl)amino)-2-oxoethyl)-2-((S)-2-(2,2-dimeth yl-4-(4)-(2-(methylsulfonyl)pyrimidin-5-yl)-1H-1,2,3-triazol-1-yl)butyramido)-3-methylbutyrami do)-6-(dimethylamino)hexanamide (DL-5)

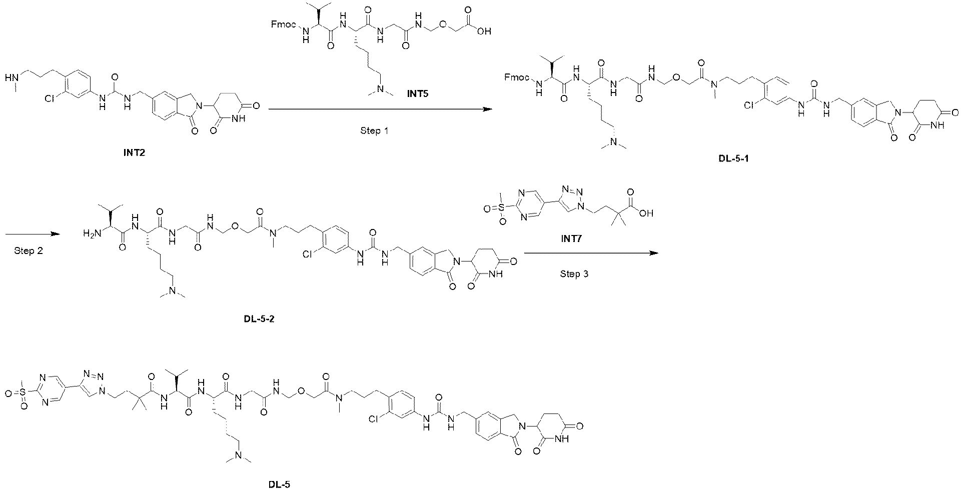

### Step 1:

Compound **INT2** (100 mg, 0.20 mmol) was dissolved in DMF (2 mL), and compound **INT5** (128 mg, 0.20 mmol), 2-(7-azabenzotriazol-1-yl)-N,N,N',N'-tetramethyluronium hexafluorophosphate (76 mg, 0.20 mmol) and N,N-diisopropylethylamine (26 mg, 0.50 mmol) were sequentially added. After the addition, the reaction solution was stirred at room temperature for 1 h. The reaction was monitored by LCMS. Methyl *tert*-butyl ether (80 mL) was added to the reaction solution, the mixture was left to stand for 10 min, and then the supernatant was removed. The residue was directly concentrated under reduced pressure using an oil pump to remove excess solvent, and the crude product was directly purified by preparative TLC (DCM:MeOH = 8:1) to give the target compound **DL-5-1** (150 mg).

LCMS (ESI) [M+H]⁺ = 1119.5.

¹H NMR (400 MHz, DMSO*-d₆*) δ 10.97 (s, 1H), 8.85 (t, *J* = 8.3 Hz, 1H), 8.67-8.61 (m, 1H), 8.38-8.21 (m, 2H), 8.19-8.10 (m, 1H), 8.07-7.98 (m, 1H), 7.89 (d, *J =* 7.5 Hz, 2H), 7.84 (d, *J =* 7.5 Hz, 1H), 7.75-7.70 (m, 1H), 7.70-7.64 (m, 2H), 7.51 (s, 1H), 7.44 (d, *J* = 7.9 Hz, 1H), 7.42-7.40 (m, 1H), 7.36 (d, *J =* 7.5 Hz, 1H), 7.33-7.29 (m, 1H), 7.20-7.16 (m, 2H), 6.92-6.83 (m, 1H), 5.10 (dd, *J =* 13.6, 5.2 Hz, 1H), 4.61-4.56 (m, 2H), 4.41 (d, *J =* 5.8 Hz, 2H), 4.35-4.28 (m, 2H), 4.24-4.20 (m, 1H), 4.10 (s, 2H), 3.92-3.84 (m, 1H), 3.75-3.68 (m, 3H), 3.63-3.59 (m, 2H), 2.94-2.90 (m, 1H), 2.86 (s, 2H), 2.79 (s, 1H), 2.59-2.54 (m, 2H), 2.41-2.37 (m, 2H), 2.29-2.26 (m, 6H), 2.02-1.97 (m, 2H), 1.71-1.66 (m, 2H), 1.45-1.41 (m, 2H), 1.31-1.28 (m, 2H), 0.91-0.78 (m, 12H).

### Step 2:

Compound **DL-5-1** (100 mg, 0.09 mmol) was dissolved in DMF (2 mL), and diethylamine (26 mg, 0.36 mmol) was added to the reaction solution. The reaction solution was stirred at room temperature for 1 h. The reaction was monitored by LCMS. The reaction solution was directly placed under an oil pump and concentrated under reduced pressure to give the target compound **DL-5-2** (80 mg), which was directly used in the next step.

LCMS (ESI) [M+H]⁺ = 897.5;

### Step 3:

Compound **DL-5-2** (80 mg, 0.09 mmol) and compound **INT7** (23 mg, 0.09 mmol) were dissolved in DMF (3 mL), and N,N-diisopropylethylamine (21 mg, 0.22 mmol) and 2-(7-azabenzotriazol-1-yl)-N,N,N',N'-tetramethyluronium hexafluorophosphate (25 mg, 0.09 mmol) were added. The reaction solution was stirred at room temperature for 1 h. The reaction was monitored by LCMS. The reaction solution was directly purified by preparative high-performance liquid chromatography (acetonitrile:H₂O containing 0.05% FA = 5%-50%) to give the formate of the target compound **DL-5** (13 mg).

LCMS (ESI) [M+H]⁺ = 1218.8.

¹H NMR (400 MHz, DMSO*-d₆*) δ 10.98 (s, 1H), 9.47 (s, 2H), 8.93 (s, 1H), 8.85 (d, *J =* 7.0 Hz, 1H), 8.64 (t, *J =* 6.4 Hz, 1H), 8.27 (t, *J =* 5.3 Hz, 1H), 8.20 (s, 1H), 8.06 (d, *J =* 7.0 Hz, 1H), 7.69 (d, *J* = 7.8 Hz, 1H), 7.65 (s, 1H), 7.51 (s, 1H), 7.43 (t, *J* = 6.7 Hz, 2H), 7.23-7.13 (m, 2H), 6.93-6.84 (m, 1H), 5.10 (dd, *J =* 13.2, 5.0 Hz, 1H), 4.62-4.52 (m, 2H), 4.44-4.39 (m, 4H), 4.31 (d, *J = 17.5* Hz, 1H), 4.28-4.22 (m, 1H), 4.14 (t, *J =* 7.9 Hz, 1H), 4.09 (s, 2H), 3.74-3.69 (m, 2H), 3.44 (s, 3H), 2.96-2.89 (m, 1H), 2.82 (d, *J =* 29.0 Hz, 3H), 2.64-2.53 (m, 4H), 2.43-2.36 (m, 2H), 2.35 (s, 6H), 2.21-2.10 (m, 2H), 2.05-1.96 (m, 2H), 1.80-1.72 (m, 1H), 1.71-1.63 (m, 2H), 1.62-1.38 (m, 4H), 1.30-1.23 (m, 4H), 1.22 (d, *J =* 3.2 Hz, 6H), 0.87 (t, *J =* 7.1 Hz, 6H).

### Example 4. Preparation and Identification of Antibodies

### 4.1.1 Preparation of B7H3 antigens and anti-B7H3 antibodies

A nucleic acid sequence for amino acids at positions 29-245 (NCBI protein number NP_001316557.1) of human 2Ig B7H3 was selected, in which a 6×His detection tag was added at the C-terminus, and the sequence was named human B7H3-2IgG-his; a nucleic acid sequence for amino acids at positions 27-461 (Uniprot protein number Q5ZPR3-1) of human 4Ig B7H3 was selected, in which a 6×His purification tag was added at the C-terminus, and the sequence was named human B7H3-4IgG-his; a nucleic acid sequence for amino acids at positions 27-465 (Uniprot protein number F7G5V3) of monkey B7H3 4Ig was selected, in which a 6×His purification tag was added at the C-terminus, and the sequence was named monkey B7H3-4IgG-his; B7H3-C1 was referenced from antibody M30-H1-L4 in CN 103687945B; B7H3-C2 was referenced from antibody mAb-C-DUBA in CN109069633A.

Genes of the three B7H3 antigens described above were synthesized, and genes of antibodies B7H3-C1 and B7H3-C2 were synthesized by codon optimization (Sangon Biotech (Shanghai) Co., Ltd.). The genes were constructed into PTT5 expression vectors, and a large amount of plasmids were extracted. The extracted and prepared expression vectors were transiently transfected into and expressed in HEK293F for 7 days, and the expression supernatants were prepared by Protein A to obtain the antigen proteins, B7H3-C1 antibody and B7H3-C2 antibody. The amino acid sequence of the constant region of IgG1 (SEQ ID NO: 9) was separately added to the VH amino acid sequences SEQ ID NO: 1 and SEQ ID NO: 5 by codon optimization and gene synthesis (Sangon Biotech (Shanghai) Co., Ltd.), and then the sequences were constructed into PTT5 vectors and named PTT5-01-CH and PPT5-02-CH, respectively; the Kappa constant region sequence (SEQ ID NO: 10) was added to the VL amino acid sequences SEQ ID NO: 2 and SEQ ID NO: 6 by codon optimization and gene synthesis (Sangon Biotech (Shanghai) Co., Ltd.), and then the sequences were constructed into PTT5 vectors and named PTT5-01-CL and PTT5-02-CL, respectively; antibody B7H3 antibody expression plasmids PTT5-01-CH/PTT5-01-CL and PTT5-02-CH/PTT5-02-CL were co-transfected into HEK293F cells by PEI max reagent and expressed in a shaker at 37 °C with 5% CO₂ for 7 days. The supernatants were collected and purified by ProA magnetic beads to obtain anti-B7H3 antibodies named 1D1-01 (the heavy chain sequence thereof is set forth in SEQ ID NO: 3, and the light chain sequence is set forth in SEQ ID NO: 4) and 2E3-02 (the heavy chain sequence thereof is set forth in SEQ ID NO: 7, and the light chain sequence is set forth in SEQ ID NO: 8), respectively.

### 4.1.2 Protein affinity assay of anti-B7H3 antibodies

The affinity of the B7H3 fully human antibodies for human B7H3-4IgG-his and monkey B7H3-4IgG-his proteins was assayed by an ELISA method. The specific experimental procedures are as follows: the human B7H3-4IgG-his or monkey B7H3-4IgG-his protein was diluted with a carbonate buffer with a pH value of 9.6 to a final concentration of 1 µg/mL and added to 96-well enzyme-labeled wells at 100 µL/well for coating at 4 °C overnight; the coating solution was discarded, the plate was washed once with PBST, 100 µL of PBS (containing 2% BSA) was added to each well, and the plate was blocked at 37 °C for 1 h; then 100 µL of the B7H3 fully human antibody diluted with PBS (containing 2% BSA) (starting at 10 µg, 3-fold dilution in duplicate) was added to each well, and the plate was incubated at 37 °C for 2 h; the plate was washed 3 times with PBST, a horseradish peroxidase-labeled anti-human Fc antibody (diluted with PBS at 1:10000) was added at 100 µL/well, and the system was reacted at 37 °C for 1 h; the plate was washed 5 times with PBST. TMB color development solution was added for color development at 100 µL/well at 37 °C for 7 min, and a stop solution was added to stop the reaction at 50 µL/well. The absorbance was measured at 450 nm. EC50 values were calculated from the original data.

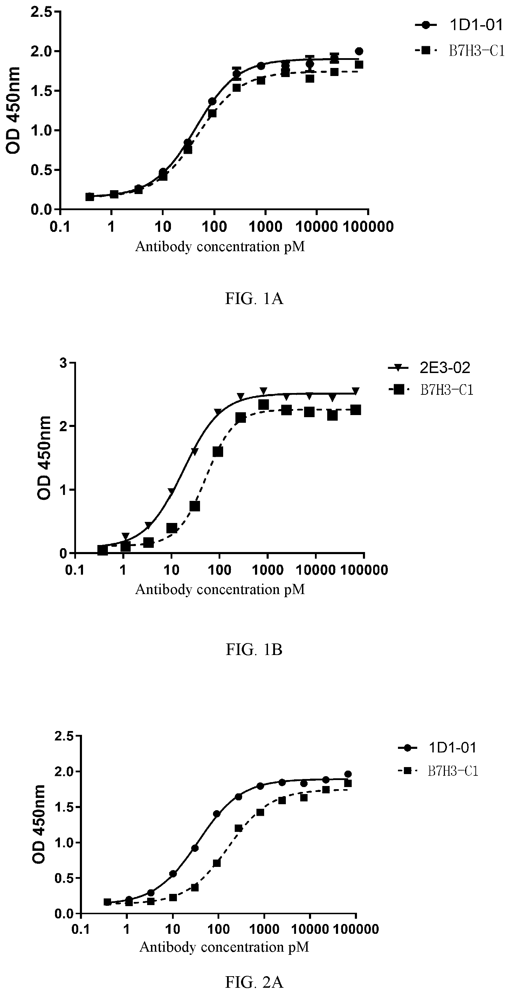

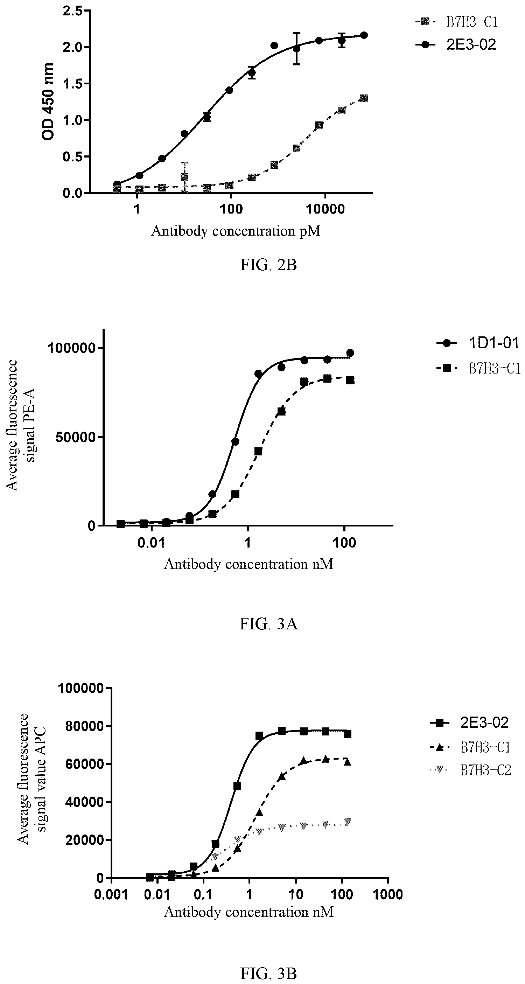

The results are shown in FIGs. 1A, 1B, 2A and 2B, and detailed results are shown in Tables 1-1 and 1-2. 1D1-01 and 2E3-02 both had relatively high affinity for human B7H3-4IgG, which were 43.56 pM and 17.54 pM, respectively, wherein the affinity of 2E3-02 was higher than that of B7H3-C1, i.e., 51.85 pM; 1D1-01 and 2E3-02 had relatively high binding capacity to monkey B7H3-4IgG, which were 35.82 pM and 27.72 pM, respectively.

**Table 1-1. Protein affinity assay results of 1D1-01 antibody**

| Antibody | Human B7H3-4IgG-His | Monkey B7H3-4IgG-His |
|---|---|---|
| | EC50 (pM) | EC50 (pM) |
| 1D1-01 | 43.56 | 35.82 |
| B7H3-C1 | 48.03 | 166.52 |

**Table 1-2. Protein affinity assay results of 2E3-02 antibody**

| Antibody | Human B7H3-4IgG-His | Monkey B7H3-4IgG-His |
|---|---|---|
| | EC50 (pM) | EC50 (pM) |
| 2E3-02 | 17.54 | 27.72 |
| B7H3-C1 | 51.85 | 3921 |

### 4.1.3 Cell affinity assay of anti-B7H3 antibodies

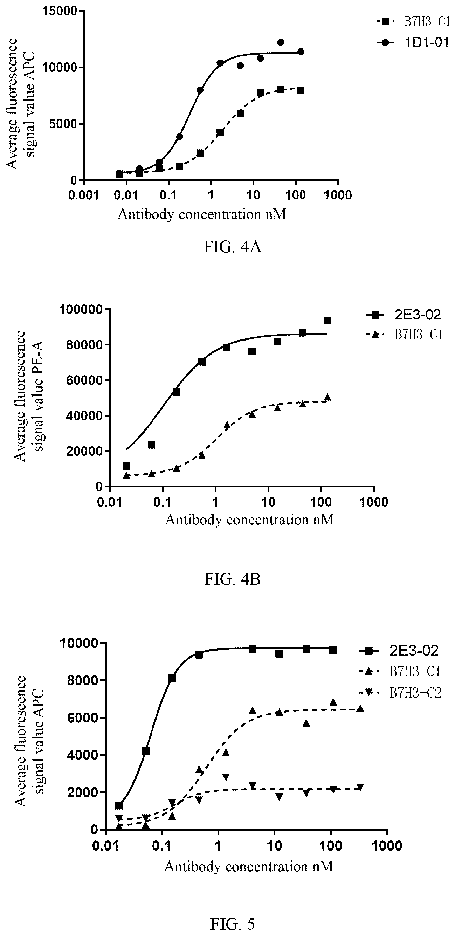

B7H3 proteins are highly expressed in various solid tumor cells. The affinity of the B7H3 fully human antibody for tumor cells was assayed by a FACS method. The specific experimental procedures are as follows: MCF-7 breast cancer cells (from Nanjing Cobioer, Catalog No. CBP60380), A549 human non-small cell lung cancer cells (from ATCC, Catalog No. CRM-CCL-185) and PC3 human prostate cancer cells (from ATCC, Catalog No. CRL-1435) in the log phase were digested with trypsin and resuspended to 5 × 10⁵ cells/mL in a PBS buffer containing 2% BSA, and 100 µL of the cell suspension was added to a 96-well plate; B7H3-C1, B7H3-C2, 1D1-01 and 2E3-02 were subjected to 3-fold dilution with a PBS buffer containing 2% BSA from a starting concentration of 40 µg/mL, and then 100 µL of the antibody suspension was added to the 96-well plate and mixed well; the plate was incubated at 4 °C for 1 h, washed 2 times with pre-cooled PBS at 300 µL/well for each time, and centrifuged at 500 g for 5 min; 1:50 fluorescent secondary antibody APC anti-human IgG or PE anti-human IgG (purchased from Biolegend) was added at 100 µL/well and mixed well; the plate was incubated at 4 °C for 0.5 h, washed 2 times with pre-cooled PBS at 300 µL/well for each time, centrifuged at 500 g for 5 min, and rescreened with 500 µL of PBS. The average fluorescence signal values were measured using a Beckman flow cytometer. The EC50 values were calculated from the average fluorescence signal values. The results are shown in FIGs. 3A, 3B, 4A and 4B as well as FIG. 5, and the specific data are shown in Tables 2-1 and 2-2.

**Table 2-1. Cell affinity assay results of 1D1-01 antibody**

| Antibody | MCF-7 tumor cell | | A549 tumor cell | |
|---|---|---|---|---|
| | EC50 (nM) | Maximum MFI | EC50 (nM) | Maximum MFI |
| 1D1-01 | 0.524 | 94465 | 0.322 | 11288 |
| B7H3-C1 | 1.709 | 83992 | 1.865 | 8273 |

**Table 2-2. Cell affinity assay results of 2E3-02 antibody**

| Antibody | MCF-7 tumor cell | | A549 tumor cell | | PC-3 tumor cell | |
|---|---|---|---|---|---|---|
| | EC50 (nM) | Maximum MFI | EC50 (nM) | Maximum MFI | EC50 (nM) | Maximum MFI |
| 2E3-02 | 0.398 | 77732 | 0.150 | 84806 | 0.063 | 9729 |
| B7H3-C1 | 1.360 | 63194 | 1.054 | 47956 | 0.618 | 6436 |
| B7H3-C2 | 0.278 | 27925 | Not detected | Not detected | 0.167 | 2170 |

As shown in Tables 2-1 and 2-2, in the MCF-7 tumor cells highly expressing B7H3, the affinity of 1D1-01 and 2E3-02 were 0.524 nM and 0.398 nM, respectively, both of which were much higher than that of B7H3-C1, and the maximum fluorescence signal values were 12% and 23% higher than that of B7H3-C1, respectively, wherein the maximum fluorescence signal value of 2E3-02 was 2.78 times higher than that of control antibody 2. In the A549 tumor cells moderately expressing B7H3, the affinity of 1D1-01 and 2E3-02 were 0.322 nM and 0.150 nM, respectively, both of which were much higher than that of B7H3-C1, and the maximum fluorescence signal values were 36% and 77% higher than that of B7H3-C1, respectively. In the PC-3 tumor cells lowly expressing B7H3, the affinity of 2E3-02 was 0.06 nM, which was much higher than that of B7H3-C1 of 0.618 nM and also higher than that of B7H3-C1 of 0.167 nM, and the maximum fluorescence signal value was 1.51 times and 4.48 times higher than those of B7H3-C1 and B7H3-C2, respectively. In conclusion, the anti-B7H3 antibodies 1D1-01 and 2E3-02 have better tumor cell affinity than those of B7H3-C1 and B7H3-C1.

### 4.1.4 Endocytic activity assay of anti-B7H3 antibodies

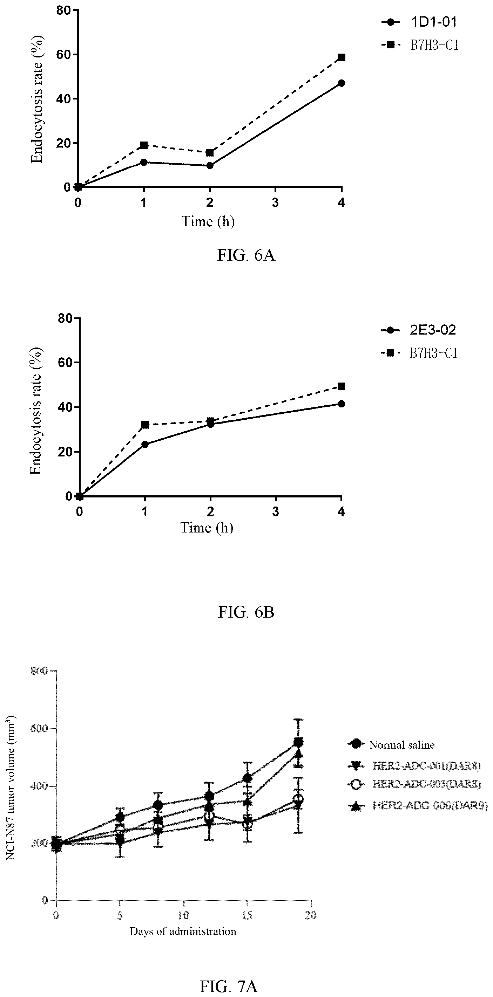

The endocytosis of the anti-B7H3 antibodies by A549 human non-small cell lung cancer cells was assayed by a FACS method. A549 cells in the log phase were digested with trypsin, and the cells were collected by centrifugation and washed three times with pre-cooled PBS; the cells were resuspended in PBS (containing 1% BSA), the anti-B7H3 antibody to be tested at a concentration of 10 µg/mL was added, and the cells were incubated at 4 °C for 1 h; the cells were collected by centrifugation, washed three times with PBS, and resuspended in DMEM + 10% FBS, and the resuspended cells were divided into 4 parts and incubated at 37 °C for 0 h, 1 h, 2 h and 4 h, respectively; after the incubation phase was completed, the cells were collected by centrifugation, washed three times with pre-cooled PBS, and resuspended in 50 µL of 1% BSA (in PBS), 1 µL of fluorescent secondary antibody APC anti-human IgG (Biolegend) was added to each well and mixed well, and the cells were incubated at 4 °C for 0.5 h; the cells were collected by centrifugation, washed three times with pre-cooled PBS, resuspended in 200 µL of PBS, and tested on the machine. According to the formula: endocytosis rate (%) = [1 - (average fluorescence value of test sample at this time point - average fluorescence value of negative control sample at this time point)/(average fluorescence value of test sample at 0 h - average fluorescence value of negative control sample at 0 h)] × 100. The results of 1D1-01 and 2E3-02 are shown in FIGs. 6A and 6B, respectively. The anti-B7H3 antibodies 1D1-01 and 2E3-02 had relatively strong endocytic activity in A549 tumor cells, reaching about 40% at 4 h, which was comparable to that of the antibody B7H3-C1.

### Example 5. Synthesis of Antibody-Drug Conjugates (ADCs)

### Example 5.1 Preparation of B7H3-ADC

### Example 5.1.1 Preparation of B7H3-ADC-001

25 mL of 2E3-02 antibody (anti-B7H3, at a concentration of 28.5 mg/mL, 20 mM acetic acid buffer) was taken, to which 25 mL of 20 mM acetic acid buffer was added for dilution, and then 1 mL of an aqueous solution containing 0.25 M EDTA was added and mixed well. The pH of the sample was adjusted to 7.6 with 0.5 M aqueous disodium hydrogen phosphate solution, and then 4.5-fold equivalents of 20 mM TCEP (tris(2-carboxyethyl)phosphine hydrochloride) relative to the antibody was added and mixed well. The mixture was reacted at room temperature for 90 min. Finally, 10-fold equivalents (relative to the antibody) of compound DL-1 dissolved in DMSO was added and mixed well. The mixture was reacted at room temperature for another 2 h. After the reaction was completed, the sample was exchanged into 10 mM histidine buffer with a pH of 5.5 using a 30 KDa ultrafiltration tube to remove low molecular weight substances, and finally, the sample was concentrated to obtain a solution of the ADC containing the anti-B7H3 antibody B7H3-ADC-001, whose DAR value was determined to be 8.0 by mass spectrometry.

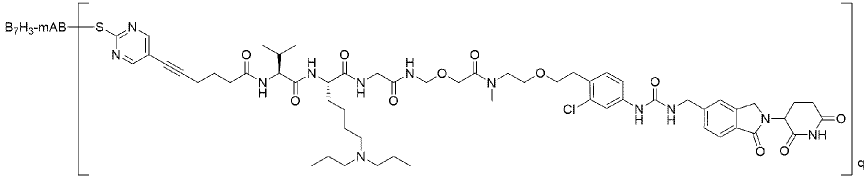

### Example 5.2 Preparation of HER2-ADC

### Example 5.2.1: Preparation ofHER2-ADC-001

0.8 mL of trastuzumab (20.4 mg/mL) was taken and diluted with 0.008 mL of 20 mM PB + 100 mM edetate disodium solution (pH 7.6), the pH was adjusted to 7.81 with 0.5 M Na₂HPO₄ solution, and 20 mM TCEP (tris(2-carboxyethyl)phosphine, 0.0303 mL, 0.606 µmol) solution was added and mixed well. The mixture was left to stand at room temperature for 90 min. A solution of DL-2 (1.654 mg) in dimethyl sulfoxide (0.1323 mL) was added to the solution described above, and the resulting solution was mixed well and left to stand at room temperature for 2 h. After that, buffer exchange was performed using an ultrafiltration centrifuge tube (Merck, Amicon Ultra-15). The coupling product HER2-ADC-001 of DL-2 and trastuzumab was obtained. The DAR value was determined to be 8.0 by mass spectrometry.

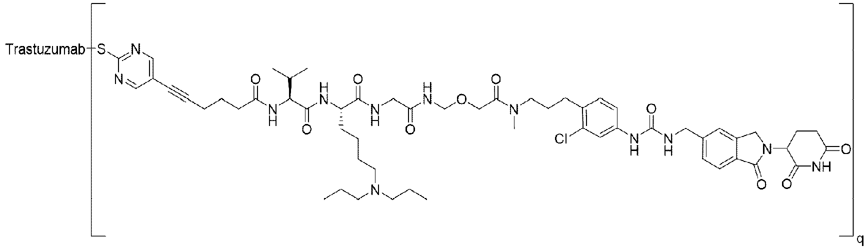

| HER2-ADC-001 | | | | | |
|---|---|---|---|---|---|
| Peptide chain | Theoretical value | Measured value | Area | Ratio | DAR |
| LC | 23438 | Not detected | 0 | 0 | 8.0 |
| LC+DAR1 | 24560.5 | 24562.27 | 140814.18 | 1.0 | |
| HC | 50594 | Not detected | 0 | 0 | |
| HC+DAR1 | 51716.5 | Not detected | 0 | 0 | |
| HC+DAR2 | 52839 | Not detected | 0 | 0 | |
| HC+DAR3 | 53961.5 | 53964.59 | 78645.95 | 1.0 | |

In the table, LC represents an antibody light chain; HC represents an antibody heavy chain; DAR1 represents a conjugate comprising a light chain or a heavy chain conjugated to 1 drug linker conjugate; DAR2 represents a conjugate comprising a light chain or a heavy chain conjugated to 2 drug linker conjugates; DAR3 represents a conjugate comprising a light chain or a heavy chain conjugated to 3 drug linker conjugates. In the following text, LC, HC, DAR1, DAR2 and DAR3 are as described above.

The light chain of trastuzumab was determined to be conjugated to 1 toxin molecule (LC + DAR1 ratio was 100%), and the heavy chain was determined to be conjugated to 3 toxin molecules (HC + DAR3 ratio was 100%), and thus the drug-to-antibody ratio (DAR value) of HER2-ADC-001 (DAR8) was calculated to be 8.0.

### Determination method for drug-to-antibody ratio (DAR value):

50 µg of an ADC sample was taken and diluted to 0.5 mg/mL with ultrapure water, and then 1 µL of 1 M DTT was added. The resulting solution was mixed well and then centrifuged, and the supernatant was collected for sample injection.

### Liquid phase parameters:

| Chromatographic column | Xbridge Protein BEH SEC (2.5 µm, 4.6 × 150 mm) | |
|---|---|---|
| Flow rate | 0.3 mL/min | |
| Column temperature | 30°C | |
| Injection volume | 1 µl | |
| Sample chamber temperature | 8°C | |
| Elution method | Isocratic elution | |

| Mobile phase | Mobile phase A: 0.1%FA in H₂O | |
|---|---|---|
| | Mobile phase B: 0.1% FA in ACN | |
| Time/min | 0 | 10 |
| A% | 65 | 65 |
| B% | 35 | 35 |

### Mass spectrometry parameters:

| Mass spectrometry parameters | | |
|---|---|---|
| Convention | Run time: | 10 min |
| | Total scanning time: | 1.028 s |
| | Estimated number of cycles: | 584 |
| | Complete protein model: | Open |
| | Large protein (> 70 kDa): | No |
| | Decrease detector voltage: | Yes |
| | Scanning type: | Primary parent ion full scan |
| Source gas parameters | Source gas 1: | 45 psi |
| | Source gas 2: | 45 psi |
| | Air curtain gas: | 30 psi |
| | Collision gas: | 7 |
| | Temperature of heated gas (°C): | 450 |
| | Ionization polarity: | Positive |
| | Lower limit of mass number scanning range (Da): | 600 |
| | Upper limit of mass number scanning range (Da): | 4000 |
| | Cumulative time: | 1 s |
| Primary mass spectrum | Electrospray voltage: | 5000 V |
| | Declustering voltage (V): | 120 V |
| | Declustering voltage variation step | 0 V |
| | Collision energy: | 12 V |
| | Collision energy variation step: | 0 V |
| | Total number of ions counted per unit time: | 80 |

### Example 5.2.2: Preparation of HER2-ADC-002

The coupling product HER2-ADC-002 of DL-3 and trastuzumab was obtained by replacing DL-2 with DL-3 using the same method as that in Example 5.2.1. The DAR value was determined to be 8.0 by mass spectrometry.

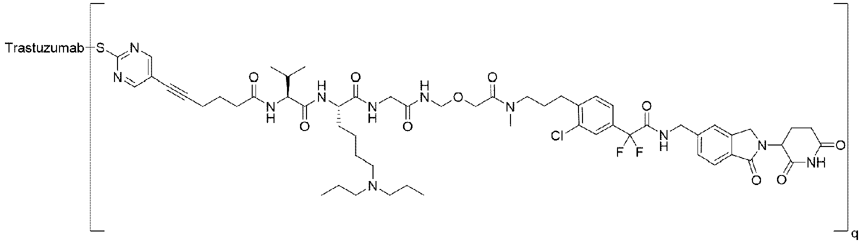

| HER2-ADC-002 | | | | | |
|---|---|---|---|---|---|
| Peptide chain | Theoretical value | Measured value | Area | Ratio | DAR |
| LC | 23438 | Not detected | 0 | 0 | 8.0 |
| LC+DAR1 | 24595.5 | 24597.12 | 157318.64 | 1 | |
| HC | 50594 | Not detected | 0 | 0 | |
| HC+DAR1 | 51751.5 | Not detected | 0 | 0 | |
| HC+DAR2 | 52909 | Not detected | 0 | 0 | |
| HC+DAR3 | 54066 | 54069.53 | 104445.93 | 1 | |

### Example 5.2.3: Preparation of HER2-ADC-003

The coupling product HER2-ADC-003 of DL-1 and trastuzumab was obtained by replacing DL-2 with DL-1 using the same method as that in Example 5.2.1. The DAR value was determined to be 8.0 by mass spectrometry.

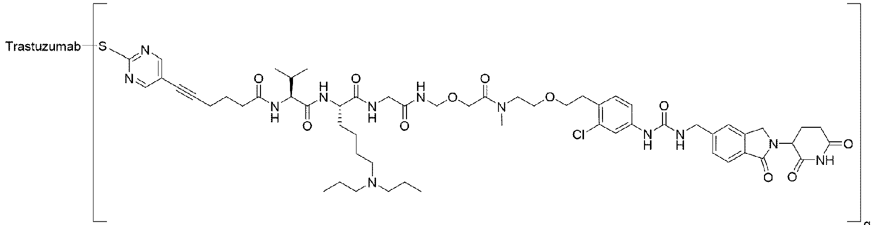

| HER2-ADC-003 | | | | | |
|---|---|---|---|---|---|
| Peptide chain | Theoretical value | Measured value | Area | Ratio | DAR |
| LC | 23438.0 | Not detected | 0 | 0 | 8.0 |
| LC+DAR1 | 24590.6 | 24592.4 | 332639.5 | 1.0 | |
| HC | 50594.0 | Not detected | 0 | 0 | |
| HC+DAR1 | 51746.6 | Not detected | 0 | 0 | |
| HC+DAR2 | 52899.2 | Not detected | 0 | 0 | |
| HC+DAR3 | 54051.8 | 54055.5 | 317389.1 | 1.0 | |

### Example 5.2.4: Preparation of HER2-ADC-004

The coupling product HER2-ADC-004 of DL-5 and trastuzumab was obtained by replacing DL-2 with DL-5 using the same method as that in Example 5.2.1. The DAR value was determined to be 8.0 by mass spectrometry.

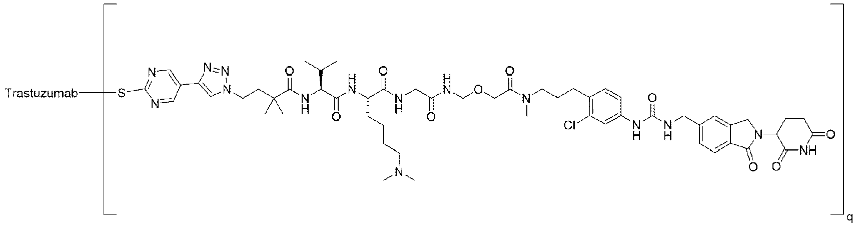

| HER2-ADC-004 | | | | | |
|---|---|---|---|---|---|
| Peptide chain | Theoretical value | Measured value | Area | Ratio | DAR |
| LC | 23438.0 | Not detected | 0 | 0 | 8.0 |
| LC+DAR1 | 24575.5 | 24577.5 | 271205.4 | 1.0 | |
| HC | 50594.0 | Not detected | 0 | 0 | |
| HC+DAR1 | 51731.5 | Not detected | 0 | 0 | |
| HC+DAR2 | 52869.0 | Not detected | 0 | 0 | |
| HC+DAR3 | 54006.5 | 54010.3 | 237615.5 | 1.0 | |

### Example 5.2.5: Preparation of HER2-ADC-005

The coupling product HER2-ADC-005 of DL-4 and trastuzumab was obtained by replacing DL-2 with DL-4 using the same method as that in Example 5.2.1. The DAR value was determined to be 7.0 by mass spectrometry.

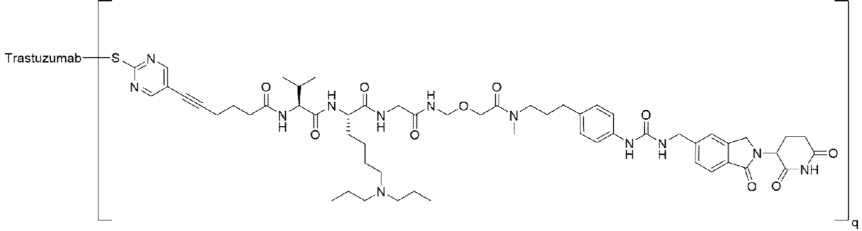

| HER2-ADC-005 | | | | | |
|---|---|---|---|---|---|
| Peptide chain | Theoretical value | Measured value | Area | Ratio | DAR |
| LC | 23438.0 | 23438.8 | 100111.6 | 0.4 | 7.0 |
| LC+DAR1 | 24526.6 | 24528.1 | 139246.6 | 0.6 | |
| HC | 50594.0 | Not detected | 0 | 0 | |
| HC+DAR1 | 51682.6 | Not detected | 0 | 0 | |
| HC+DAR2 | 52771.2 | 52772.0 | 13386.1 | 0.1 | |
| HC+DAR3 | 53859.8 | 53862.0 | 112460.9 | 0.9 | |

### Example 5.2.6: Preparation of HER2-ADC-006

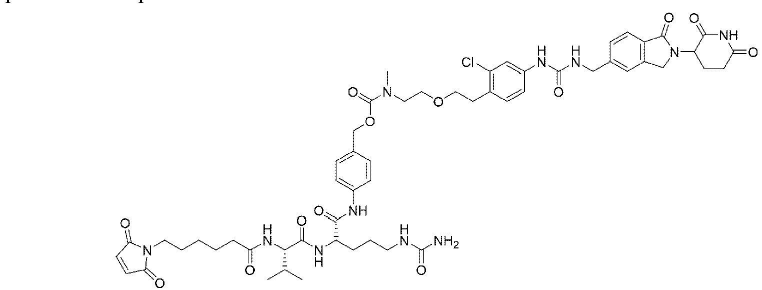

DL-6 (the preparation method was referenced from the patent document WO2021198965) The coupling product HER2-ADC-006 of DL-6 and trastuzumab was obtained by replacing DL-2 with DL-6 using the same method as that in Example 5.2.1. The DAR value was determined to be 9.0 by mass spectrometry.

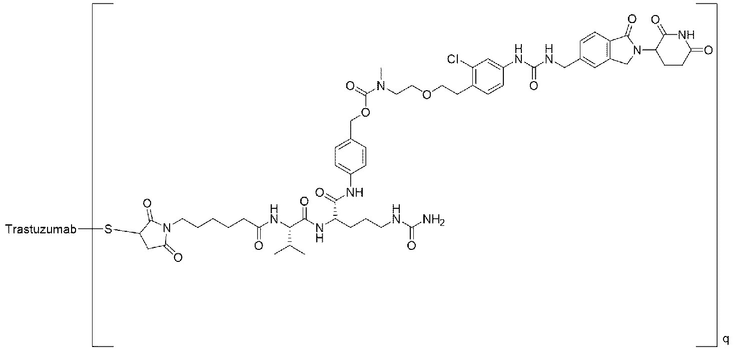

| HER2-ADC-006 | | | | | |
|---|---|---|---|---|---|
| Peptide chain | Theoretical value | Measured value | Area | Ratio | DAR |
| LC | 23438 | Not detected | 0 | 0 | |
| LC+DAR1 | 24564.5 | 24565.07 | 97436.65964 | 0.806977 | |
| LC+DAR2 | 25691 | 25691.91 | 21680.25728 | 0.179557 | |
| LC+DAR3 | 26817.5 | 26818.21 | 1625.896347 | 0.013466 | |
| HC | 50594 | Not detected | 0 | 0 | |
| HC+DAR1 | 51720.5 | Not detected | 0 | 0 | 9.0 |
| HC+DAR2 | 52847 | Not detected | 0 | 0 | |
| HC+DAR3 | 53973.5 | 53972.94 | 30279.84267 | 0.708974 | |
| HC+DAR4 | 55100 | 55099.76 | 11571.98211 | 0.270947 | |
| HC+DAR5 | 56226.5 | 56226.36 | 857.5296917 | 0.020078 | |

### Example 6. In Vitro Cell Activity Assay for Bioactive Compounds (Payloads)

The structure of control compound 1 is shown below:

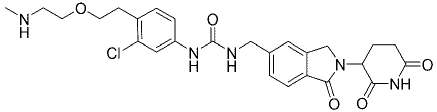

### Control compound 1 (the preparation method was referenced from the patent document WO2021198965)

### Example 6.1 Assay of inhibitory activity of bioactive compounds (payloads) on proliferation of N87, MCF7 and NUGC4 cells

### I. Assay method:

N87 (1640 + 10% FBS), MCF7 (DMEM + 10% FBS + 10 µg/mL insulin) and NUGC4 (1640 + 10% FBS) cells were thawed, cultured, and seeded into a 96-well flat-bottom plate at a density of 5000 cells/well and 100 µL/well. The cells were incubated in an incubator at 37 °C overnight. The payload to be tested was dissolved in DMSO, and the resulting solution was aliquoted and cryopreserved at -80 °C. The next day, the payload to be tested was subjected to 4-fold dilution to a maximum concentration of 10000 nM for 10 points using the respective complete medium. 100 µL of the diluted drug was added to the cell culture wells (2 replicates per well). After the cells were cultured at 37 °C for 4 days, 50 µL of Cell titer glo substrate was added to each well. After the cells were incubated for 5 min, the fluorescence values were measured. Among them, the key material sources are shown in Table 1.

**Table 1. Key material sources**

| **Name** | **Manufacturer or source** | **Catalog No.** |
|---|---|---|
| N87 cells | Procell | CL-0211 |
| MCF7 cells | Cobioer, Nanjing | CBP60380 |
| NUGC4 cells | Cobioer, Nanjing | CBP60493 |
| DMEM medium | Damas life | C8013 |
| 1640 medium | Damas life | C8016 |
| Fetal bovine serum | Cornning | 35-081-CV |
| Insulin | YEASEN | 40112ES60 |
| Cell titer glo substrate | Damas life | RA-GL11-A |

### II. Experimental conclusion:

The assay results show that the payloads in the present disclosure had significant inhibitory activity on the proliferation of N87 cells, MCF7 cells and NUGC4 cells, wherein the proliferation inhibitory activity of PL-1 and PL-4 was significantly superior to that of control compound 1. The assay results are shown in Table 2.

**Table 2. Assay results of inhibitory activity of payloads on proliferation of N87, MCF7 and NUGC4 cells**

| Payload | N87 (EC₅₀, nmol) | MCF7 (EC50, nmol) | NUGC4 (EC50, nmol) |
|---|---|---|---|
| Control compound 1 | 572.4 | 982.9 | 345 |
| PL-1 | 14.31 | 62.06 | / |
| PL-2 | 192.6 | / | / |
| PL-3 | 850.9 | / | / |
| PL-4 | 36.07 | 44.68 | 43.18 |
| PL-5 | 1591 | 228 | 501.9 |

### Example 7. In Vitro Cell Activity Assay of Antibody-Drug Conjugates (ADCs)

### Example 7.1 Assay of inhibitory activity of ADCs on proliferation of in vitro cells (NCI-H358 tumor cells)

NCI-H358 tumor cells were digested with trypsin by using a conventional method. The cells were collected in a tube, counted, resuspended in the corresponding assay medium (containing 2% FBS), and seeded into a 96-well plate at 5000 cells/well. 100 µL of the ADC diluted with 2% FBS medium (from a starting concentration of 150 µg/mL, 3-fold dilution (12 concentration gradients)) was added to the 96-well plate. After the cells were cultured at 37 °C with 5% CO₂ for 7 days, 20 µL of CCK8 reagent was added to each well, and the system was reacted for 4 h. The plate was read on a microplate reader (at a detection wavelength of 450 nm). The assay results show that the ADC molecules of the present disclosure had tumor cell-killing effects.

### Example 7.2 Assay of inhibitory activity of ADCs on proliferation of in vitro cells (N87 tumor cells)

### I. Assay method:

N87 cells were thawed and cultured using 1640 + 10% FBS, and the cells were seeded into a 96-well flat-bottom plate at a density of 5000 cells/well and 100 µL/well and incubated in an incubator at 37 °C overnight. The next day, the ADC molecule to be tested was diluted with 1640 + 10% FBS. The maximum concentration of ADC molecule-002 to be tested was 200 nM, and the maximum concentration of the other 4 samples was 2000 nM. 4-fold dilution was performed for 10 points. 100 µL of the diluted drug was added to the cell culture wells. After the cells were cultured at 37 °C for 4 days, 50 µL of Cell titer glo substrate was added to each well. After the cells were incubated for 5 min, the fluorescence values were measured. Among them, the key material sources are shown in Table 3.

**Table 3. Key material sources**

| **Name** | **Manufacturer or source** | **Catalog No.** |
|---|---|---|
| N87 cells | Procell | CL-0211 |
| 1640 medium | Damas life | C8016 |
| Fetal bovine serum | Cornning | 35-081-CV |
| Cell titer glo substrate | Damas life | RA-GL11-A |

### II. Experimental conclusion:

The assay results show that the ADC molecules of the present disclosure all had significant inhibitory activity on the proliferation of the N87 tumor cell line. The assay results are shown in Table 4.

**Table 4. Assay results of inhibitory activity of ADCs on proliferation of N87 tumor cells**

| ADC | N87 (EC₅₀, nmol) |
|---|---|
| HER2-ADC-001 | 0.029 |
| HER2-ADC-002 | 2.09 |
| HER2-ADC-003 | 0.073 |
| HER2-ADC-004 | 0.062 |
| HER2-ADC-005 | 1.49 |

### Example 8. In Vivo Activity Assay of Antibody-Drug Conjugates (ADCs)

### Example 8.1 Efficacy assay of antibody-drug conjugates (ADCs) on NCI-N87 xenografts (administration dose of 1 mg/kg)

### 1. Materials

Test compounds: HER2-ADC-001, HER2-ADC-003, HER2-ADC-006, and normal saline as a negative control.

Experimental cells: NCI-N87 cells, purchased from Nanjing Cobioer Biosciences Co., Ltd.

Experimental animals: Balb/c nu nude mice, female, 5-6 weeks old, purchased from Vital River Laboratory Animal Technology Co., Ltd.

### 2. Experimental schemes

### 2.1. Cell treatment

NCI-N87 cells were cultured in a 1640 medium containing 10% FBS in a culture dish with a diameter of 15 cm and digested with trypsin-EDTA when the confluence reached about 80%-90%. The cells were washed twice with PBS, centrifuged, resuspended in pre-cooled PBS and counted on a cell counter. The cells were diluted with PBS to a cell concentration of 5 × 10⁷ cells/mL.

### 2.2. Tumor cell transplantation

Balb/c nu mice were acclimated to the laboratory environment for 2-5 days, and subcutaneously inoculated with NCI-N87 cells in the right flank in an inoculation amount of 5 × 10⁶ cells/mouse with an inoculation volume of 0.2 mL (containing 50% Matrigel). When the tumor grew to about 200 mm³, the experiment was conducted.

### 2.3. Animal administration and detection

The enrolled tumor-bearing nude mice were administered according to the following schemes:

**Table 5**

| No. | Group | Number of animals | Dose of administration | Route and period of administration |
|---|---|---|---|---|
| 1 | Normal saline | 5 | / | i.v., QW×3 |
| 2 | HER2-ADC-001 | 5 | 1 mg/kg | i.v., QW×3 |
| 3 | HER2-ADC-003 | 5 | 1 mg/kg | i.v., QW×3 |
| 4 | HER2-ADC-006 | 5 | 1 mg/kg | i.v., QW×3 |

### 2.4. Measurement of tumor volume and body weight:

The tumor volume and body weight were measured 2 times per week and T/C% and TGI (%) were calculated as follows: relative tumor proliferation rate T/C (%) = TRTV / CRTV × 100%

(TRTV: mean RTV of treatment group; CRTV: mean RTV of control group; RTV = Vt / V0, wherein V0 is the tumor volume of the animal at the time of grouping, and Vt is the tumor volume of the animal after treatment); relative tumor inhibition rate TGI% = (1 - T/C) × 100% (T and C are the relative tumor volume (RTV) of the treatment group and the control group, respectively, at a particular time point).

Tumor volume (V) was calculated as follows: V = 1/2 × L long × L short², wherein L long and L short represent the long and short diameters of the tumor, respectively.

### 3. Experimental results

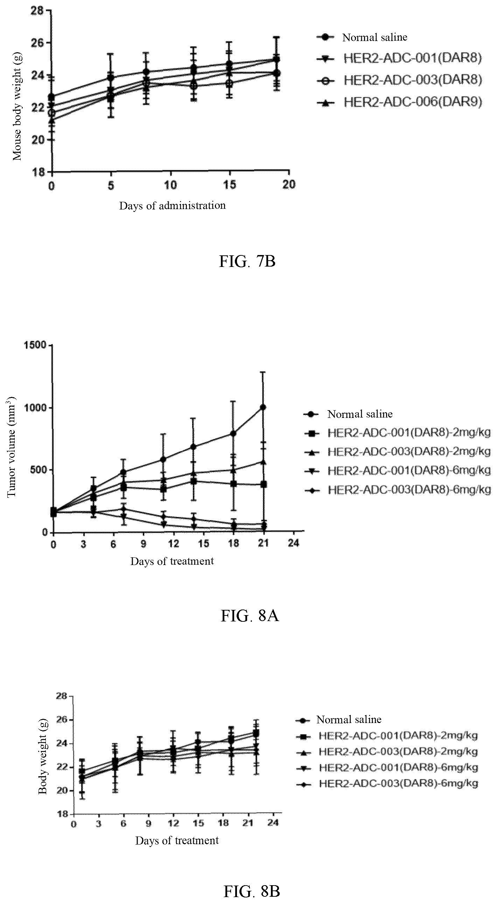

The assay results show that the ADCs of the present disclosure all showed significant anti-tumor effects, wherein the tumor inhibitory effects of HER2-ADC-001 and HER2-ADC-003 were significantly superior to that of the control compound HER2-ADC-006. During the administration, there was no significant weight loss and significant drug toxicity in each group of animals. The specific results are shown in Table 6 and FIGs. 7A and 7B.

**Table 6. NCI-N87 xenograft model data**

| | Tumor volume (mm³) | | | | | | T/C (%) | TGI (%) |
|---|---|---|---|---|---|---|---|---|
| Sample | Day0 | Day5 | Day8 | Day12 | Day15 | Day19 | | |
| Normal saline | 195.6 | 292 | 334 | 364.8 | 427.8 | 552.2 | / | / |
| HER2-ADC-001 | 197 | 198.6 | 236.6 | 266.8 | 273.8 | 332.4 | 59.8 | 40.2 |
| HER2-ADC-003 | 196.6 | 246.4 | 255.2 | 297.4 | 267.2 | 354.2 | 63.8 | 36.2 |
| HER2-ADC-006 | 196.0 | 231.8 | 288.4 | 336.0 | 349.2 | 516.4 | 93.3 | 6.7 |

### Example 8.2 Efficacy assay of antibody-drug conjugates (ADCs) on NCI-N87 xenografts (administration doses of 2 mg/kg and 6 mg/kg)

### 1. Materials

Test compounds: HER2-ADC-001, HER2-ADC-003, and normal saline as a negative control. Experimental cells: NCI-N87 cells, purchased from Nanjing Cobioer Biosciences Co., Ltd. Experimental animals: Balb/c nu nude mice, female, 5-6 weeks old, purchased from Vital River Laboratory Animal Technology Co., Ltd.

### 2. Experimental schemes

### 2.1. Cell treatment

NCI-N87 cells were cultured in a 1640 medium containing 10% FBS in a culture dish with a diameter of 15 cm and digested with trypsin-EDTA when the confluence reached about 80%-90%. The cells were washed twice with PBS, centrifuged, resuspended in pre-cooled PBS and counted on a cell counter. The cells were diluted with PBS to a cell concentration of 5 × 10⁷ cells/mL.

### 2.2. Tumor cell transplantation

Balb/c nu mice were acclimated to the laboratory environment for 2-5 days, and subcutaneously inoculated with NCI-N87 cells in the right flank in an inoculation amount of 5 × 10⁶ cells/mouse with an inoculation volume of 0.2 mL (containing 50% Matrigel). When the tumor grew to about 165 mm³, the experiment was conducted.

### 2.3. Animal administration and detection

The enrolled tumor-bearing nude mice were administered according to the following schemes:

**Table 7**

| No. | Group | Number of animals | Dose of administration | Route and period of administration |
|---|---|---|---|---|
| 1 | Normal saline | 5 | / | i.v., QW×3 |
| 2 | HER2-ADC-001 | 5 | 2 mg/kg | i.v., QW×3 |
| 3 | HER2-ADC-003 | 5 | 2 mg/kg | i.v., QW×3 |
| 4 | HER2-ADC-001 | 5 | 6 mg/kg | i.v., QW×3 |
| 5 | HER2-ADC-003 | 5 | 6 mg/kg | i.v., QW×3 |

### 2.4. Measurement of tumor volume and body weight

The tumor volume and body weight were measured 2 times per week and T/C% and TGI (%) were calculated as follows: relative tumor proliferation rate T/C (%) = TRTV / CRTV × 100% (TRTV: mean RTV of treatment group; CRTV: mean RTV of control group; RTV = Vt / V0, wherein V0 is the tumor volume of the animal at the time of grouping, and Vt is the tumor volume of the animal after treatment); relative tumor inhibition rate TGI% = (1 - T/C) × 100% (T and C are the relative tumor volume (RTV) of the treatment group and the control group, respectively, at a particular time point).

Tumor volume (V) was calculated as follows: V = 1/2 × L long × L short², wherein L long and L short represent the long and short diameters of the tumor, respectively.

### 3. Experimental results

The assay results show that the ADCs of the present disclosure all showed significant anti-tumor effects, wherein the tumor growth inhibition rate of HER2-ADC-001 at 2 mg/kg was 62.47%, and the tumor growth inhibition rate thereof at 6 mg/kg was 98.30%; the tumor growth inhibition rate of HER2-ADC-003 at 2 mg/kg was 44.08%, and the tumor growth inhibition rate thereof at 6 mg/kg was 94.15%. During the administration, there was no significant weight loss and significant drug toxicity in each group of animals. The specific results are shown in Table 8 and FIGs. 8A and 8B.

**Table 8. NCI-N87 xenograft model data**

| | Tumor volume (mm³) | | | | | | | T/C (%) | TGI (%) |
|---|---|---|---|---|---|---|---|---|---|
| Sample | Day0 | Day4 | Day7 | Day11 | Day14 | Day18 | Day21 | / | / |
| Normal saline | 166 | 350 | 479 | 579 | 680 | 784 | 992 | / | / |
| HER2-ADC-001-2 mg/kg | 166 | 280 | 357 | 340 | 404 | 379 | 372 | 37.53 | 62.47 |
| HER2-ADC-003-2 mg/kg | 166 | 313 | 398 | 415 | 470 | 490 | 555 | 55.92 | 44.08 |
| HER2-ADC-001-6 mg/kg | 165 | 164 | 118 | 54 | 35 | 25 | 17 | 1.70 | 98.30 |
| HER2-ADC-003-6 mg/kg | 164 | 160 | 185 | 122 | 103 | 60 | 57 | 5.85 | 94.15 |

### Example 9. ADC Plasma Stability Test

Solution preparation: each ADC prepared in Example 5 was diluted with PBS to a concentration of 2 mg/mL.

Sample incubation: a bacteriostatic agent ProClin was added to human plasma at a final concentration of 0.1% under sterile conditions. Each ADC prepared in Example 5 was added to the above sterile plasma at a final concentration of 200 µg/mL, and the mixture was incubated in a cell incubator at 37 °C and shaken at 80 rpm. The mixture was incubated for 0 day and 14 days, and then ADC samples were taken out. 100 µL of Protein A was added to each tube, and the resulting mixture was adsorbed for 2 h and eluted to obtain the incubated ADCs. The DAR values of the incubated ADCs were measured (the measurement and calculation methods were the same as those in Example 5.2.1) to determine the plasma stability of the samples.

The test results show that the DAR value of each ADC prepared in Example 5 was substantially maintained during the incubation in the plasma, while the DAR value of an ADC with a maleimide linker (e.g., the control compound HER2-ADC-006) was significantly reduced after 14 days of incubation. Therefore, the ADCs provided by the present invention all have better plasma stability, and thus can reduce the shedding of bioactive compounds in non-target tissues.

### Example 10. Stability Tests of Antibody-Drug Conjugates in Tumor Tissue Homogenates and Muscle Tissue Homogenates

Referring to Example 6.11 of patent WO 2022/170971A1, each ADC prepared in Example 5 was simulated to be cleaved in tumor tissue/tumor microenvironment, and each ADC prepared in Example 5 was subjected to *in vitro* investigation for stability in 22RV1 Balb/c nu nude mouse tumor tissue homogenates, Balb/c mouse muscle tissue homogenates and homogenate matrix normal saline to determine the payload in an incubation system. The tumor tissue homogenate results were compared with the plasma stability results to evaluate the release efficiency of the payload in the tumor tissue/tumor microenvironment.

The test results show that each ADC prepared in Example 5 could release a large number of payloads in the tumor tissue homogenates, but there was no significant release in the normal tissue homogenates. Therefore, the ADCs provided by the present invention can increase the effective release of the bioactive compounds at the tumor site, thereby having a higher therapeutic index.

Unless otherwise defined, all terms used herein have the meanings commonly understood by those skilled in the art.

The embodiments described in the present invention are for illustrative purposes only and are not intended to limit the scope of the present invention, and various other substitutions, alterations, and modifications may be made by those skilled in the art within the scope of the present invention. Therefore, the present invention is not limited to the embodiments described above, but is limited only by the claims.

## Claims

1. An antibody-drug conjugate of formula I, 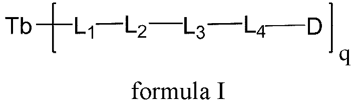
or a stereoisomer of the antibody-drug conjugate, a prodrug thereof, a pharmaceutically acceptable salt thereof or a pharmaceutically acceptable solvate thereof;
wherein,
Tb is an antibody or an antigen-binding fragment thereof;
q is the number of L₁-L₂-L₃-L₄-D coupled to Tb;
D is a protein degradation agent bioactive compound fragment;
L₁ is a linker unit;
L₂ is a connector unit;
L₃ is selected from an amino acid residue represented by AA¹ or a short peptide consisting of 2-10 amino acid residues comprising the amino acid residue represented by AA¹; the amino acid residue is selected from a natural amino acid residue, a non-natural amino acid residue, the amino acid residue represented by AA¹ or a stereoisomer thereof;
the amino acid residue represented by AA¹ has a structure shown below: 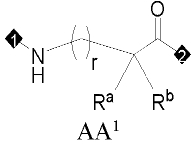 wherein:
R^{a} and R^{b} are each independently selected from hydrogen or 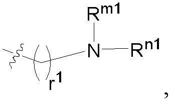
and R^{a} and R^{b} are not both hydrogen;
or R^{a} and R^{b}, together with the carbon atom to which they are both linked, form a 4-10 membered heterocycle, and the 4-10 membered heterocycle is optionally substituted with one or more R⁰; r and r¹ are each independently selected from any integer between 0 and 20;
R^{m1} and Rⁿ¹ are each independently selected from hydrogen, C1-6 alkyl or C3-6 cycloalkyl;
or R^{m1} and Rⁿ¹, together with the nitrogen atom to which they are both linked, form a 4-10 membered heterocycle, and the 4-10 membered heterocycle is optionally substituted with one or more R^{0'};
R⁰ and R^{0'} are each independently selected from C1-6 alkyl, C3-6 cycloalkyl, -NR^{m2}Rⁿ², or 4-10 membered heterocyclyl optionally substituted with C1-6 alkyl;
R^{m2} and Rⁿ² are each independently selected from hydrogen or C1-6 alkyl;
L₄ is absent or present, and when L₄ is present, L₄ is selected from 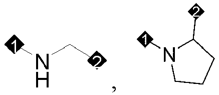 or 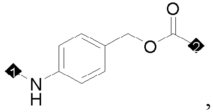 wherein position 1 is attached to L₃, and position 2 is attached to D.

2. The antibody-drug conjugate or the stereoisomer of the antibody-drug conjugate, the prodrug thereof, the pharmaceutically acceptable salt thereof or the pharmaceutically acceptable solvate thereof according to claim 1, wherein one or more of the following conditions are met:
(1) q is selected from any value between 0.1 and 12.0; preferably, q is selected from any value between 1.0 and 10.0; more preferably, q is selected from 1, 2, 3, 4, 5, 6, 7, 8, 9 or 10;
(2) L₁ is selected from 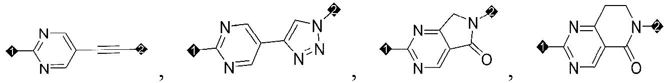 or 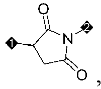 wherein position 1 is attached to Tb via an S atom, and position 2 is attached to L₂;
(3) L₂ is selected from 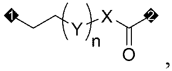 wherein position 1 is attached to L₁, and position 2 is attached to L₃;
preferably, L₂ is selected from 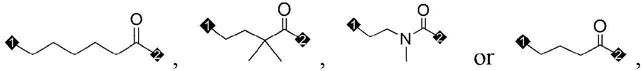 wherein position 1 is attached to L₁, and position 2 is attached to L₃;
(4) n is selected from any integer between 0 and 10; preferably, n is selected from 0, 1, 2 or 3;
(5) Y is selected from -CH₂- or -OCH₂CH₂-;
(6) X is selected from -CR^{m}Rⁿ- or -NR^{m}-;
(7) R^{m} and Rⁿ are each independently selected from hydrogen, C1-4 alkyl, C2-4 alkenyl, C2-4 alkynyl, C3-6 cycloalkyl or 3-6 membered heterocyclyl;
or R^{m} and Rⁿ, together with the carbon atom to which they are both linked, form a 3-6 membered carbocycle or a 3-6 membered heterocycle;
preferably, R^{m} and Rⁿ are each independently selected from hydrogen or methyl;
(8) L₃ is selected from the amino acid residue represented by AA¹ or a dipeptide, a tripeptide or a tetrapeptide comprising the amino acid residue represented by AA¹; preferably, L₃ is selected from an amino acid residue, a dipeptide or a tripeptide as shown below: AA¹, Val-AA¹, Ala-AA¹, Gly-AA¹, AA¹-Gly, AA¹-Ala, Val-AA¹-Gly, Ala-AA¹-Gly, Gly-AA¹-Gly or Val-AA¹-Ala; more preferably, L₃ is selected from a dipeptide or a tripeptide as shown below: Val-AA¹, Val-AA¹-Ala or Val-AA¹-Gly; particularly preferably, L₃ is a tripeptide represented by Val-AA¹-Gly;
(9) one of R^{a} and R^{b} is H, and the other is 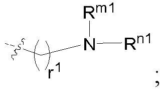 or R^{a} and R^{b}, together with the carbon atom to which they are both linked, form a 5-6 membered heterocycle substituted with R⁰;
(10) r and r¹ are each independently selected from 0, 1, 2, 3, 4 or 5;
preferably, r and r¹ are each independently selected from 0 or 4;
more preferably, r is 0, and r¹ is 4;
(11) R^{m1} and Rⁿ¹ are each independently selected from hydrogen or C1-6 alkyl; or R^{m1} and Rⁿ¹, together with the nitrogen atom to which they are both linked, form a 5-6 membered heterocycle optionally substituted with R^{0'};
preferably, R^{m1} and Rⁿ¹ are each independently selected from hydrogen, methyl, ethyl, n-propyl or n-butyl; or R^{m1} and Rⁿ¹, together with the nitrogen atom to which they are both linked, form a piperidine ring or piperazine ring optionally substituted with R^{0'}; more preferably, R^{m1} and Rⁿ¹ are each independently selected from methyl, ethyl, n-propyl or n-butyl;
(12) R⁰ and R^{0'} are each independently selected from C1-6 alkyl, -NR^{m2}Rⁿ², or 5-6 membered heterocyclyl optionally substituted with C1-6 alkyl;
(13) R^{m2} and Rⁿ² are methyl; and
(14) the protein degradation agent bioactive compound is a molecule that can lead to the degradation of GSPT1 family proteins.

3. The antibody-drug conjugate or the stereoisomer of the antibody-drug conjugate, the prodrug thereof, the pharmaceutically acceptable salt thereof or the pharmaceutically acceptable solvate thereof according to claim 1 or 2, wherein one or more of the following conditions are met:
(1) L₁-L₂ is selected from 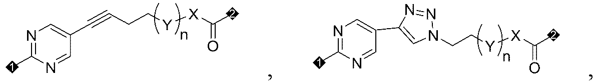 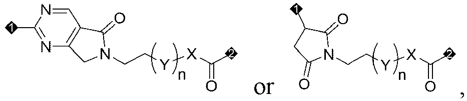 wherein position 1 is attached to Tb via an S atom, and position 2 is attached to L₃; preferably, L₁-L₂ is selected from 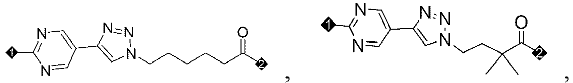 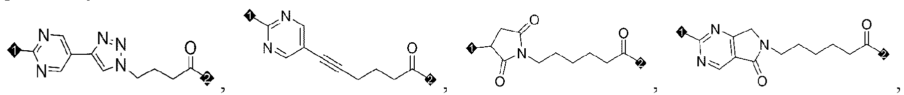 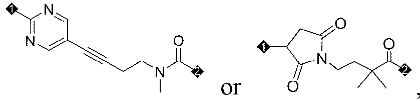 wherein position 1 is attached to Tb via an S atom, and position 2 is attached to L₃;
(2) L₃ is selected from 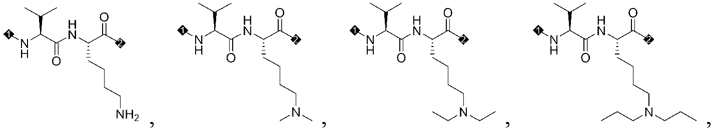 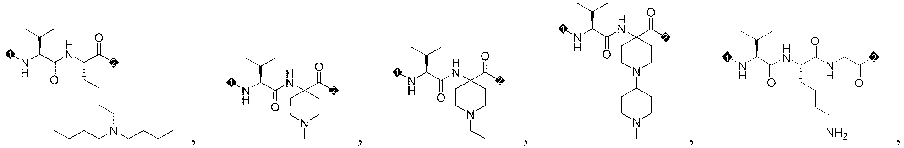 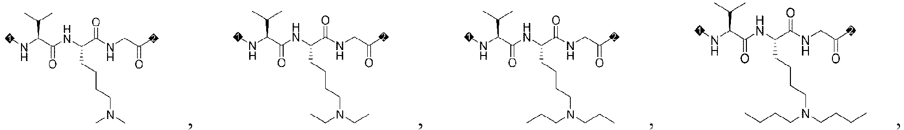 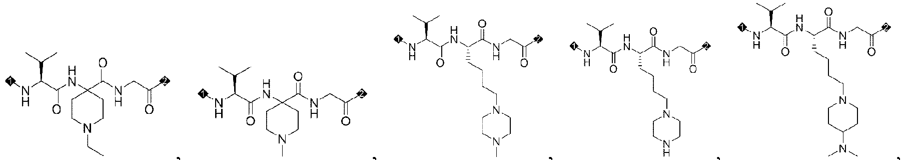 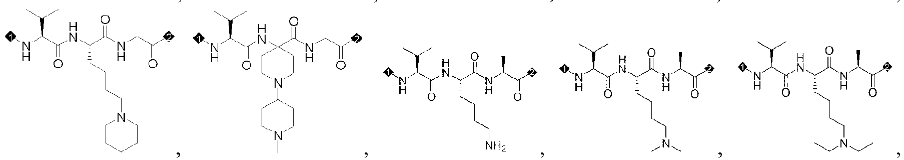 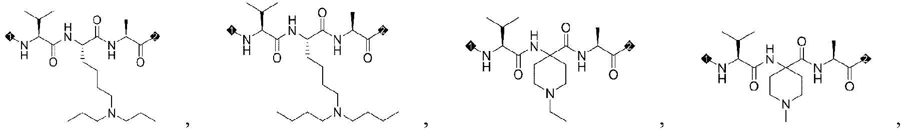 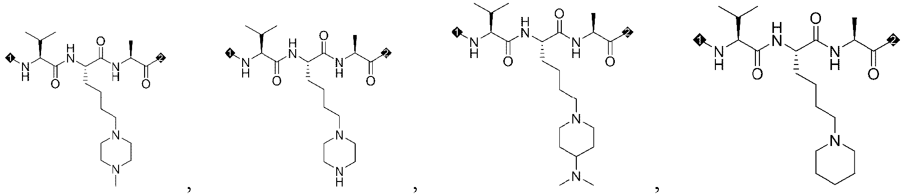 or 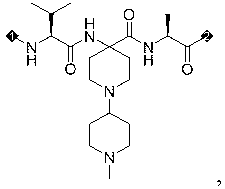 wherein position 1 is attached to L₂, and position 2 is attached to L₄ or D;
preferably, L₃ is selected from 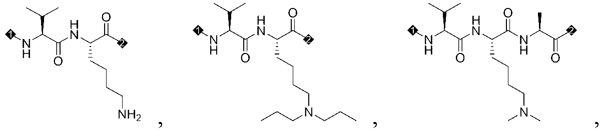 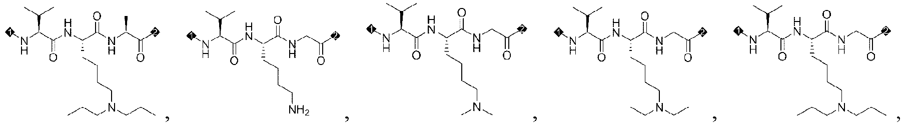 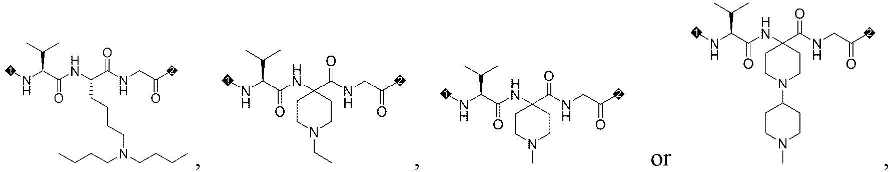 wherein position 1 is attached to L₂, and position 2 is attached to L₄ or D; and
(3) the amino acid residue represented by AA¹ is selected from 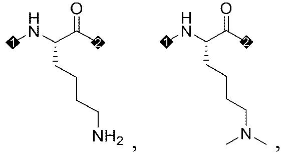 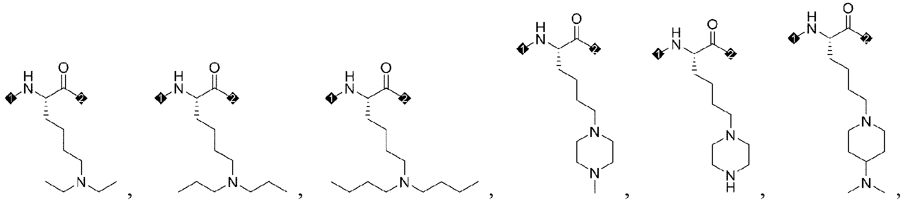 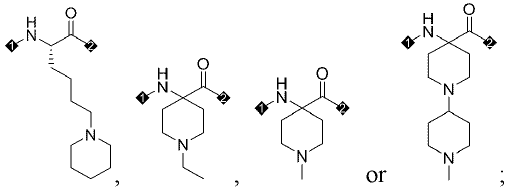 preferably, the amino acid residue represented by AA¹ is selected from

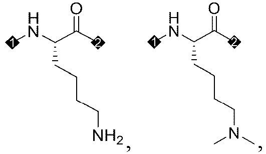

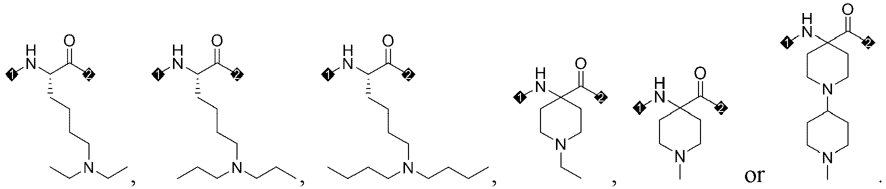

4. The antibody-drug conjugate or the stereoisomer of the antibody-drug conjugate, the prodrug thereof, the pharmaceutically acceptable salt thereof or the pharmaceutically acceptable solvate thereof according to any one of claims 1-3, wherein, 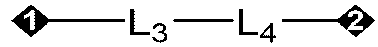 is selected from: 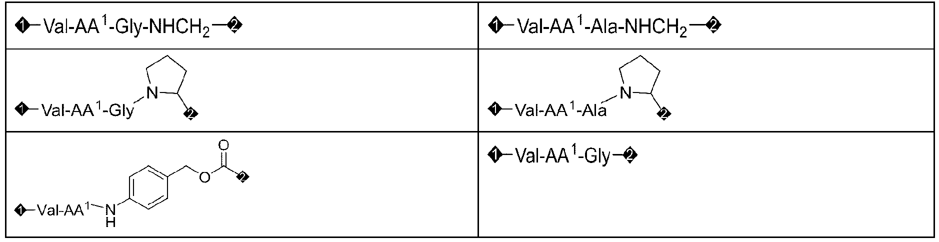
wherein position 1 is attached to L₂, position 2 is attached to D, and AA¹ is as defined in any one of claims 1-3;
preferably, 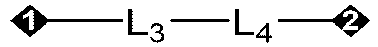 is selected from: 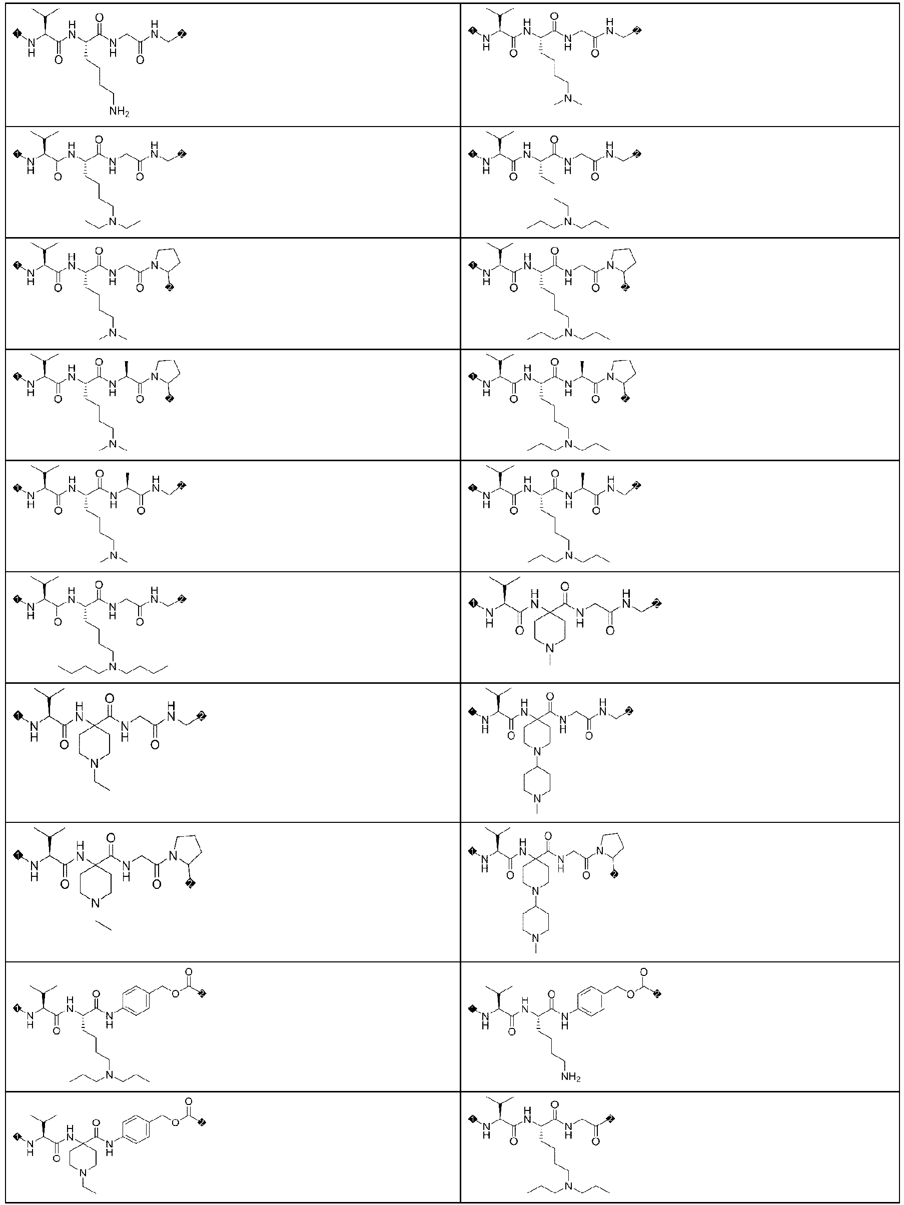
wherein position 1 is attached to L₂, and position 2 is attached to D;
more preferably, 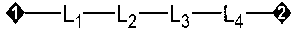 is selected from: 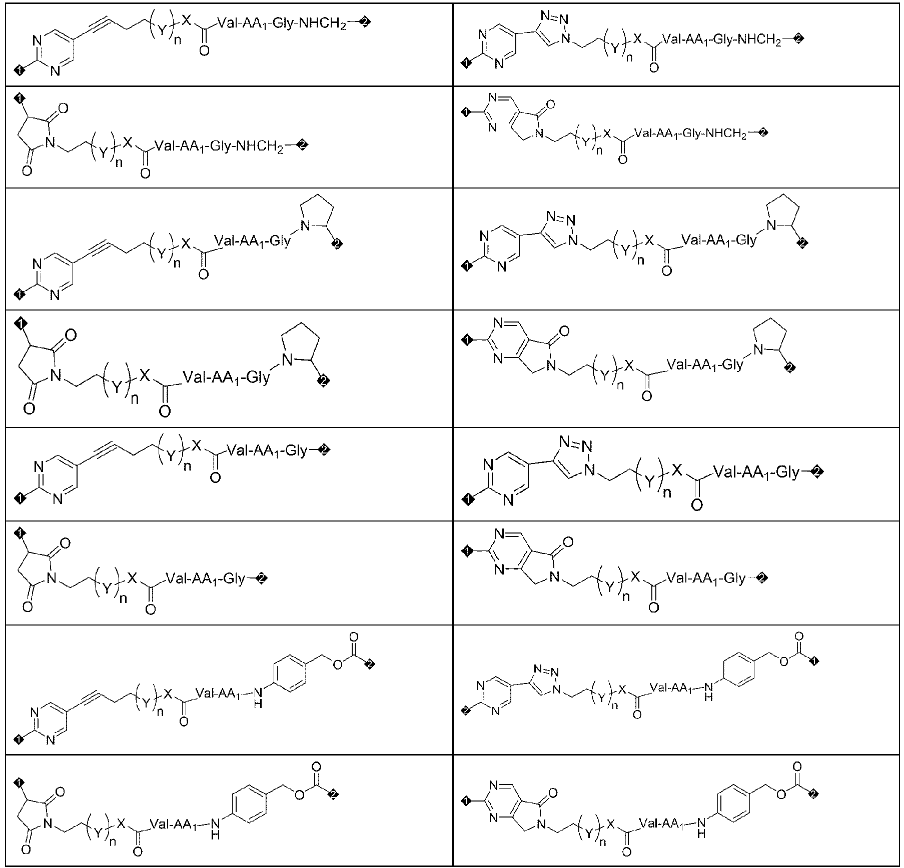
wherein position 1 is attached to Tb via an S atom, position 2 is attached to D, and Y, X, AA¹ and n are as defined in any one of claims 1-3;
further preferably, 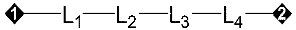 is selected from: 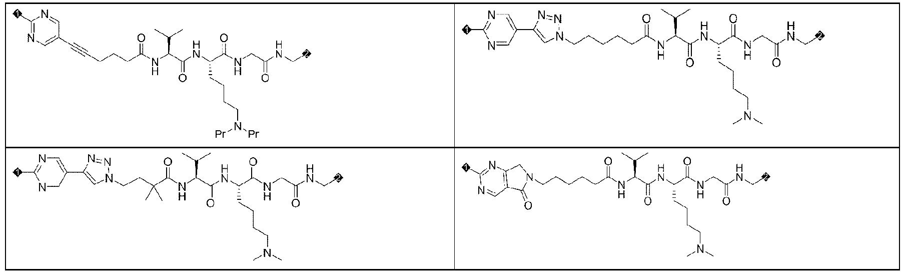 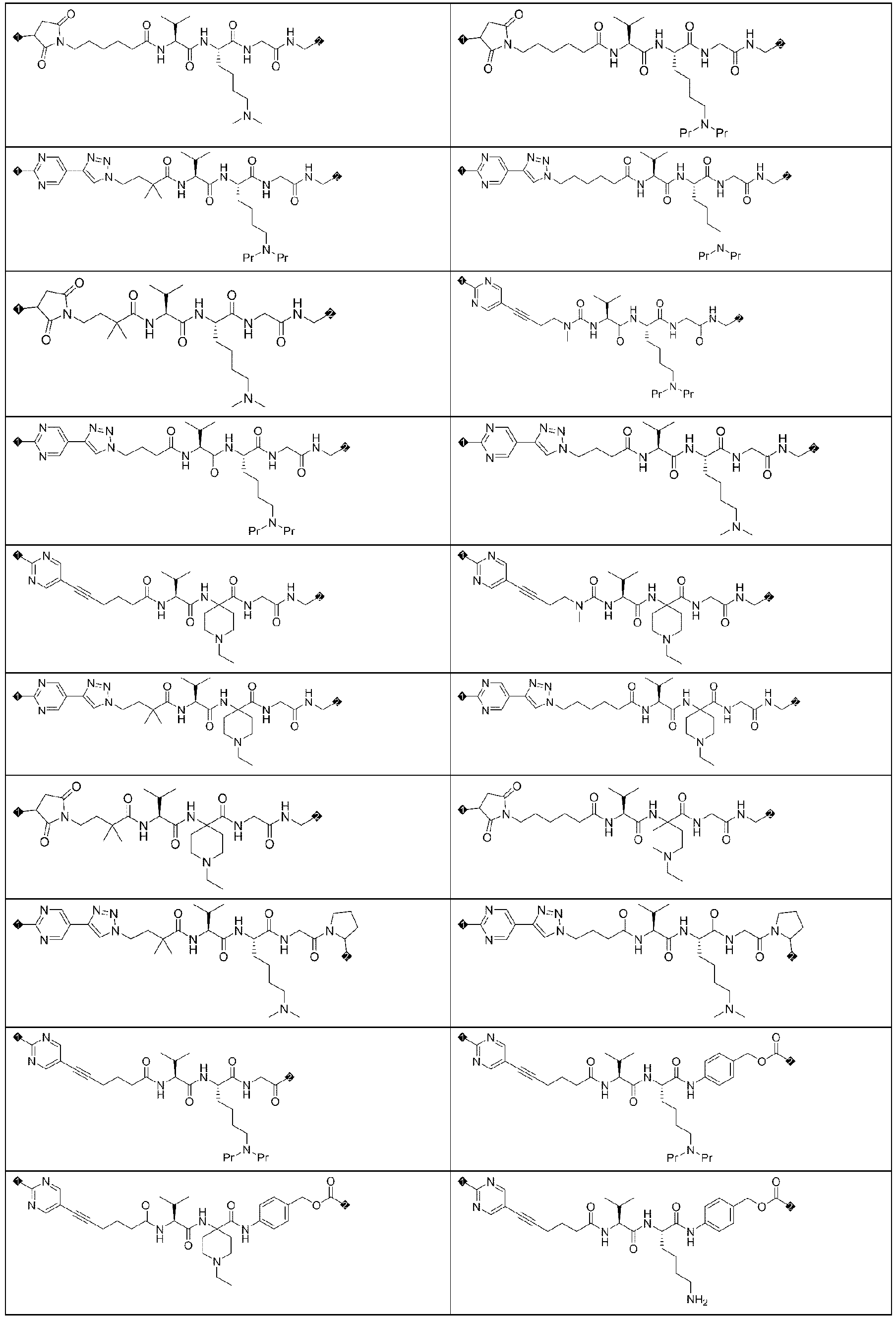
wherein position 1 is attached to Tb via an S atom, and position 2 is attached to D.

5. The antibody-drug conjugate or the stereoisomer of the antibody-drug conjugate, the prodrug thereof, the pharmaceutically acceptable salt thereof or the pharmaceutically acceptable solvate thereof according to any one of claims 1-4, wherein D is a structural unit of formula II, wherein position 1 is attached to L₄ or L₃; 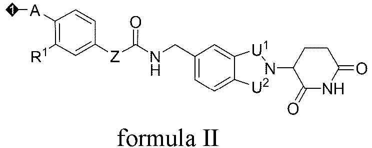 wherein,
R¹ is selected from hydrogen, deuterium, halogen, cyano, amino, nitro, C1-4 alkoxy or C1-4 alkyl;
Z is selected from -NH-, -CR^{2a}R^{3a}- or -C(O)-;
R^{2a} and R^{3a} are each independently selected from F or OH;
U¹ and U² are each independently selected from -CH₂- or -C(O)-, and U¹ and U² are not both -CH₂-;
A is selected from O, S, NR⁹, 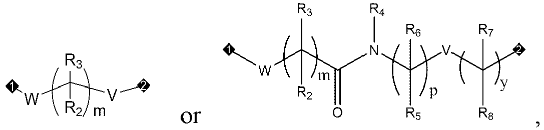
wherein position 1 is attached to L₄ or L₃, and position 2 is attached to a benzene ring; or position 1 is attached to a benzene ring, and position 2 is attached to L₄ or L₃; R₂, R₃, R₄, R₅, R₆, R₇ and R₈ are each independently selected from hydrogen, deuterium, C1-4 alkyl, C3-6 cycloalkyl or 3-6 membered heterocyclyl;
or R₂ and R₃, together with the carbon atom to which they are linked, form a 3-7 membered carbocycle or a 3-7 membered heterocycle;
R₄ and R₅, together with the carbon atom and the nitrogen atom to which they are each linked, form a 4-7 membered heterocycle;
R₅ and R₆, together with the carbon atom to which they are linked, form a 3-7 membered carbocycle or a 3-7 membered heterocycle;
R₇ and R₈, together with the carbon atom to which they are linked, form a 3-7 membered carbocycle or a 3-7 membered heterocycle;
m, p and y are each independently selected from any integer between 0 and 8;
W and V are each independently selected from a direct bond, O, S or NR⁹;
R⁹ is selected from hydrogen, deuterium, C1-4 alkyl or C3-6 cycloalkyl.

6. The antibody-drug conjugate or the stereoisomer of the antibody-drug conjugate, the prodrug thereof, the pharmaceutically acceptable salt thereof or the pharmaceutically acceptable solvate thereof according to claim 5, wherein one or more of the following conditions are met:
(1) R¹ is selected from hydrogen, halogen or C1-4 alkyl; preferably, R¹ is halogen;
(2) Z is -NH-;
(3) U¹ and U² are each independently selected from -CH₂- or -C(O)-, and U¹ and U² are different; preferably, U¹ is -CH₂-, and U² is -C(O)-;
(4) A is 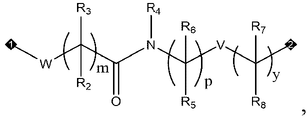 wherein position 1 is attached to L₄ or L₃, and position 2 is attached to a benzene ring; W, V, R₂, R₃, R₄, R₅, R₆, R₇, R₈, m, p and y are as defined in claim 5; preferably, A is selected from -O(CH₂)ₘC(O) NH (CH₂)_{P}-, -O(CH₂)ₘC(O) N(CH₃) (CH₂)_{P}-, -O(CH₂)ₘC(O) NH (CH₂)_{P}O-, -O(CH₂)ₘC(O) N(CH₃) (CH₂)_{P} O-, -S(CH₂)ₘC(O) NH (CH₂)_{P}-, -S(CH₂)ₘC(O) N(CH₃) (CH₂)_{P}-, -NH(CH₂)ₘC(O) NH (CH₂)_{P}-, -NH(CH₂)ₘC(O) N(CH₃) (CH₂)_{P}-, -O(CH₂)ₘC(O) NH (CH₂)_{PO} (CH₂)_{y}- or -O(CH₂)ₘC(O) N(CH₃) (CH₂)_{P}O(CH₂)_{y}-; preferably, A is selected from -O(CH₂)ₘC(O) N(CH₃) (CH₂)_{P}- or -O(CH₂)ₘC(O) N(CH₃) (CH₂)_{P}O(CH₂)_{y}-; preferably, A is selected from -OCH₂C(O) N(CH₃) (CH₂)₃- or -OCH₂C(O) N(CH₃) (CH₂)₂O(CH₂)₂-;
(5) W is selected from O, S or NR⁹, and preferably, W is O;
(6) V is selected from a direct bond, O, S or NR⁹, and preferably, V is selected from a direct bond or O;
(7) R₂, R₃, R₄, R₅, R₆, R₇ and R₈ are each independently selected from hydrogen, deuterium or C1-4 alkyl;
(8) m, p and y are each independently selected from 0, 1, 2, 3 or 4; and
(9) R₉ is selected from hydrogen, deuterium or methyl.

7. The antibody-drug conjugate or the stereoisomer of the antibody-drug conjugate, the prodrug thereof, the pharmaceutically acceptable salt thereof or the pharmaceutically acceptable solvate thereof according to claim 5 or 6, wherein D is a structural unit of II-1, II-2 or II-3: 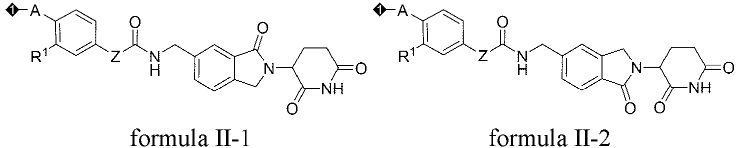 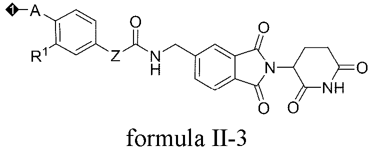 wherein R¹, A and Z are as defined in claim 5 or 6, and position 1 is attached to L₄ or L₃.

8. The antibody-drug conjugate or the stereoisomer of the antibody-drug conjugate, the prodrug thereof, the pharmaceutically acceptable salt thereof or the pharmaceutically acceptable solvate thereof according to any one of claims 1-7, wherein D is selected from the following structures, and position 1 is attached to L₄ or L₃:

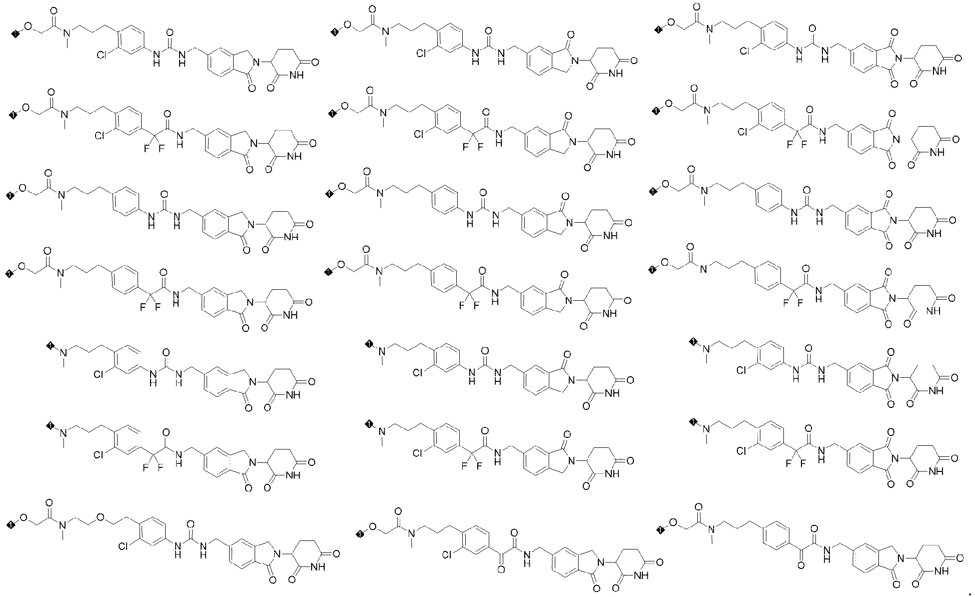

9. The antibody-drug conjugate or the stereoisomer of the antibody-drug conjugate, the prodrug thereof, the pharmaceutically acceptable salt thereof or the pharmaceutically acceptable solvate thereof according to any one of claims 1-8, wherein the antibody-drug conjugate has a structure of formula III, 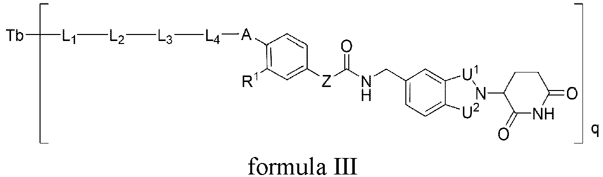 wherein Tb, q, L₁, L₂, L₃, L₄, R¹, A, Z, U¹ and U² are as defined in any one of claims 1-8.

10. The antibody-drug conjugate or the stereoisomer of the antibody-drug conjugate, the prodrug thereof, the pharmaceutically acceptable salt thereof or the pharmaceutically acceptable solvate thereof according to any one of claims 1-9, wherein the antibody-drug conjugate is selected from: 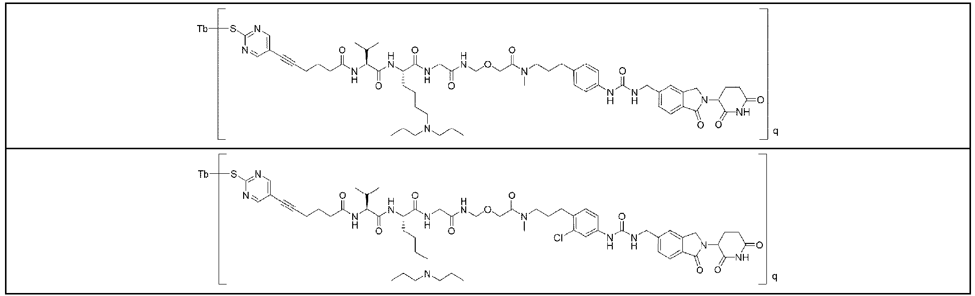 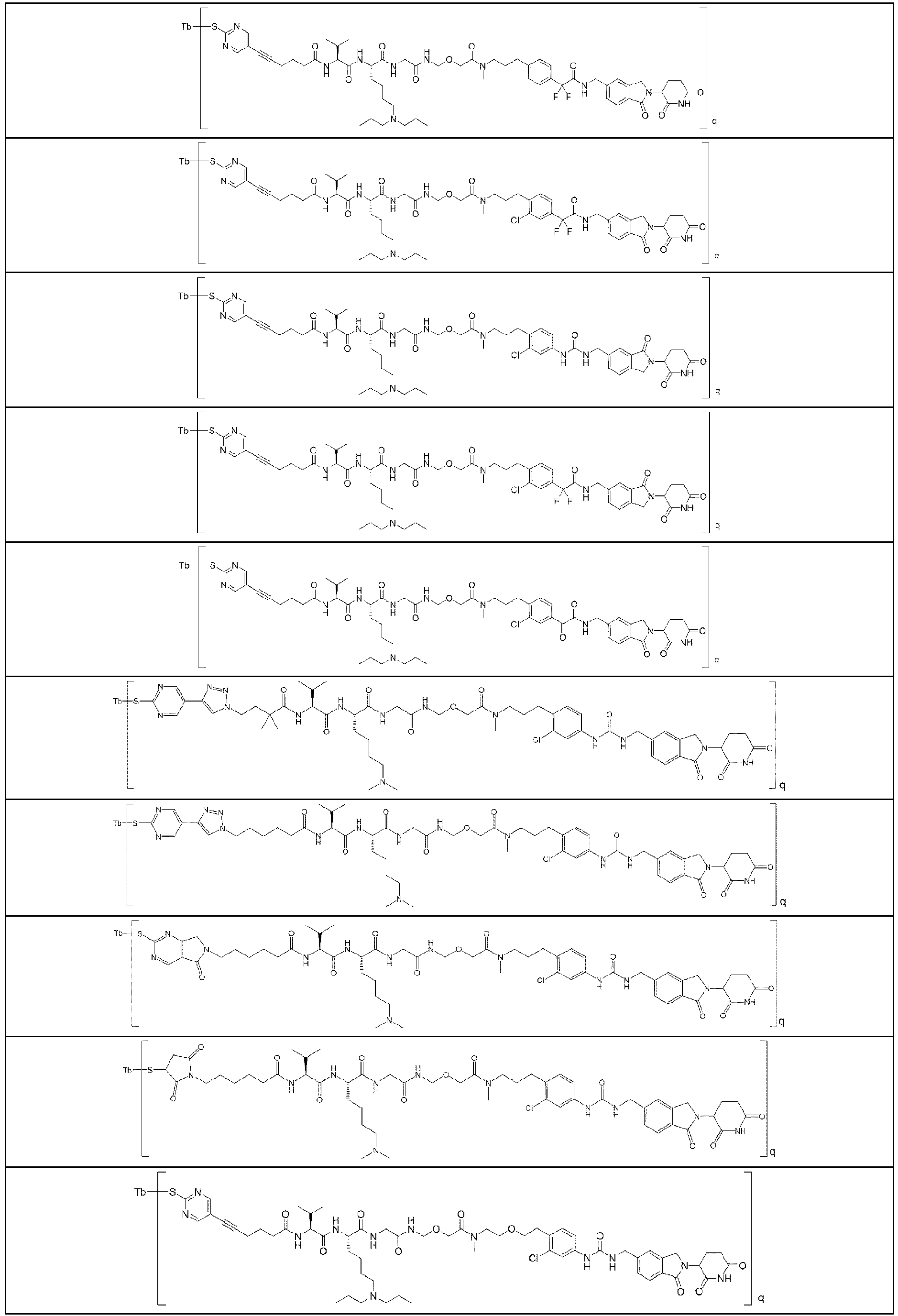 wherein q is selected from any value between 1.0 and 10.0; preferably, q is selected from 1, 2, 3, 4, 5, 6, 7, 8, 9 or 10; more preferably, q is selected from 2, 4, 6 or 8.

11. The antibody-drug conjugate or the stereoisomer of the antibody-drug conjugate, the prodrug thereof, the pharmaceutically acceptable salt thereof or the pharmaceutically acceptable solvate thereof according to any one of claims 1-10, wherein Tb is selected from one or more of:
(1) Tb being an antibody or an antigen-binding fragment thereof with endocytic activity;
(2) Tb being an antibody or an antigen-binding fragment thereof with activity of binding to a tumor cell surface antigen;
(3) Tb being an anti-B7H3 antibody or an antigen-binding fragment thereof, an anti-Trop-2 antibody or an antigen-binding fragment thereof, an anti-Her2 antibody or an antigen-binding fragment thereof, an anti-Her3 antibody or an antigen-binding fragment thereof, or an anti-EGFR antibody or an antigen-binding fragment thereof;
(4) Tb being an anti-B7H3 antibody or an antigen-binding fragment thereof, such as 1D1-01, 2E3-02 antibody, enoblituzumab, mirzotamab, omburtamab, or an antigen-binding fragment thereof;
(5) Tb being an anti-Her2 antibody or an antigen-binding fragment thereof, such as anbenitamab, coprelotamab, disitamab, gancotamab, margetuximab, pertuzumab, timigutuzumab, zanidatamab, Trastuzumab, Pertuzumab, or an antigen-binding fragment thereof; preferably, Tb being Trastuzumab or Pertuzumab;
(6) Tb being an anti-EGFR antibody or an antigen-binding fragment thereof, such as demupitamab, depatuxizumab, futuximab, imgatuzumab, laprituximab, losatuxizumab, matuzumab, modotuximab, necitumumab, nimotuzumab, panitumumab, pimurutamab, serclutamab, tomuzotuximab, zalutumumab, Cetuximab, or an antigen-binding fragment thereof;
(7) Tb being an anti-B7H3 antibody or an antigen-binding fragment thereof having a VH sequence set forth in SEQ ID NO: 1 and a VL sequence set forth in SEQ ID NO: 2; preferably, Tb being an anti-B7H3 antibody or an antigen-binding fragment thereof having a heavy chain sequence set forth in SEQ ID NO: 3 and a light chain sequence set forth in SEQ ID NO: 4; and
(8) Tb being an anti-B7H3 antibody or an antigen-binding fragment thereof having a VH sequence set forth in SEQ ID NO: 5 and a VL sequence set forth in SEQ ID NO: 6; preferably, Tb being an anti-B7H3 antibody or an antigen-binding fragment thereof having a heavy chain sequence set forth in SEQ ID NO: 7 and a light chain sequence set forth in SEQ ID NO: 8.

12. A drug-linker conjugate of formula IV,
Lg-L₁-L₂-L₃-L₄-D formula IV
or a stereoisomer of the drug-linker conjugate, a prodrug thereof, a pharmaceutically acceptable salt thereof or a pharmaceutically acceptable solvate thereof,
wherein,
when L₁ is 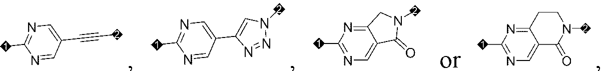 position 1 is attached to Lg, and position 2 is attached to L₂;
Lg is a leaving group when reacting with an antibody; L₂, L₃, L₄ and D are as defined in any one of claims 1-10;
when L₁ is 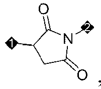 Lg-L₁ is 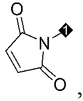 and L₂, L₃, L₄ and D are as defined in any one of claims 1-10.

13. The drug-linker conjugate or the stereoisomer of the drug-linker conjugate, the prodrug thereof, the pharmaceutically acceptable salt thereof or the pharmaceutically acceptable solvate thereof according to claim 12, wherein Lg is selected from halogen, sulfonyl, a tertiary amine salt group (Me₃N⁺ or Et₃N⁺), a diazonium salt group, -OMs, MeSO₂- or CF₃SO₃-; preferably; Lg is selected from F, Cl or MeSO₂-; more preferably, Lg is selected from F or MeSO₂-.

14. The drug-linker conjugate or the stereoisomer of the drug-linker conjugate, the prodrug thereof, the pharmaceutically acceptable salt thereof or the pharmaceutically acceptable solvate thereof according to claim 12 or 13, wherein the drug-linker conjugate has a structure of formula V, 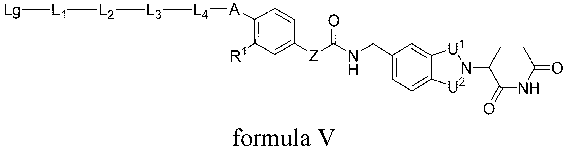 wherein Lg, L₁, L₂, L₃, L₄, R¹, A, Z, U¹ and U² are as defined in any one of claims 1-13.

15. The drug-linker conjugate or the stereoisomer of the drug-linker conjugate, the prodrug thereof, the pharmaceutically acceptable salt thereof or the pharmaceutically acceptable solvate thereof according to any one of claims 12-14, wherein the drug-linker conjugate is selected from:

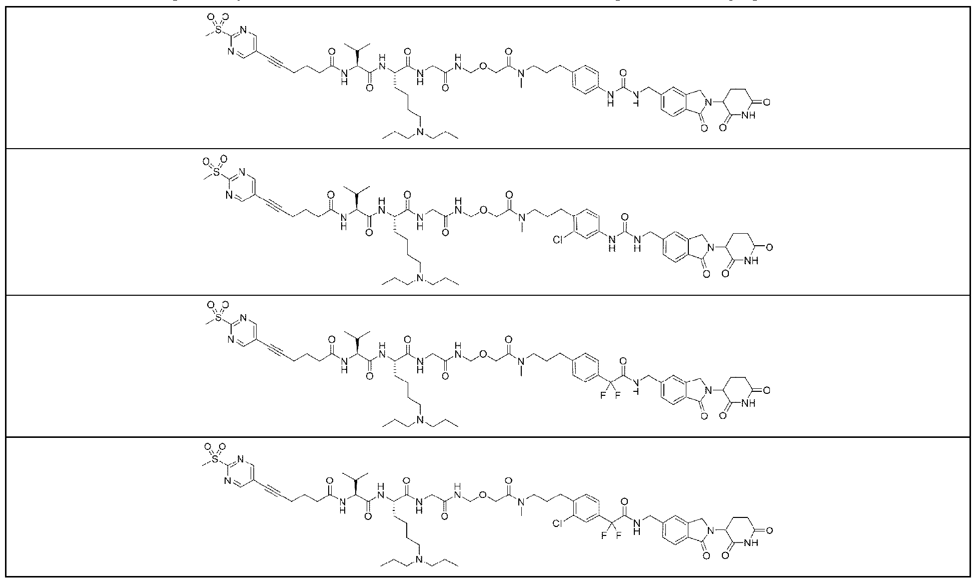

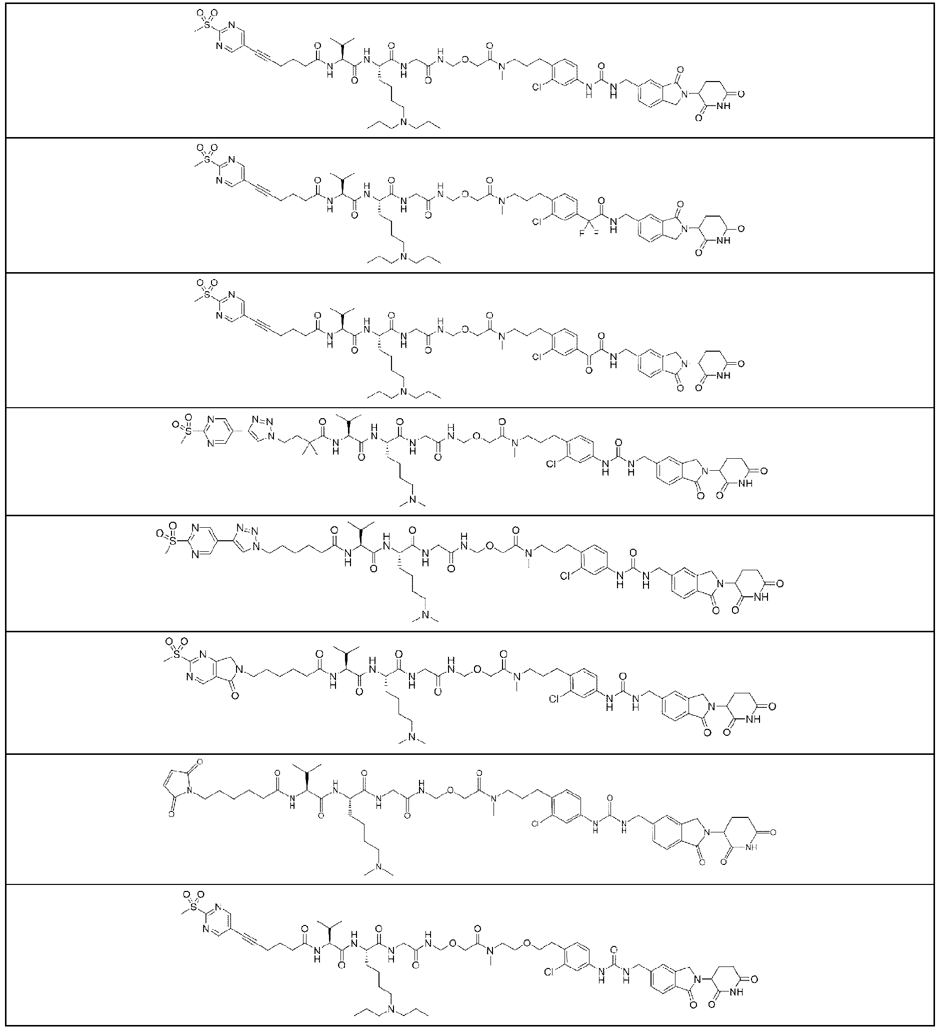

16. A bioactive compound of formula IIA, 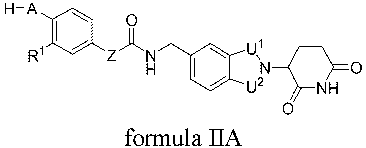
or a stereoisomer of the bioactive compound, a prodrug thereof, a pharmaceutically acceptable salt thereof or a pharmaceutically acceptable solvate thereof,
wherein,
R¹ is selected from hydrogen, halogen or C1-4 alkyl; preferably, R¹ is halogen;
Z is -NH-;
U¹ and U² are each independently selected from -CH₂- or -C(O)-, and U¹ and U² are different; preferably, U¹ is -CH₂-, and U² is -C(O)-;
A is 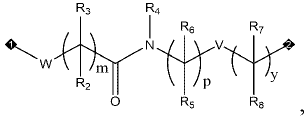 wherein position 1 is attached to L₄ or L₃, and position 2 is attached to a benzene ring; preferably, A is selected from -O(CH₂)ₘC(O) NH (CH₂)_{P}-, -O(CH₂)ₘC(O) N(CH₃) (CH₂)_{P}-, -O(CH₂)ₘC(O) NH (CH₂)_{P}O-, -O(CH₂)ₘC(O) N(CH₃) (CH₂)_{P} O-, -S(CH₂)ₘC(O) NH (CH₂)_{P}-, -S(CH₂)ₘC(O) N(CH₃) (CH₂)_{P}-, -NH(CH₂)ₘC(O) NH (CH₂)_{P}-, -NH(CH₂)ₘC(O) N(CH₃) (CH₂)_{P}-, -O(CH₂)ₘC(O) NH (CH₂)_{P}O (CH₂)_{y}- or -O(CH₂)ₘC(O) N(CH₃) (CH₂)_{P}O(CH₂)_{y}-; preferably, A is selected from -O(CH₂)ₘC(O) N(CH₃) (CH₂)_{P}- or -O(CH₂)ₘC(O) N(CH₃) (CH₂)_{P}O(CH₂)_{y}-; preferably, A is selected from -OCH₂C(O) N(CH₃) (CH₂)₃- or -OCH₂C(O) N(CH₃) (CH₂)₂O(CH₂)₂-;
W is selected from O, S or NR⁹, and preferably, W is O;
V is selected from a direct bond, O, S or NR⁹, and preferably, V is selected from a direct bond or O;
R₂, R₃, R₄, R₅, R₆, R₇ and R₈ are each independently selected from hydrogen, deuterium or C1-4 alkyl;
m, p and y are each independently selected from 0, 1, 2, 3 or 4;
R₉ is selected from hydrogen, deuterium or methyl.

17. The bioactive compound or the stereoisomer of the bioactive compound, the prodrug thereof, the pharmaceutically acceptable salt thereof or the pharmaceutically acceptable solvate thereof according to claim 16, wherein the bioactive compound is selected from:

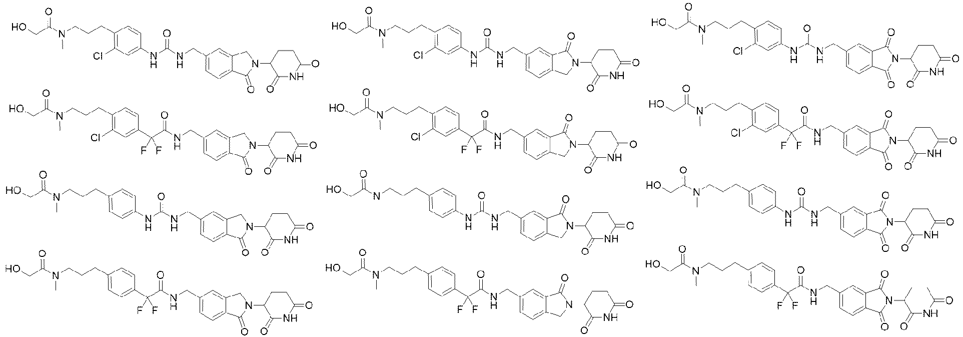

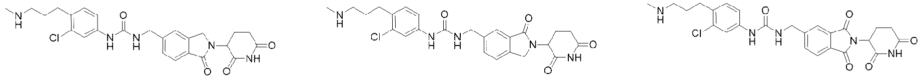

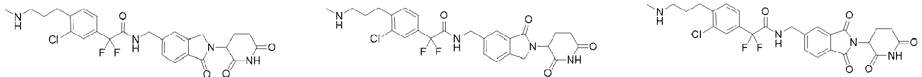

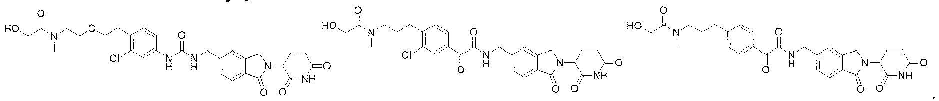

18. A method for preparing an antibody-drug conjugate of formula I, comprising: subjecting Tb and a drug-linker conjugate of formula IV Lg-L₁-L₂-L₃-L₄-D to a coupling reaction, wherein Tb, L₁, L₂, L₃, L₄, Lg and D are as defined in any one of claims 1-15.

19. The method according to claim 18, wherein the method comprises a step of subjecting Tb and the drug-linker conjugate of formula IV Lg-L₁-L₂-L₃-L₄-D to the coupling reaction to form a C-S bond;
Tb and the drug-linker conjugate are in a molar ratio of 1:(1-20), such as 1:(2-20), 1:(4-20), 1:(6-20), 1:(8-20), 1:(10-20), 1:(12-20), 1:(14-20), 1:(16-20) or 1:(18-20);
the coupling reaction is preferably carried out in water and/or an organic solvent; the organic solvent is preferably one or more of N,N-dimethylformamide, dimethyl sulfoxide, N-methylpyrrolidone, a nitrile solvent and an alcohol solvent.

20. A linker of formula V-A-1, 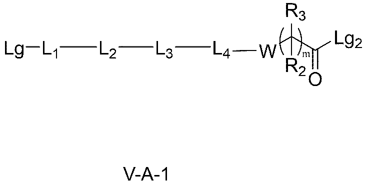 wherein Lg, L₁, L₂, L₃, L₄, W, R₂, R₃ and m are as defined in any one of claims 12-15, and Lg₂ is selected from hydroxy, halogen, activated hydroxy, preferably from hydroxy, chlorine, bromine,

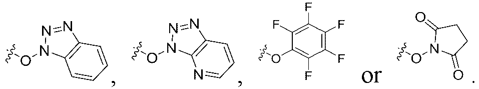

21. The linker according to claim 20, wherein the linker is selected from:

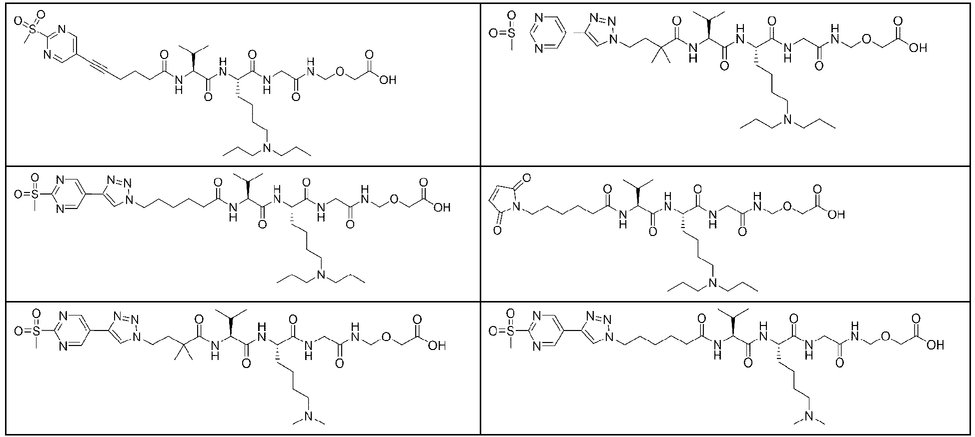

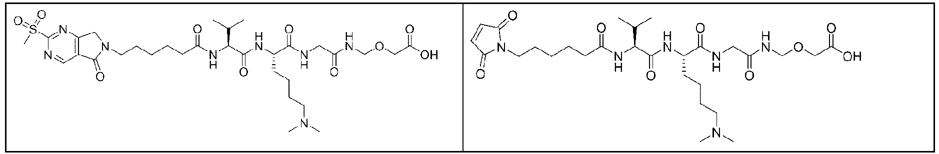

22. A pharmaceutical composition, comprising the antibody-drug conjugate or the stereoisomer of the antibody-drug conjugate, the prodrug thereof, the pharmaceutically acceptable salt thereof or the pharmaceutically acceptable solvate thereof according to any one of claims 1-11; or the drug-linker conjugate or the stereoisomer of the drug-linker conjugate, the prodrug thereof, the pharmaceutically acceptable salt thereof or the pharmaceutically acceptable solvate thereof according to any one of claims 12-15; or the bioactive compound or the stereoisomer of the bioactive compound, the prodrug thereof, the pharmaceutically acceptable salt thereof or the pharmaceutically acceptable solvate thereof according to any one of claims 16-17, and optionally one or more pharmaceutical adjuvants.

23. The pharmaceutical composition according to claim 22, wherein in the antibody-drug conjugate, a drug-to-antibody ratio (DAR) is any value between 1.0 and 12.0; preferably, the drug-to-antibody ratio (DAR) is any value within 2±0.4, 4±0.4, 6±0.4 or 8±0.4.

24. Use of the antibody-drug conjugate or the stereoisomer of the antibody-drug conjugate, the prodrug thereof, the pharmaceutically acceptable salt thereof or the pharmaceutically acceptable solvate thereof according to any one of claims 1-11; or the drug-linker conjugate or the stereoisomer of the drug-linker conjugate, the prodrug thereof, the pharmaceutically acceptable salt thereof or the pharmaceutically acceptable solvate thereof according to any one of claims 12-15; or the bioactive compound or the stereoisomer of the bioactive compound, the prodrug thereof, the pharmaceutically acceptable salt thereof or the pharmaceutically acceptable solvate thereof according to any one of claims 16-17; or the pharmaceutical composition according to claim 22 or 23 in the preparation of a medicament for treating and/or preventing a disease associated with abnormal cell activity (e.g., a cancer disease), wherein
preferably, the cancer disease is selected from esophageal cancer (e.g., esophageal adenocarcinoma or esophageal squamous cell carcinoma), brain tumor, lung cancer (e.g., small cell lung cancer or non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, urothelial cancer, solid tumors, non-Hodgkin's lymphoma, central nervous system tumors (e.g., neuroglioma, glioblastoma multiforme, glioma or sarcoma), prostate cancer or thyroid cancer.
